(19) Europäisches Patentamt
European Patent Office
Office européen des brevets

(11) EP 2 045 321 A2

(12) **EUROPEAN PATENT APPLICATION**

(43) Date of publication:
**08.04.2009 Bulletin 2009/15**

(51) Int Cl.:
***C12N 9/00*** *(2006.01)* ***C12N 15/00*** *(2006.01)*
***C12Q 1/37*** *(2006.01)*

(21) Application number: **09150549.5**

(22) Date of filing: **26.05.2006**

(84) Designated Contracting States:
**AT BE BG CH CY CZ DE DK EE ES FI FR GB GR HU IE IS IT LI LT LU LV MC NL PL PT RO SE SI SK TR**

(30) Priority: **27.05.2005 EP 05104543**

(62) Document number(s) of the earlier application(s) in accordance with Art. 76 EPC:
**06763303.2 / 1 883 696**

(71) Applicant: **Direvo Biotech AG**
**50829 Köln (DE)**

(72) Inventors:
- **Koltermann, André**
  **82057 Icking (DE)**
- **Kettling, Ulrich**
  **81477 München (DE)**
- **Haupts, Ulrich**
  **51519 Odenthal (DE)**
- **Coco, Wayne**
  **50737 Köln (DE)**
- **Tebbe, Jan**
  **50733 Köln (DE)**
- **Votsmeier, Christian**
  **50259 Pulheim (DE)**
- **Scheidig, Andreas**
  **50823 Köln (DE)**

(74) Representative: **von Kreisler Selting Werner Patentanwälte**
**P.O. Box 10 22 41**
**50462 Köln (DE)**

Remarks:
This application was filed on 14-01-2009 as a divisional application to the application mentioned under INID code 62.

# (54) Serine proteases with altered sensitivity to activity-modulating substances

(57) The present invention provides variants of serine proteases of the S1 class with altered sensitivity to one or more activity-modulating substances. A method for the generation of such proteases is disclosed, comprising the provision of a protease library encoding polynucleotide sequences, expression of the enzymes, screening of the library in the presence of one or several activity-modulating substances, selection of variants with altered sensitivity to one or several activity-modulating substances and isolation of those polynucleotide sequences that encode for the selected variants.

EP 2 045 321 A2

## Description

The present invention provides variants of serine proteases with altered sensitivity to one or more activity-modulating substances. A method for the generation of such proteases is disclosed, comprising the provision of a protease library encoding polynucleotide sequences, expression of the enzymes, screening of the library in the presence of one or several activity-modulating substances, selection of variants with altered sensitivity to one or several activity-modulating substances and isolation of those polynucleotide sequences that encode for the selected variants.

## Background of the Invention

Today many severe medical conditions remain untreatable and require innovative new approaches complementing the traditional medicinal chemistry development of drugs. One alternative emerges from the recent successful introduction of biological therapeutics for the treatment of a number of diseases. Examples for biological therapeutics ("biologics") comprise peptides, proteins, polynucleic acids, lipids or combinations thereof. Traditionally, biologics replaced the bodies own missing or inactive proteins. The potential of biologics has been dramatically broadened by the use of molecules with functions that are not present in the bodies own repertoire, e.g. antibodies directed against a number of targets which are inactivated by binding. In addition, enzymes with different catalytic functions have been developed that increase the rate of a desired reaction with a positive effect on the condition of the patient.

However, the activity of enzymes is highly regulated in the human or animal body at different levels. For example, the expression of an enzyme may be stimulated by activation of transcription factors, or an enzyme may be activated by a reversible posttranslational modification such as phosphorylation. In signal transduction kinase cascades are known in which upstream kinases phosphorylate and thereby activate downstream kinases. The biological effect is downregulated by the action of phosphatases which remove the phosphate residue and render the kinase inactive. The situation is different for example in proteolytic cascades such as known from the coagulation or complement cascade. The proteases are expressed as inactive proenzymes and are activated by proteolytic cleavage. In this case the downregulation of the protease activity is accomplished by the interaction with inhibitors which are present in blood or other body fluids and tissue at high concentrations. The inactivated proteases are degraded and cleared from the bloodstream.

With respect to applications as biological therapeutics proteases represent a particularly promising example as they can specifically activate or inactivate proteins that are involved in a disease or disease symptoms.

While antibodies bind targets in a fixed stoichiometry, a protease can activate or inactivate hundreds or thousands of target proteins. Therefore lower doses can be given with the potential of less side effects and lower manufacturing costs. Since nature does not provide proteases which cleave arbitrary targets of interest with sufficient specificity, ways of generating such specific proteases by molecular techniques have been devised. Specificity is an essential element of enzyme function. A cell consists of thousands of different, highly reactive catalysts. Yet the cell is able to maintain a coordinated metabolism and a highly organized three-dimensional structure. This is due in part to the specificity of enzymes, i.e. the selective conversion of their respective substrates. Specificity is a qualitative and a quantitative property. In nature, the specificity of an organism's enzymes has been evolved to the particular needs of the organism. Arbitrary specificities with high value for therapeutic, research, diagnostic, nutritional or industrial applications are unlikely to be found in any organism's enzymatic repertoire due to the large space of possible specificities. Therefore, defined specificities have to be generated de *novo.*

The application of therapeutic proteases in the treatment of diseases requires their activity in the presence of activity-modulating substances that are present in the application matrix where enzymatic activity is required, e.g. blood serum, extracellular fluid, cerebrospinal fluid, the intracellular environment, or any other environment in the body where activity is required. The serum in particular contains a large variety of protease inhibitors present in high concentrations, most notably serpins (serine protease inhibitors such as alpha1-antitrypsin, antithrombin, antiplasmin, and others) and macroglobulins (such as alpha2-macroglobulin, and others). While serpins inhibit predominantly serine and cysteine proteases, macroglobulins inhibit also other proteases such as metallo proteases.

There are proteases with lower sensitivity to protease inhibitors then others. A comparatively low sensitivity towards serum inhibitors when comparing it with other human proteases such as trypsin or chymotrypsin has been described for mesotrypsin, a human trypsin variant expressed in the brain and pancreas (Rinderknecht H. et al. Mesotrypsin: A new inhibitor-resistant protease from a zymogen in human pancreatic tissue and fluid. Gastroenterology (1984) 86:681-92). Another example for a protease with comparatively low sensitivity is granzyme B, a serine protease in granules of cytotoxic T-lymphocytes. Kurschus et al. report a 40%-50% residual activity of granzyme B in a solution that corresponds to 80% human serum (Kurschus et al. Killing of target cells by redirected granzyme B in the absence of perforin FEBS Letters (2004) 562:87-92). However, more recent studies have shown that the activity of these proteases in human application matrices containing natural levels of protease inhibitors is not high enough to obtain sufficient activity. And, their specificity is likely to be different from what the application requires.

Besides the therapeutic use, proteases can be used in industrial, cosmetic, diagnostic or synthetic applications.

To qualify as an effective protease, in particular for therapeutic purposes, proteases should have a low sensitivity, preferably they should be essentially insensitive, to activity-modulating substances present in the targeted application matrices. Therapeutic protease should be insensitive towards different activity-modulating substances to a degree that provides an activity level sufficient to effect its indicated function and at the same time must have sufficient specificity to avoid side effects.

**Summary of the Invention**

By a specific screening process novel serine proteases with altered sensitivity to one or more activity-modulating substances were identified. The present invention thus provides

(1) a protease with reduced sensitivity towards activity-modulating substances being derived from a serine protease of the structural class S1 and having one or more mutations at positions selected from the group of positions that correspond structurally or by amino acid sequence homology to the regions or positions 18-28, 34-41, 46-68, 78, 90-102, 110-120, 123-137, 162-186, 195 or 214 in wild-type human cationic trypsin with the amino acid sequence shown in SEQ ID NO:5, or a modified form thereof;
(2) a DNA encoding the protease as defined in (1) above;
(3) a vector comprising the DNA as defined in (2) above;
(4) a cell transformed/transfected with the vector as defined in (3) above and/or containing the DNA as defined in (2) above;
(5) a method for preparing the protease as defined in (1) above, which method comprises cuturing the cell as defined in (4) above 0 and isolating the protease from the culture broth and/or the cell culture;
(6) a pharmaceutical, diagnostic or cosmetic composition comprising the protease as defined in (1) above;
(7) the use of the protease as defined in (1) above for preparing a medicament for preventing or treating a desease curable by protease therapy; and
(8) a method for generating a protease, preferably a protease as defined in (1) above, having reduced sensitivity towards activity-modulating substances present within an application matrix, comprising

(a) providing a library of one or more proteases derived from one or more parent proteases,
(b) contacting the proteases with at least one activity-modulating substance, and
(c) selecting one or more protease variants with reduced sensitivity towards activity-modulating substances as compared to the parent protease(s).

**Brief Description of the Drawings**

Figure 1: *General scheme of the method for screening and selection of proteases with altered sensitivity to activity-modulating substances.* A library of polynucleotides coding for a population of proteases is generated (A), a suitable host is transformed (B), cells are dispensed microtiter plate, and the proteins expressed (C,D). Assay and selection is performed (E,F,G), and improved variants are selected (H) and subjected to a further round of screening and selection (I).

Figure 2: *Scheme detailing the basis for selection and iterative improvement of variants.* The plot shows exemplarily the residual activity of proteases as a function of human blood serum concentration in the protease assay solution. Sigmoidal lines 1 to 9 represent proteases with increasing $IC_{50}$, i.e. less sensitivity towards the inhibitory effect of human serum.

Figure 3: *Schematic representation of different screening strategies.Different* embodiments of the screening strategies are depicted schematically. Horizontal bars represent one component of the application matrix and its length is indicative for the concentration. Hatched bars represent activity-modulating substances of the application matrix.

Figure 4: *Distributions of activity of a protease library screened at two different serum concentrations.* Histograms of the activity distribution are shown for a protease library screened at 20% and 50% serum concentration.

Figure 5: *Determination of serum inhibition in serum for different protease variants.* Residual activities of different protease variants selected according to the method of the invention were measured in a dilution series of human blood serum.

Figure 6: *Determination of $IC_{50}$ values with alpha2-macroglobulin and anti-plasmin.* Residual activity of two protease variants selected according to the method of the invention was measured in a dilution series of alpha2-macroglobulin and antiplasmin.

Figure 7: *Determination of $IC_{50}$ values with anti-plasmin and anti-thrombin.* Determination of residual activity of two

protease variants as in figure 6, except that anti-plasmin and anti-thrombin are used as inhibitors.

Figure 8: *Alignment between human trypsin variants.* TRY1_HUMAN is human cationic trypsin (SEQ ID NO:5), TRY2_HUMAN is human anionic trypsin (trypsin-2 precursor; SEQ ID NO:6) and TRY3_HUMAN is human mesotrypsin (trypsin-3 precursor; SEQ ID NO:7)

## Definitions

In the framework of this invention the following terms and definitions are used. The term **"polynucleotide"** corresponds to any genetic material of any length and any sequence, comprising single-stranded and double-stranded DNA and RNA molecules, including regulatory elements, structural genes, groups of genes, plasmids, whole genomes, and fragments thereof.

The term **"site"** in a polynucleotide or polypeptide refers to a certain position or region in the sequence of the polynucleotide or polypeptide, respectively.

The term **"position"** in a polynucleotide or polypeptide refers to specific single bases or amino acids in the sequence of the polynucleotide or polypeptide, respectively.

The term **"region"** in a polynucleotide or polypeptide refers to stretches of several bases or amino acids in the sequence of the polynucleotide or polypeptide, respectively.

The term **"polypeptide"** comprises proteins such as enzymes, antibodies and the like, medium-length polypeptides such as peptide inhibitors, cytokines and the like, as well as short peptides down to a amino acid sequence length below ten, such as peptidic receptor ligands, peptide hormones, and the like.

The term **"protease**" means any protein molecule catalyzing the hydrolysis of peptide bonds. It includes naturally-occurring proteolytic enzymes, as well as protease variants. It also comprises any fragment of a proteolytic enzyme, or any molecular complex or fusion protein comprising one of the aforementioned proteins. The term **"protease variants**" means any protease molecule obtained by site-directed or random mutagenesis, insertion, deletion, recombination and/or any other protein engineering method, that leads to proteases that differ in their amino acid sequence from the parent protease.

The **"parent protease"** can be either an isolated wild-type protease, or one or more protease variants selected from a library of proteases.

The term **"protease library**" describes at least one protease variant or a mixture of proteases in which every single protease, resp. every protease variant, is encoded by a different polynucleotide sequence.

The term **"gene library"** indicates a library of polynucleotides that encodes the library of proteases.

The term **"isolated"** describes any molecule separated from its natural source.

The term **"specificity"** means the ability of an enzyme to recognize and convert preferentially certain substrates. Specificity can be expressed qualitatively and quantitatively. "Qualitative specificity" refers to the chemical nature of the substrate residues that are recognized by an enzyme. "Quantitative specificity" refers to the number of substrates that are accepted as substrates. Quantitative specificity can be expressed by the term s, which is defined as the negative logarithm of the number of all accepted substrates divided by the number of all possible substrates. Proteases, for example, that accept preferentially a small portion of all possible peptide substrates have a "high specificity". Proteases that accept almost any peptide substrate have a "low specificity".

The term **"catalytic activity"** describes quantitatively the conversion of a given substrate under defined reaction conditions.

The term **"activity-modulating substance"** describes all substances that, when present in the reaction mixture, physically interact with the protease and alter its catalytic activity compared to the activity in the absence of the substance when all other parameters are kept constant. It therefore comprises all modulators, activators and inhibitors of a protease, and all substances that otherwise alter catalytic activity.

The term **"inhibitor"** describes all substances that, when present in the reaction mixture, physically interact with a protease and decrease its catalytic activity compared to the activity in the absence of the substance when all other parameters and concentrations are kept constant.

The term **"activator"** describes all substances that, when present in the reaction mixture, physically interact with a protease and increase its catalytic activity compared to the activity in the absence of the substance when all other parameters and concentrations are kept constant.

The term **"application matrix"** represents all compositions of molecules, fractions or isolated components that the protease is contacted with at the site where activity is required and during its transfer from the site of first contact with the medium assigned for the specific use and the site where activity of the protease is required. A composition of molecules denotes the entirety of molecules, in particular in their respective combinations and concentrations present at a particular point in space and time. The application matrix comprises both activity-modulating substances, in particular inhibitors or activators, and other activity-modulating substances as well as further components.

The term **"compartmentation of samples"** describes the coupling of protease genotype and phenotype by

use of devices or tools that enable compartmentation of samples. The distribution of genotypes, e.g. into sample carriers is done at a multiplicity per compartment that allows sufficient differentiation of phenotypes.

The term **"substrate"** or **"peptide substrate"** means any peptide, oligopeptide, or protein molecule of any amino acid composition, sequence or length, that contains a peptide bond that can be hydrolyzed catalytically by a protease. The peptide bond that is hydrolyzed is referred to as the "cleavage site".

The term **"correspond structurally"** refers to amino acid residues or regions of amino acid residues that are located at equivalent positions when performing either a 3-dimensional alignment of structures of human cationic trypsin and structures of other members of the S1 serine protease class or a one-dimensional sequence alignment of human cationic trypsin with the respective proteases. Particular proteins corresponding structurally with the human cationic trypsin of SEQ ID NO:5 are human anionic trypsin und human mesotrypsin shown in SEQ ID NOs:6 and 7, respectively. The respective alignment is shown in Fig. 8.

The term "**Ki**" defines the affinity of an inhibitor "I" to the enzyme "E". A general kinetic description for a competitive inhibitor is given by the following scheme, whereby "S" indicates the substrate and "p" the product:

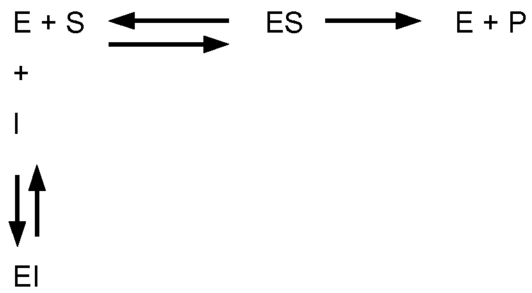


Ki is defined as Ki = [E] [I] / [EI]. It represents the dissociation constant of the inhibitor and the enzyme. A large value for Ki corresponds to a weak inhibitor, a small value represents a strong inhibitor.

The term **"residual activity"** is defined defined as the the ratio of the catalytic activity of the enzyme in the presence of an inhibitor (vi) to the catalytic activity in the absence of the inhibitor (v0), all other parameters being equal. Therefore the residual activity $a_i$ is given by $a_i=v_{i/}v_0$. and $a_i$*100 is the residual activity in percent. From the above scheme a general equation relating the residual activity to the concentrations of inhibitor and substrate as well as to Km and Ki can be derived:

$$\frac{v_i}{v_0} = \frac{K_m + [S]}{K_m\left(1 + \frac{[I]}{K_i}\right) + [S]}$$

where Km = [E] [S] / [ES], Ki = [E] [I] / [EI] and vi/v0 *100 represents to the residual acitvity in percent.

The term **"$IC_{50}$"** is defined as the concentration of activity-modulating substance at which the activity of a protease is reduced to 50% compared to the activity in the absence of the activity-modulating substance, all other parameters and concentrations being equal. In the context of the equation given above this means that [I]= $IC_{50}$ when vi/v0= 1/2:

$$\frac{1}{2} = \frac{K_m + [S]}{K_m\left(1 + \frac{[IC50]}{K_i}\right) + [S]}$$

which can be transformed in the following

way

$$2(K_m + [S]) = K_m + IC50 * \frac{K_m}{K_i} + [S]$$

and

$$K_m + [S] = IC50 * \frac{K_m}{K_i}$$

and

$$IC50 = (K_m + [S]) * \frac{K_i}{K_m} = \left(1 + \frac{[S]}{K_m}\right) * K_i$$

**Detailed Description of the Invention**

As set forth above the present invention provides serine protease variants of the structural class S1 with reduced sensitivity towards activity-modulating substances as present in the application matrix of the protease variant and provides a method for the generation of such proteases. The method can be applied to proteases belonging to any known protease class, or sub-class thereof, namely aspartic, cysteine, serine, metallo and threonine proteases. Preferably the method is applied to serine protease of the structural class S1 as disclosed below in Table 2.

Due to the high degree of structural conservation between S1 proteases the substitutions disclosed here for the human cationic trypsin scaffold, may be transferred to other S1 proteases. Namely, substitutions in other scaffolds of the serine protease class S1 at positions that correspond structurally and/or by sequence homology to the positions and/or substitutions disclosed here for human cationic trypsin that to lead to a decreased sensitivity to inhibitors may have an influence on their respective inhibitor-insensitivity of these other scaffolds.

Library generation

In a preferred embodiment the protease is derived from human trypsin which is sensitive to a variety of inhibitors in the blood, most notably the serpins. Said proteases may have a desired catalytic activity and or substrate specificity but undesired sensitivity to the activity-modulating substances. The invention provides a method to identify and select proteases with a desired change in the sensitivity against said substances.

According to the invention this is achieved by providing a protease library derived from one or more parent proteases with desired catalytic activity, contacting said proteases with at least one activity-modulating substance and selecting one or more protease variants with improved $IC_{50}$ compared to the parent protease(s).

The first step in selecting proteases with reduced sensitivity towards activity-modulating substances is the generation of libraries of polynucleic acids that encode proteases with different genotypes and/or phenotypes. Different strategies of introducing changes in the coding sequences are applied including but not limited to single or multiple point mutations, exchange of single or multiple nucleotide triplets, insertions or deletions of one or more codons, homologeous or heterologeous recombination between different genes, fusion of additional coding sequences at either end of the encoding sequence or insertion of additional encoding sequences or any combination of these methods. The selection of sites to be mutagenized is based on different strategies as detailed in the following embodiments of the invention. The manipulation of the polynucleic acids to implement these strategies are described in the following embodiments of this first step.

In a first embodiment the generation of libraries is based on the comparison of two or more genes that are

different with respect to the sensitivity towards activity-modulating substances. Changes in the gene of interest are then introduced at sites where the amino acid sequences of the two or more proteases differ. The change can result in substitution of one or more amino acids or randomization at these positions or randomization of amino acids one, two or three amino acids upstream and/or downstream from these positions. The same applies to insertions or deletions of one or more amino acids at such positions or any combination of substitution, insertion and deletion.

In a further embodiment the strategy is guided by the analysis of the crystal structure, if available, of the complex between the protease and an activity-modulating substance. The distances between atoms belonging to the protease and those belonging to the activity-modulating substance are analyzed and ranked. In a preferred aspect of this embodiment positions are identified that correspond to amino acids whose atoms have a less than a minimal distance to the closest atom of the activity-modulating substance. Either these positions or amino acids in addition to one, two or three amino acids upstream and/or downstream are randomized, or amino acids are inserted or deleted at these positions or any combination of these changes. The minimal distance of the atoms is less than 10 Å. In a more preferred embodiment the minimal distance is less than 5 Å. If no structure of a complex is available such structure is computer modelled from structures of proteases and/or inhibitors that are related to the proteases and/or inhibitors of interest.

The next embodiment is based on the identification of amino acids which are near the active site and located on the surface of the molecule as preferred sites of mutagenesis. In this embodiment the active site of the protease is identified and a line drawn from the center of mass of the molecule through the center of the active site. A plane perpendicular to this line is approached stepwise from a distant position to the protease towards the open side of the active site. As the plane approaches the protease it will come closer to certain amino acids of the structure. As the plane is approached further it will contact successively more amino acids. The amino acids that are contacted first are the preferred sites for the introduction of mutations. Either these positions or amino acids in addition to one, two or three amino acids upstream and/or downstream are randomized, or amino acids are inserted or deleted at these positions or any combination of these changes.

In another embodiment the sites targeted for the introduction of changes in the gene are random. Such random point mutations are introduced into the gene of interest by means of mutagenic PCR. Depending on the desired mutation spectrum, this can be accomplished either by a method analogous to the protocol of Cadwell and Joyce (Cadwell RC and Joyce GF. Mutagenic PCR PCR Methods and Applications (1994) 3:136-140; Cadwell RC and Joyce GF. Randomization of Genes by PCR Mutagenesis PCR Methods and Applications (1992) 2:28-33), or by the method of Spee et al. (Spee JH et al. Efficient random mutagenesis method with adjustable mutation frequency by use of PCR and dITP Nucleic Acid Research (1993) 3:777-778), or by similar methods or methods derived thereof.

According to a further embodiment, primer extension PCR is utilized to introduce certain changes into a gene basically as described by Ho et al. (Ho SN et al. Site-directed mutagenesis by overlap extension using the polymerase chain reaction Gene (1989) 77:51-59 and Horton RM et al. Engineering hybrid genes without the use of restriction proteases: gene splicing by overlap extension Gene (1989) 77:61-68) or a method derived thereof. The method is applied to mutagenize one or more codons, or to insert one or more codons, or to accomplish complete codon mutagenesis.

In a further embodiment, selective combinatorial randomization (SCR®) is applied for saturating mutagenesis at specific positions within the gene of interest as described in EP 1419248 B1. Using this method, the region to be randomized is determined by a base pair mismatch within a DNA fragment. This can be generated by annealing complementary single strands of different gene variants forming a heteroduplex. The mismatch position is then recognized and selectively randomized.

In the next embodiment, several variants which were generated by a method analogous to one or more of the above embodiments are recombined by recombination chain reaction (RCR®) as described in EP 1230390 B1. Using this method, two largely complementary single strands of different gene variants are annealed. The generated heteroduplex is partially digested by an exonuclease and resynthesized with a polymerase, thus adopting the sequence of one strand into the other one during the extension reaction.

In order to generate enzyme variants with different phenotypes, the libraries of polynucleic acids that encode these different protease variants are translated into proteins by different means.

Therefore, a suitable host cell is transformed with the encoding polynucleic acid and cultivated under appropriate conditions leading to expression and possible secretion of the protease variant. Different organisms may function as hosts including mammalian or non-mammalian cell lines, microbial organisms or viral expression systems. In a preferred embodiment expression is performed in a microbial system such as yeasts, fungi or bacteria. In a preferred embodiment a bacterial host, preferably *Echerichia coli* or *Bacillus subtilis* is used. Alternatively, the expression is performed applying a viral expression system and in a preferred embodiment a viral display system is used. In addition, a further embodiment comprises in-vitro translation and transcription systems that allow the generation of active protein from the polynucleic acid in the absence of any living organism.

In another embodiment the coupling between genotype and phenotype is performed by surface display expression methods. Such methods include, for example, phage or viral display, cell surface display and in vitro display. Phage or viral display typically involves fusion of the protease to a viral/phage protein. Cell surface display, i.e. either

bacterial or eukaryotic cell display, typically involves fusion of the protease to a peptide or protein that is located at the cell surface. In in-vitro display, the protease is typically made in vitro and linked directly or indirectly to the mRNA encoding the protein (DE 19646372 C1). With phage panning as described by Russel et al. (Russel M, Lowman HB, Clackson T. Introduction to phage biology and phage display, In: Clackson T, Lowman HB, editors. Phage display - a practical approach. Oxford: Oxford University Press; 2004:1-26) the protease is displayed a fusion molecule to a phage surface protein, e.g. as N-terminal part of the gIII surface protein of bacteriophage M13. This can be accomplished by fusing a protease gene library to the C-terminal fragment of the gene gIII and inserting this construct into a phagemid vector. After transformation into an Escherichia coli strain phage particles can be obtained by infection with a helper phage. In a preferred embodiment this procedure is performed with a library of enzyme variants wherein all variants have a defined mutation in the active site rendering the proteases catalytically inactive.

The recovery of the polynucleic acid that encodes the protease with the desired properties requires a strict coupling of the genotype with the protein and its phenotype.

In one embodiment this is performed by separating individual transformants of the host cells or individual viruses into isolated compartments of any type followed by cultivation and expression of the protease variants therein. In a preferred embodiment these compartments are given by the individual wells of a micro titer plate, in a more preferred embodiment this is a high-density micro titer plate of any format.

In another embodiment coupling of genotype and phenotype is obtained by in-vitro transcription and translation of individual polynucleic acids isolated in individual compartments which can be represented by the wells of microtiter plates or droplets of water-in-oil, or water-oil-water emulsions (Tawfik DS and Griffiths AD. Man-made cell-like compartments for molecular evolution Nature Biotechnology (1998) 16:652-656; Bernath K et al. In vitro compartmentalization by double emulsions: sorting and gene enrichment by fluorescence activated cell sorting Analytical Biochemistry (2004) 325:151-157).

<u>Selection of proteases</u>

In the second step the multitude of expressed proteases are contacted with the at least one activity-modulating substance. Either simultaneously or consecutively the proteases are contacted with at least one substrate.

The consecutive contact is preferred when preselection of a subset of proteases that interact to a lower extent or do not interact with the activity-modulating substance is required. Therefore, the proteases are contacted first with the at least one activity-modulating substance and preincubated. Proteases that interact to a lower extent or do not interact with the activity-modulating substance are selected within a subset. This subset of proteases is subsequently contacted with the at least one substrate to identify those variants that are catalytically active. The contact with the at least one substrate is performed either alone or in combination with the activity-modulating substance. In contrast simultaneous contact of the proteases with the at least one activity- modulating substance and the at least one substrate allows the direct determination of the catalytic activity in the presence of the activity-modulating substance.

The application matrix and therefore the activity-modulating substances depends on the use of the selected proteases. Proteases can be used in industrial, cosmetic, diagnostic or synthetic applications. For these uses the application matrix is given by the composition of any environment relevant for the industrial process, any composition of a cosmetic product or diagnostic reagent, or compositions used in a synthetic application. In one embodiment the proteases are used in the generation of hydrolysates of protein from different plant or animal sources, such as soy, casein and rice. These contain a significant amount of protease inhibitors, e.g. the soy protease inhibitors, Bowman-Birk protease inhibitors (BBI), or soybean trypsin1 inhibitor (SBTI), which reduce the activity of the processing enzyme. Similarly, proteases are used on bakery to enhance the dough properties. The ingredients of the dough, namely flour, contain inhibitors for proteases and other enzymes. The method of the invention provide proteases with reduced inhibitor sensitivity and favourable process performance.

<u>Applications of proteases</u>

A preferred use of the proteases selected by the method of the present invention is as pharmaceutically active substances that reduce or cure the cause or symptoms of a disease. Depending on the indication for which a pharmaceutical protease is intended to be used, catalytic activity is required at different locations in the body. Intended application matrices for pharmaceutical proteases are human or animal body fluids or cytoplasm of cells. The term "body fluid" is not limited to fluids in the strict sense but to all kind of body matrices, such as mucosa, organelles or entire organs. Preferred body fluids include but are not limited to blood, blood serum, blood plasma, digestive fluids such as intestinal and gastric juice and mucosa, synovial fluid, interstitial fluid, mucosal fluid, peritoneal fluid, extracellular matrix, the eye, cerebrospinal fluid, the brain, different organs as well as epithelial and mucosal surfaces of the body and the intracellular space including cytoplasm or cellular organelles such as lysosomes, endosomes, endoplasmic reticulum, Golgi apparatus, nucleus and mitochondria.

Each of the different application matrices have its particular composition of inhibitors of the enzymatic activity and appropriate proteases with activity in these environments are generated by the method of the invention irrespective of the particular composition of the inhibitors. In a preferred embodiment the proteases are active and insensitive or less sensitive to inhibitors in the blood, synovial fluid or the extracellular matrix.

The compositions of substances that the proteases are contacted with comprise at least one activity-modulating substance or a mixture of several such substances. Activity-modulating substances can either reduce, enhance or otherwise change the catalytic activity of a protease, e.g. they act as inhibitors or activators of the protease, respectively. In a preferred embodiment of the invention the activity-modulating substances are inhibitors which reduce or eliminate the catalytic activity of the protease.

The mechanism of inhibition is different for different inhibitors. Some inhibitors are competitive inhibitors, which reversibly bind to the protease. Other inhibitors bind irreversibly to the protease via a covalent bond or the inhibitors are irreversible by practical standards due to an extremely low binding constant. The invention provides a method for the selection of proteases with reduced inhibitor sensitivity independent of the mechanism of inhibition.

Protease inhibitors in different matrices

Depending on the intended application matrix, the activity-modulating substances include but are not limited to carbohydrates, lipids, fats, polynucleic acids, peptides and proteins as well as all molecules belonging to the metabolism of the organism in which a therapeutic protease is intended to be used or any combination thereof. In a preferred embodiment the activity-modulating substances are polypeptide or protein inhibitors of the enzymatic function. In a more preferred embodiment the one or more activity-modulating substances are selected from the table 1 below.

In a most preferred embodiment the activity-modulating substances are protein inhibitors present in any part of the diseased body for which the protease is intended to be used. These inhibitors include but are not limited to protease inhibitors such as serpins, selected from the group consisting of alpha1-antitrypsin, alpha1-antichymotrypsin, kallistatin, protein C-inhibitor, leucocyte elastase inhibitor, plasminogen activator inhibitor, maspin, serpin B6, megsin, serpin B9, serpin B10, serpin B11, serpin B12, serpin B13, antithrombin, heparin cofactor, plasminogen activator inhibitor, alpha-2-plasmin inhibitor, C1-inhibitor, neuroserpin, serpin I2 and thyroxin-binding globulin; cystein protease inhibitors, selected from the group consisting of cystatin A, cystatin B, cystatin C, cystatin D, cystatin E/M, cystatin F, cystatin S, cystatin SA, cystatin SN, cystatin G, kininogen inhibitor unit 2 and kininogen inhibitor unit 3; metallo protease inhibitors, selected from the group consisting of TIMP-1, TIMP-2, TIMP-3 and TIMP-4; macroglobulins such as alpha2-macroglobulin; BIRC-1, BIRC-2, BIRC-3, BIRC-4, BIRC-5, BIRC-6, BIRC-7 and BIRC-8, and others. Other inhibitors are known to those skilled in the art (Rawling ND et al. Evolutionary families of peptidase inhibitors Biochemistry Journal (2004) 378: 705-716)

Substrates of inhibitor-sensitive proteases

Either simultaneously or consecutively to the contact with the activity-modulating substance the protease variants are contacted with at least one substrate. The substrates include all substances amenable to chemical modification by a protease. These include peptides or proteins as present in the metabolism of an organism. In a preferred embodiment of the invention the substrate is a polypeptide or protein. In a more preferred embodiment the substrate is a protein whose function is relevant for the development of a disease or symptoms. In a most preferred embodiment the protein is a cytokine, such as APRIL, BAFF, BDNF, BMP, CD40-L, EGF, FasL, FGF, Flt3-L, Galectin-3, G-CSF, GM-CSF, IFN-alpha, INF-gamma, IL-1, IL-2, IL-3, IL-4, IL-5, IL-6, IL-7, IL-8, IL-9, IL-10, IL-11, IL-12, IL-13, IL-14, IL-15, IL-16, IL-17, IL-18, IL-19, IL-20, IL-21, IL-22, IL-23, IL-24, IL-25, IL-26, IL-27, IL-28, Leptin, LIGHT, Lymphotactin, M-CSF, MIF, NGF, Oncostatin-M, PDGF, RANKL, RANTES, TGF-alpha, TGF-beta, TNF-alpha, TNF-beta, TRAIL, or VEGF, or their respective receptors.

In addition the combination of at least one activity-modulating substance and at least one substrate may comprise any further number of substances which are neither activity-modulating nor substrate for the enzymatic activity (non-target molecules). These compositions include but are not limited to all substances of the application matrix of the protease or rather the entire application matrix which already comprises the at least one activity-modulating substance.

Compositions of matrices for screening

In a third step proteases with reduced sensitivity against the activity-modulating substances are selected. These proteases constitute the parent proteases for the generation of new libraries of protease variants that are subjected again to the selection process. Different compositions of activity-modulating substances are optionally contacted with the variety of proteases. These are sketched in figure 3 and described in more detail below.

In a first embodiment of this step the concentration of the substances that the variety of proteases are contacted with in the step before is the same as the concentration of substances that are present in the application matrix. This

embodiment can be applied when the parent protease has a residual activity that can reliably be measured in the presence of activity-modulating substances at the concentration of the application matrix. For example proteases are contacted with 100% serum, a substrate molecule and more active variants are selected. The complete method of the present invention when iteratively applied leads to variants with a higher activity in the presence of 100% serum than the starting protease.

In another embodiment the concentration of the activity-modulating substances is equivalent to the concentration of substances in the application matrix and thus the activity may be changed to a level that is outside the dynamic range of the assay format applied. This embodiment provides an approach applicable under these conditions. In such a case the protease variants are contacted with a dilution of the composition of substances in order to reduce the activity-modulating capacity of the composition to an extend that allows the activity of the proteases to be measured within the dynamic range of the assay. In a preferred aspect of this embodiment the dilution leads to concentration of the composition substances in the assay that corresponds to less than 100% of the concentration in the application matrix, more preferred to concentrations of 99%, 90%, 80%, 70%, 60%, 50%, 40%, 30%, 20%, 10%, 5%, 1%, 0.1% or less or any concentration in between and most preferred to concentrations from 70% to 5%. Improved variants that are selected at such at reduced concentration represent the basis for the generation of a new library of proteases. These proteases are subsequently contacted with a composition of substances at a concentration higher than the concentration applied to screen the parent proteases. In this iterative process the concentration of the composition of substances including the activity-modulating substances is increased stepwise in each round of the method. This generates proteases with gradually improved properties and allows screening to be performed under conditions where the residual activity of the proteases is within the dynamic range of the assay even in the presence of activity-modulating substances.

In a further embodiment the concentrations of all substances are increased beyond the concentration present in the application matrix, preferably to 101%, 110%, 120%, 150%, 200%, 300% or more or any concentration in between, more preferably from 120% to 200%. This embodiment provides a means to increase the selective pressure where the activity of proteases is measurable in the presence of 100% of the concentration of substances. In a preferred aspect of this embodiment the concentration of substances is increased stepwise over several cycles beyond the concentration of substances as they occur in the application matrix. In a more preferred aspect the concentrations of serum components in the assay correspond to 101%, 110%, 120%, 150%, 180%, 200%, 250% or 300% or any concentration in between. Most preferably the concentrations range from 120% to 200% of serum.

In the next embodiment the composition of substances present in the application matrix is selectively depleted of one or more of the activity-modulating substances while the concentrations of all other substances remain unchanged. The extend of depletion is adjusted in order to perform selection of proteases under conditions where the activity-modulating capacity of the composition of substances is reduced to a level that allows the enzymatic activity to fall within the dynamic range of the assay. In following rounds of the iterative optimization process the depletion of said components is reduced stepwise until the full concentration is reached. In a preferred aspect of this embodiment serum is depleted of protease inhibitors by one of several means including but not limited to standard chromatographic procedures such as affinity chromatography to reduce selectively the concentration of protease inhibitors. In one embodiment, molecules with high affinity for the inhibitor, the concentration of which needs to be lowered, are attached to a solid phase. These molecules include but are not limited to antibodies and proteases. In a next step the application matrix is contacted with the immobilized molecule, e.g. either in a batch mode, or a flow column. Alternatively, e.g. serum is incubated with a known amount of a serine protease such as trypsin, chymotrypsin, subtilisin or others which will react with and thereby reduce the concentration of inhibitors such as serpins, in particular alpha1-antitrypsin, antithrombin, antiplasmin and others. In successive rounds of optimization the amount of depletion of the activity-modulating substances is decreased stepwise, or it can be replenished at increasing levels.

In a further embodiment one or several of the components of the application matrix are enriched compared to the concentration of the application matrix. The relative enrichment is increased in successive rounds of screening to provide enhanced selection pressure. The enrichment factor is 101%, 110%, 120%, 150%, 180%, 200%, 250% or 300% of the concentration being present in the application matrix or any concentration in between. Preferred enrichment factors range from 120% to 200% of the concentration.

In a further embodiment the variety of proteases is contacted with isolated activity-modulating substances or a mixture thereof. The concentration of said activity-modulating substances are lower, equal or higher than the concentration of the respective substances in the application matrix. In a preferred aspect of this embodiment the activity-modulating substances are protease inhibitors. In a more preferred aspect the inhibitors applied are alpha1-anti-trypsin, anti-thrombin, anti-plasmin or alpha2-macroglobulin or any combination thereof. The concentrations vary from 200% down to 1% of the concentration present in serum.

<u>Use of display technology for screening</u>

All of the embodiments detailed above may optionally include a pre-incubation step of the proteases with the

activity-modulating substances for different lengths of time. In a preferred embodiment of the invention this pre-incubation time is 1 s, 1 min, 10 min, 30 min, 60 min, 2 h, 4 h, 6 h, 12 h, 24 h, 48 h, 72 h or longer, or any time in between. In a more preferred embodiment of the invention the pre-incubation time is 30min, 60min, 2h or 4h.

In another embodiment the population of proteases is displayed using phage panning. In order to identify proteases with altered sensitivity to activity-modulating substances, the library of phage particles is subject to incubation with one or more activity-modulating substances. Simultaneously or thereafter, the phage suspension is incubated with the substrate. Therefore, the substrate is coupled to a solid phase as is well known in the art for typical phage display targets, e.g. on latex beads, on living cells, in whole organisms or tissues, in multi-well plates, in "immuno-tubes", on a column matrix or a number of other known formats. Such interactions rely on physico-chemical protein-surface interactions. The substrate can also be associated with other substances, but remain in the solution phase. Either type of interaction (solution or solid phase) rely on adsorption or on affinity interactions. There are a number of well known affinity interactions such as binding to specific antibodies, e.g. an anti-target antibody, or specific protein-protein interactions, e.g. antibody-protein A interactions. In a preferred aspect the affinity interactions are mediated by a peptide or chemical "tag" that is added to the target as a peptide fusion such as addition of a (his)6-tag, biotin binding peptide or a FLAG tag, or by post-translational conjugation such as addition of a biotin tag by chemical conjugation.

Phage particles presenting a protease which is insensitive or less sensitive to the activity-modulating substance, and possessing specificity for the substrate, bind to the substrate. Where the substrate is in solution (with or without additional associated substances), the substrate is then immobilized by direct capture of the target substrate or indirectly by capture of the associated substance. Where the substrate is bound to a solid phase before panning, this last immobilization step is unnecessary. Once the phage-substrate complex is immobilized, other phages that display more sensitive proteases or display no proteases or display proteases which are less specific, are depleted by washing. Typical washing include washing with solutions of adapted temperature and pH, containing low, medium or high salt, detergent, competitor protein or substrate, competitor phage, and other components. Washings are either done manually or automated, are done continuously or in discrete steps, and can involve changing the washing buffer composition as the washing progresses. The desired phages and thus the desired proteases are thus enriched on the solid phase. The phage is lysed and the encoding DNA recovered, e.g. by cloning or PCR amplification, or the phage is released from the solid phase, e.g., by acid washing, cleavage of the phage away from the protease or other means known in the art and used to infect a host cell for biological amplification. Other specialized selection schemes, such as the use of "selectively infective phage" where the substrate is bound to a ligand needed for host cell infection can also be used in the phage display enrichment of proteases with lowered sensitivity to activity-modulating substances.

In one aspect of this embodiment, protease variants, each with a defined mutation in the active site rendering the enzyme variants catalytically inactive, are displayed on a phage and selection is performed by panning on immobilised peptid substrates after incubation of the phages with the activity-modulating substances. In a more preferred embodiment such activity-modulating substances are protease inhibitors such as serpins, e.g. alpha1-antitrypsin, antithrombin and antiplasmin, or macroglobulins, e.g. alpha2-macroglobulin.

In another aspect of this embodiment one or more activity-modulating substances are covalently linked to a solid phase such as latex beads, living cells, whole organisms or tissues, multi-well plates, "immuno-tubes", column matrix or other known solid phases. Then, the phage suspension is incubated with this activity-modulating substance, which preferentially binds phages presenting a protease variant susceptible to the activity-modulating substance and which preferentially leaves the insensitive or less sensitive variants unbound. In a further aspect of this embodiment the activity-modulating substances are left in the solution phase, either alone or associated with other substances in analogy to linkages and associations mentioned above. Then, the activity-modulating substances is immobilized by direct capture of the activity-modulating substances or indirectly by capture of the associated substance. Where the activity-modulating substance was bound to a solid phase before panning, this last immobilization step is unnecessary. The immobilization of the phage-activity-modulating substance complex will preferentially immobilize phage which display proteases that are more sensitive to activity-modulating substances. The non-bound phage, which are enriched for phage displaying proteases that are resistant to activity-modulating substances, are captured by recovering the fluid supernatant. In a preferred aspect this step is repeated once or several times. It is well known in the art that the density of the activity-modulating substances can be a critical parameter. Methods to modify the density or concentration of capture molecules are well known in the art. This "depletion" of undesired phage leaves the supernatant enriched for phage displaying proteases with lowered sensitivity to activity-modulating substances. The two aspects can be also combined, whereby a fraction of the activity-modulating substances is covalently linked and another fraction is left in the solution phase. The ultimately recovered phage are lysed and the encoding DNA recovered, e.g., by cloning or PCR amplification, or the phage are used to infect a host cell for biological amplification.

<u>High-throughput screening of protease variants</u>

Testing of the enzymatic properties is performed in a screening format where variants of the proteases are

tested with respect to catalytic activity. In a preferred embodiment the screen is performed in a parallel high-throughput fashion in a miniaturized format in assay volumes less than 1ml. In a more preferred embodiment the volume is less than 100μl, for example 80μl, 60μl, 40μl, 20μl, or 10μl or any volume in between. In an most preferred embodiment the (well-based) assay volume is less than 10μl, for example 8μl, 6μl, 4μl, 2μl or 1μl or any volume in between. In a further embodiment of the invention the screening volume is less than 1μl, namely 800nl, 600nl, 400nl, 200nl, 100nl or any volume in between.

In a preferred embodiment the coupling between phenotype and genotype is achieved by distributing individual cells of the transformed host into separated compartments. In a preferred embodiment the compartments are represented by the wells of a micro-titer plate. Variants of the enzymes are expressed in the compartments and contacted with activity-modulating substances and substrate. Activity is measured and variants with improved properties are selected.

Detection of the enzymatic activity is performed by measuring a physical change accompanied with the modification of the substrate. Changes introduced in the molecule by the modification include changes in activity, size, structure, composition, mass, reactivity, binding characteristics, or chemical properties such as solubility, acidity, color or fluorescence. A change in some of said properties can be measured indirectly by the incorporation of a chemical label into the substrate which changes its properties in response to the enzymatic conversion. In a preferred embodiment one or two fluorescent labels are covalently coupled to the substrate molecule. Substrate conversion is reflected in the change in one or several parameters of the fluorescence such as intensity, anisotropy, fluorescence lifetime, diffusion coefficient, fluorescence energy transfer, fluorescence intensity distribution, fluorescence coincidence analysis or cross-correlation. In a more preferred embodiment the substrate is covalently coupled with a fluorescent label in such a way that proteolytic cleavage leads to a change in the fluorescence anisotropy (EP 1307482). In a most preferred embodiment the proteolytic cleavage of the substrate is monitored by the accompanied loss of biological activity in a cell-based assay. In another preferred embodiment of the invention detection of cleavage of the substrate is performed by separation and detection of proteolytic fragments by chromatography, such as HPLC.

In another embodiment of the invention the method further comprises the step of selecting for protease variants having substantially similar or higher specificity with regard to the substrate as compared to the parent protease(s). Alternatively the protease variants obtained in step (c) may be modified as to exhibit a catalytic activity of defined specificity, whereby said defined specificity is not or only to a smaller extent occurring in the parent protease and/or the protease variants obtained in step (c). By such additional steps proteases are provided which have a defined specificity for therapeutic, research, diagnostic, nutritional, personal care or industrial purposes. Defined specificity means that the proteases are provided with specificities that do not exist in naturally occurring proteases. The specificities can be chosen by the user so that one or more intended target substrates are preferentially recognized and converted by the proteases. The specificity of proteases, i.e. their ability to recognize and hydrolyze preferentially certain peptide substrates, can be expressed qualitatively and quantitatively. Qualitative specificity refers to the kind of amino acid residues that are accepted by a protease at certain positions of the peptide substrate. For example, trypsin and t-PA are related with respect to their qualitative specificity, since both of them require at the P1 position an arginine or a similar residue. On the other hand, quantitative specificity refers to the relative number of peptide substrates that are accepted as substrates by the protease, or more precisely, to the relative $k_{cat}/k_M$ ratios of the protease for the different peptides that are accepted by the protease. Proteases that accept only a small portion of all possible peptides have a high specificity, whereas the specificity of proteases that, as an extreme, cleave any peptide substrate would theoretically be zero. WO 2004/113521 provides a method for the generation and identification of proteases with desired specificities based on the combination of a protease scaffold that provides the general catalytic activity with variable specificity determining regions (SDRs) which provide the basis for the discrimination between different targets. In addition, the proteases can be fused either on DNA level or chemically to a binding module, e.g. a receptor fragment, an antibody domain or a fragment thereof, to address the target molecule. Furthermore, different mutagenesis methods can be employed to engineer specific proteases, e.g. single or multiple site-directed or random mutagenesis or transfer of amino acid residues or sequence stretches from one protease sequence to another. In another approach the specificity is generated by rational design.

Before subjected to a further round of screening selected variants may optionally be characterized in more detail with respect to the improved properties. In a preferred embodiment of the invention the $IC_{50}$ of the variant is determined by incubating it with a serial dilution of the composition of activity-modulating substances and measuring the residual activity.

## Determination of the primary structure of protease variants

In a preferred embodiment of the invention, protease variants with the desired properties in terms of activity, inhibitor insensitivity or any other property are identified in a screening process as described above. The result of the screening process is a culture of a clone of the organism expressing the protease variant of interest. From this culture deoxyribonucleic acid (DNA) sequence coding for said protease variant can be extracted by standard molecular cloning techniques known to anyone skilled in the art (e.g. Sambrook, J.F; Fritsch, E.F.; Maniatis, T.; Cold Spring Harbor

Laboratory Press, Second Edition, 1989, New York). Isolation and cloning of the gene into suitable vectors allows the determination of the sequence of the deoxyribonucleic acid and thereby the amino acid sequence of the encoded protease by standard techniques.

Description of inhibitor-insenstive protease variants

The scaffold of the parent protease preferably belongs to the class of S1-serine proteases. Preferably the protease is derived from a trypsin-like protease, more preferably is derived from a human trypsin such as human cationic trypsin, human anionic trypsin and human mesotrypsin, most preferably the protease is derived from human cationic trypsin with the amino acid sequence shown in SEQ ID NO: 5. As set forth in (1) above the protease has one or more mutations at positions that correspond structurally or by amino acid sequence homology to the regions 18-28, 34-41, 46-68, 90-102, 110-120, 123-137 and 162-186, 195 and 214 in human cationic trypsin. It is preferred that the protease has one or more mutations at one or more positions selected from the group of positions that correspond structurally or by amino acid sequence homology to the regions 20-26, 36-39, 51-59, 63-67, 78, 92-99, 112-118, 124-128, 131-134, 172-184, 195 and 214 in human trypsin. It is more preferred that the protease has one or more mutations corresponding to the following positions in human trypsin: 21, 22, 23, 24, 28, 37, 39, 46, 52, 55, 56, 57, 64, 66, 67, 78, 92, 93, 98, 99, 112, 115, 118, 124, 125, 128, 131, 133, 163, 172, 174, 181, 183, 184, 195 and 214, and most preferred at one or more of the following positions 22, 23, 24, 37, 52, 57, 64 and 133.

Even more preferably the protease has one or more mutations at positions that correspond structurally or by amino acid sequence homology to the positions:

G at position 21 is substituted by A, D, S or V, preferably D or V;
Y at position 22 is substituted by T, H, Q, S, W, G or A, preferably by T or H;
H at position 23 is substituted by T, N, G, D, R or Y, preferably by T or N;
F at position 24 is substituted by I, V, Q, T, L or A, preferably by I or V;
S at position 28 is substituted by A;
S at position 37 is substituted by T;
G at position 39 is substituted by S;
I at position 46 is substituted by V, N, L or T, preferably by V;
E at position 52 is substituted by V or M, preferably by V;
N at position 54 is substituted by S;
I at position 55 is substituted by T, N or R, preferably by T or N;
E at position 56 is substituted by G or R, preferably by G;
V at position 57 is substituted by A, T or G, preferably by A;
F at position 64 is substituted by I or T, preferably by I;
N at position 66 is substituted by D;
A at position 67 is substituted by V;
R at position 78 is substituted by W;
S at position 92 is substituted by T;
R at position 93 is substituted by P;
A at position 98 is substituted by D;
R at position 99 is substituted by H;
T at position 112 is substituted by A or P, preferably by A;
K at position 115 is substituted by M;
I at position 118 is substituted by V;
T at position 124 is substituted by K or I, preferably by K;
A at position 125 is substituted by P or S, preferably by P;
G at position 128 is substituted by R, K or T, preferably by R;
Y at position 131 is substituted by F, N or H, preferably by F;
D at position 133 is substituted by G;
V at position 163 is substituted by A;
S at position 172 is substituted by T;
Q at position 174 is substituted by R;
V at position 181 is substituted by A;
C at position 183 is substituted by H, Q or R, preferably by H;
N at position 184 is substituted by K or D;
D at position 195 is substituted by E; and/or
K at position 214 is substituted by E, D, R, T, or V, preferably by E

Most preferable is a trypsin mutant of SEQ ID NO:5 having one of the following combination of mutations:

S37T, E52V, E56G, V57A, F64I, R78W, D133G, C183H (variant A); or
Y22T, H23T, F24I, S37T, E52V, E56G, V57A, F64I, R78W, D133G, C183H (variant B); or
Y22H, F24V, S37T, E52V, E56G, V57A, F64I, R78W, D133G, C183H (variant C); or
Y22T, H23T, F24I, S37T, E52V, I55N, E56G, V57A, L58A, E59Q, F64T, R78W, R93P, T124K, A125P, G128R, Y131H, D133G, L135V, D139N, V163A, C183H, D195E, D214E (variant E); or
G21D, Y22T, H23T, F24I, S28A, S37T, E52V, N54S, I55T, E56G, V57A, F64I, R78W, R93P, R99H, T124K, A125P, D133G, V163A, C183H, D195E, K214E (variant F); or
G21V, Y22T, H23T, F24I, S28A, S37T, E52M, N54S, I55T, E56R, V57A, F64I, R78W, S92T, R93P, A98D, R99H, T112A, T124K, A125P, D133G, V163A, S172T, C183Q, D195E, K214E (variant G); or
G21D, Y22T, H23T, F24I, S28A, S37T, G39S, I46T, E52M, N54S, I55T, E56G, V57A, F64I, A67V, R78W, S92T, R93P, A98D, R99H, T112A, K115M, I118V, T124K, A125P, D133G, V163A, S172T, V181A, C183Q, N184D, D195E, K214E (variant D);

The numbering in all of the described mutations refers to SEQ ID NO:5, i.e. human cationic trypsin. The variants are those prepared in the Experimental Section of the application.

Expression of protease variants

In order to express the proteases of the invention, the DNA encoding such protease is ligated into a suitable expression vector by standard molecular cloning techniques (e.g. Sambrook, J.F; Fritsch, E.F.; Maniatis, T.; Cold Spring Harbor Laboratory Press, Second Edition, 1989, New York). The vector is introduced in a suitable expression host cell that expresses the corresponding protease. Particularly suitable expression hosts are bacterial expression hosts such as Escherichia coli, Pseudomonas fluorescence or Bacillus subtilis, or yeast expression hosts such as Saccharomyces cerevisiae, Kluveromyces lactis, Hansenula polymorpha or Pichia pastoris, other fungal expression hosts such as Aspergillus niger or Trichoderma reesei or mammalian expression hosts such as mouse (e.g., NS0), Chinese Hamster Ovary (CHO) or Baby Hamster Kidney (BHK) cell lines, transgenic mammalian systems such as rabbit, goat or cattle, other eukaryotic hosts such as insect cells or viral expression systems such as bacteriophages like M13, T7 phage or Lambda, or viruses such as vaccinia and baculovirus expression systems.

Often, the DNA is ligated into an expression vector behind a suitable signal sequence that leads to secretion of the protease into the extracellular space, thereby allowing direct detection of enzyme activity in the cell supernatant. Particularly suitable signal sequences for Escherichia coli, other Gram negative bacteria and other organisms known in the art include those that drive expression of the HlyA, DsbA, PhoA, PelB, OmpA, OmpT or M13 phage GIII genes. For Bacillus subtilis, particularly suitable signal sequences include those that drive expression of the AprE, NprB, Mpr, AmyA, AmyE, Blac, SacB, and for S. cerevisiae or other yeast, include the killer toxin, Bar1, Suc2, Matα, Inu1A or Ggplp signal sequence.

Alternatively, the enzyme variants are expressed intracellularly. As an alternative, after intracellular expression of the enzyme variants, or secretion into the periplasmatic space using signal sequences such as those mentioned above, a permeabilisation or lysis step is used to release the protease into the supernatant. The disruption of the membrane barrier is effected by the use of mechanical means such as ultrasonic waves, French press, cavitation or the use of membrane-digesting enzymes such as lysozyme.

As a further alternative, the genes encoding the protease are expressed cell-free by the use of a suitable cell-free expression system. For example, the S30 extract from Escherichia coli cells is used for this purpose as described by Lesly et al. (Methods in Molecular Biology 37 (1995) 265-278). In cell-free systems, the gene of interest is typically transcribed with the assistance of a promoter, but ligation to form a circular expression vector is optional. Regardless of the presence of a circular vector or the final host organism, the DNA sequence of the protease expression construct is determined using techniques that are standard in the art.

Purification of protease variants

As described above, the identified proteases are expressed in a variety of expression systems and the appropriate down-stream processing and purification procedures are selected accordingly. In a preferred embodiment of the invention the protease variant is expressed in a microbial host and the protein is secreted into the periplasmic or extracellular space. Cells carrying an appropriate expressing construct for the protease variants may be preserved as cryo stocks, well known to anyone skilled in the art. Cultures for protein expression are inoculated from a cryo stock and the volume of the culture increased successively in the appropriate container. In a preferred embodiment the cells are grown in a fermenter under controlled conditions of pH, temperature, oxygen and nutrient supply. After harvesting a first step

comprises the separation of cells from supernatant using one or more of several techniques, such as sedimentation, microfiltration, centrifugation, flocculation or other. In a preferred embodiment the method applied is microfiltration.

In a preferred embodiment of the invention the protein is secreted into the supernatant and a further step of purification comprises the concentration of the supernatant by ultrafiltration. Protein purification from the supernatant or concentrated supernatant is performed with one or more of several preferred chromatographic methods including but not limited to ion-exchange, hydrophobic interaction, hydroxyapatite, size fractionation by gel-filtration and affinity chromatography or any combination thereof. In a more preferred method the protein is purified by combining several ion-exchange chromatographic steps to obtain a high purity protein. An even more preferred method comprises the combination of a cation-exchange and an anion-exchange chromatography, optionally combined with further cation or anion-exchange chromatographies. An appropriate purification method yields a purity of the protein of >50%, in a more preferred method the purity is >80%, in an even more preferred method the purity is >90%, in a yet more preferred method the purity is >95% and in a most preferred method the purity is >98%.

Derivatives of protease variants

To further improve the properties of the protease for the intended application it may optionally be subjected to a variety of modifications which include but are not limited to:

a) fusion to a peptidic component, preferably being selected from, but not limited to the group consisting of binding domains, receptors, antibodies, regulatory domains, pro-sequences, serum albumin, or fragments or derivatives thereof, and/or
b) covalent conjugation to a natural or synthetic polypeptide or non-polypeptide moiety, preferably being selected from the group consisting of polyethylenglycols, carbohydrates, lipids, fatty acids, nucleic acids, metals, metal chelates, nanoparticles, liposomes, dendrimers or fragments or derivatives thereof, and/or
c) introduction of consensus glycosylation sites into the protease variant that are glycosylated during the post-translational processing of the protease variant during biosynthesis and/or
d) introduction of one or more mutation, insertion, substitution or deletion that render the protease variant less sensitive to clearance of inactivation mechanisms such as, but not limited to, proteolytic degradation, induction of immunogenicity or receptor mediated cellular uptake and/or
e) incorporation into formulations and/or drug delivery devices for protection and slow release.

Use of protease variants

The protease variants selected by the method can be used in industrial, cosmetic, diagnostic or synthetic applications. A preferred application of the proteases is the use as therapeutics to reduce or cure the cause or symptoms of a disease which can be prevented or treated by a protease therapy. Indications where a protease therapy is beneficial for the patient include inflammation and autoimmune diseases, cancer, cardiovascular diseases, neurodegenerative diseases, allergies, host-versus-graft disease, bacterial or viral infections, metabolic disorders or any other diseases where a protease therapy is indicated. Preferred embodiments of the present invention comprise proteases with beneficial activity for the indications of cancer, inflammation and autoimmune diseases. A more preferred application is in chronic inflammation. Different types of arthritis, rheumatoid arthritis, osteoarthritis, Sjörgen's syndrome, systemic lupus erythematosus, ankylsing spondylitis, psoriasis, inflammatory bowel diseases, Crohn's disease and ulcerative collitis all belong to this area and even today there is a constant need for effective drugs to treat these conditions. In a particularly preferred embodiment the application is rheumatoid arthritis, inflammatory bowel diseases, psoriasis, Crohn's disease, Ulcerative colitis, diabetes type II, classical Hodgkin's Lymphoma (cHL), Grave's disease, Hashimoto's thyroiditis, Sjogren's Syndrome, systemic lupus erythematosus, multiple sclerosis, Systemic inflammatory response syndrome (SIRS) which leads to distant organ damage and multiple organ dysfunction syndrome (MODS), eosinophilia, neurodegenerative disease, stroke, closed head injury, encephalitis, CNS disorders, asthma, rheumatoid arthritis, sepsis, vasodilation, intravascular coagulation and multiple organ failure, as well as other diseases connected with hTNF-alpha.

Several combinations of the above described embodiments can be defined leading to particular useful variants of the method of the invention. It is understood that the embodiments described herein are for illustrative purposes only and that various modifications or changes in light thereof will be suggested to persons skilled in the art. The invention is further illustrated by the following examples which should not be construed as limiting. The content of all publications, patents, and patent applications cited herein are hereby incorporated by reference.

**Detailed Description of the Drawings**

Figure 1: *General scheme of the method for screening and selection of proteases with altered sensitivity to activity-modulating substances.* A library of polynucleotides coding for a population of proteases is generated from a parent molecule (A). A suitable host is transformed with the polynucleotides (B), cells are dispensed into compartments of a microtiter plate, and the proteins are expressed (C,D). Activity-modulating substances and substrate are added, the activity is measured (E,F,G), and improved variants are selected (H). The improved proteases may represent the basis for a new library of proteases which are subjected to a further round of screening and selection.

Figure 2: *Scheme detailing the basis for selection and iterative improvement of variants.* The plot shows exemplarily the residual activity of proteases as a function of human blood serum concentration in the protease assay solution. Sigmoidal lines 1 to 9 represent proteases with increasing $IC_{50}$, i.e. less sensitivity towards the inhibitory effect of human serum. In the example, if the parent protease used to generate the first library has a residual activity at 100% serum that is sufficient to be measured (variant 6; line B), the screen can be performed directly at 100% serum. Improved variants such as number 7 show twice the activity of the parent protease and can be used for the generation of the next library and so forth. Screening and selection is along line C in figure 2. Alternatively, if the residual activity of the parent protease is only measurable at a dilution of 1% (variant 1 and 2; line A) the screen is performed at this low concentration. This first round of screening and selection may yield variant 4 which has measurable activity at 10% serum and therefore screening of the library based on variant 4 can be performed at this higher concentration. In successive rounds the serum concentration n is increased stepwise until variants with the desired properties are obtained.

Figure 3: *Schematic representation of different screening strategies.* Different embodiments of the screening strategies are depicted schematically. Horizontal bars represent one component of the application matrix and its length is indicative for the concentration. Hatched bars represent activity-modulating substances of the application matrix. I. Screening is performed at concentrations representing 100% of the concentration in the application matrix, for each component. II. All components are present at higher concentration compared to the application matrix and the concentrations are increased in successive rounds. III. All components are present at lower concentration compared to the application matrix and the concentrations are increased in successive rounds IV. Individual components of the application matrix are enriched selectively, and the concentration is successively increased. V. Only one or a few of the components of the application matrix are used in the screen, with successively increasing concentrations. VI. Individual components of the application matrix are depleted compared to the concentration in the application matrix. VII. Each component of the application matrix is present at a certain concentration, be it lower, equal or higher than the corresponding concentration in the application matrix.

Figure 4: *Distributions of activity of a protease library screened at two different serum concentrations.* Histograms of the activity distribution are shown for a protease library screened at 20% and 50% serum concentration. Under both conditions the activity of the library is clearly distinguished from the negative control. Under less stringent conditions (20% serum) the library distribution shows more activity than under more stringent conditions of (50% serum). Variants with the highest activities are selected for further improvement.

Figure 5: *Determination of serum inhibition in serum for different protease variants.* Residual activities of different protease variants selected according to the method of the invention were measured in a dilution series of human blood serum. The residual activity was normalized to the uninhibited value and plotted as a function of the serum concentration. The serum concentration at which the activity is 50% corresponds to the $IC_{50}$ value. The $IC_{50}$ values increase from variant A to D demonstrating progressive reduction of sensitivity to serum inhibitors.

Figure 6: *Determination of $IC_{50}$ values with alpha2-macroglobulin and anti-plasmin.* Residual activity of two protease variants selected according to the method of the invention was measured in a dilution series of alpha2-macroglobulin and antiplasmin, two prominent inhibitors in human blood serum. The maximum concentration of 100% corresponds to the average concentration of the inhibitor in human blood serum, i.e. approximately 1.5 mg/ml for alpha2-macroglobulin and 70 μg/ml for antiplasmin, respectively. The residual activity was normalized to the uninhibited value and plotted as a function of the inhibitor concentration. The inhibitor concentration at which the activity is 50% is the $IC_{50}$ value. While both variants are relatively insensitive towards alpha2-macroglobulin, sensitivity against antiplasmin is markedly reduced in variant E.

Figure 7: *Determination of $IC_{50}$ values with anti-plasmin and anti-thrombin.* As in figure 6, residual activity of two protease variants selected according to the method of the invention was measured, except that using anti-plasmin and anti-thrombin was used as inhibitors. 100% corresponds to 70 μg/ml antiplasmin and 230 μg/ml of anti-thrombin. Variant G and F show a further increased insensitivity towards anti-plasmin compared to variant E and a high insensitivity towards anti-thrombin.

Figure 8: *CLUSTAL W (1.7) multiple sequence alignment between human trypsin variants.* It is shown human cationic trypsin (SEQ ID NO:5; top), human Anionic trypsin (Trypsin-2 precursor; SEQ ID NO:6; middle) and human Mesotrypsin (Trypsin-3 precursor, SEQ ID NO:7; bottom). * matching position.

## Experimental Section

Example 1: PCR mutagenesis and library generation

Random mutagenesis is done by a variation of the standard PCR purification protocol. A PCR reaction is set up in total volume of 100 µl containing a final concentration of 10mM Tris/HCl pH 8.3, 50 mM KCl, 0.01% (wt/vol) gelatin, 7 mM $MgCl_2$, 0.5 mM $MnCl_2$, 0.2 mM dATP and dGTP, 1 mM dCTP and TTP, 0.3 µM of primers P1 and P2, 5 fmol template DNA and 2.5 units Taq polymerase. The following PCR program is used: 95 °C 1:00 / 95 °C 0:30 / 68°C 0:30 / 72°C 1:00 with 30 PCR cycles from step four to step two.

Alternatively, mutagenesis at specific sites is done by another variation of the standard PCR protocol. Therefore, two sets of primers are used: primers P1 and P3 binding to 5' and 3' ends of the gene and primers P2 and P4 binding to the site that is to be mutagenited and containing at specific positions mixtures of the four nucleotides. For the first round of PCR, two separate reactions are set up in a total volume of 100 µl, each containing 1 x PCR-Buffer (KOD-Puffer), 0.2 mM dNTPs, 2 mM $MgSO_4$, 0.3 µM of each primer, 20 ng template DNA and 2.5 units KOD polymerase. The following PCR program is used: 94 °C 2:00 / 94 °C 0:30 / 55 °C 0:30 / 68 °C 0:45 / 6 °C for ever with 22 PCR cycles from step four to step two. To generate the first fragment, primers P1 and P3 are employed whereas primers P2 and P4 are used for the second fragment. Consecutively, the two PCR products are separated from remaining template DNA by preparative agarose gel electrophoresis and purified using a gel purification kit (Qiagen). The two PCR products are mixed in an equimolar ratio with a total amount of 100 ng and serves as a template for a extension reaction carried out in a reaction mixture essentially analogous to the one above, with the terminal primers P1 and P2.

Primer:

P1: TGGCAGGAGGGGCCACTCAGGCCTTTGCA (SEQ ID NO: 1)
P2: CACCTAGTGGCCTAGTCGGCCTTAGC (SEQ ID NO:2)
P3: GATGATCTGCTCATTCCCCTCCAAGGCTCCMNNMNNGTGCACTCCCAGTCTCAC (SEQ ID NO:3)
P4: GGGAATGAGCAGATCATC (SEQ ID NO:4)

2 µg of the generated PCR fragment are digested with restriction endonucleases. In a similar approach, 8 µg of a standard plasmid for introducing genes into Bacillus subtilis plasmid (Palva I. et al. Secretion of Eschirichia coli beta-lactamase from Bacillus subtilis by the aid of alpha-amylase signal sequence. Cell Biology (1982) 79:5582-5586) are cut with restrictionendonucleases and dephosphorylated with CIAP. The digest of the plasmid DNA is heated to 50 °C followed by phenol-extraction. Finally, the PCR fragment and the plasmid DNA are both purified. Ligation is carried out over night at 16 °C according to the manufacturer's instructions (MBI fermentas). After heat-inactivation at 65 °C for ten minutes the DNA is subjected to ethanol precipitation, dried and transformed into Escherichia coli cells. The transformed cells are suspended in LB medium and grown over night at 37 °C in a shake flask incubator. The plasmid DNA of the generated library is then purified and transformed into Bacillus subtilis according to the protocol of Spizizen (Spizizen J. Transformation of biochemically deficient strains of Bacillus subtilis by deoxyribonucleate. Proc. Natl. Acad. Sci. US (1958) 44:1072-1978) for assessing the individual variants.

Example 2: Screening and selection

The principle lying behind the selection and screening strategy is illustrated in figure 2. The plot shows the residual activity of variants as a function of serum concentration in the assay solution. Sigmoidal lines 1 to 9 represent variants with increasing $IC_{50}$, i.e. less sensitivity towards the inhibitory effect of serum. If the parent enzyme used to generate the first library has a residual activity at 100% serum that is sufficient to be measured (variant 6??), the screen can be performed directly at full serum concentration. Improved variants such as number 7? show twice the activity of the parent and can be used for the generation of the next library and so forth. Screening and selection is along line C in figure 2. Alternatively, if the residual activity of the parent enzyme is only measurable at a dilution of 1% (variant 1 and 2; line A) the screen is performed at this low concentration. This first round of screening and selection may yield variant 4? which has measurable activity at 10% serum and therefore screening of the library based on variant 4? can be performed at this higher concentration. In successive rounds the serum concentration is increased stepwise until variants with the desired properties are obtained.

In order to identify enzyme variants having the desired substrate specificity, a screening approach based on a confocal fluorescence spectroscopy set-up as disclosed in WO 9416313 was used. A cell suspension of a Bacillus subtilis in culture medium was dispensed at a cfu-concentration ensuring that single cells are dispensed in each well of the microtiter plate. Cultures are grown over night at 37 °C and protein is secreted into the supernatent. Serum is added in a dilution so that the final concentration in the assay allows detection of the enzymatic activity. After adding the

substrate (TNF-alpha covalently labelled with a fluorophore ) to the sample and incubation for a certain period of time, the samples were subjected to measurement by confocal fluorescence spectroscopy. If necessary, this procedure was repeated several times in order to measure kinetics of the proteolytic cleavage. Samples were ranked according to proteolytic activity, and samples exceeding a certain activity threshold were identified in order to isolate the gene encoding the corresponding protease variant. The distribution of proteolytic activities of protease variants at different concentrations of serum obtained by this procedure is shown in figure 4.

Example 3: Acquisition of specific protease genes and determination of DNA and protein sequence after library screening

The screening of a library that contained a diverse population of candidate protease enzymes, each individually harboured on the expression plasmid, pBSX-G3-Zero, resulted in specific candidates improved in the examined characteristics, e.g. inhibitor resistance. Since the individual members of the library were tested as individual cultures, the result of the screening process was the identification of specific cultures that express putatively improved protease genes. The best candidate culture was plated to result in single colonies on solid LB-agar plates containing neomycin for selection (20 μg/ml). A resulting isolated colony was inoculated into liquid LB medium containing neomycin for selection (20 μg/ml), and plasmid DNA was prepared by Zymoprep kit (Zymo Research). The resulting plasmid DNA was transformed into *E. coli* strain XL1-blue using standard methods, amplified by cell growth in Luria-Burtani solid and liquid medium supplemented with 20 micrograms/ml of neomycin and isolated by Qiagen kit. DNA sequencing reactions were generated using the resulting plasmid DNA with a commercial kit (GenomeLab DTCS Quick Start Kit, Beckman Coulter) and the improved protease gene sequence determined using the CEQ 2000XL DNA Analysis System (Beckman Coulter). The DNA sequence of a candidate improved protease gene was unambiguously determined by this method and the resulting DNA sequence of the protease gene, through application of the standard genetic code for nuclear genes (see Sambrook, J.F; Fritsch, E.F.; Maniatis, T.; Cold Spring Harbor Laboratory Press, Second Edition, 1989, New York), unambiguously determined the amino acid sequence of the encoded protease protein.

Example 4: Determination of $IC_{50}$'s in human serum

A more detailed characterization of the inhibitor sensitivity is obtained by the determination of $IC_{50}$'s of different variants. The $IC_{50}$ is the concentration of inhibitor at which the residual activity is reduced to 50% of the uninhibited value. The enzymes are incubated at a concentration of 1.5 μg/ml in PBS / pH 7.4 / 0.1% Triton-X-100 with a serial dilution of human serum in the same buffer, yielding the indicated final concentration. Fluorescently labelled TNF-alpha is added and proteolytic cleavage is followed over time at 37°C by measuring changes in fluorescence parameters. The residual activity is normalized to the uninhibited value and plotted against the serum concentration (figure 5). Clearly, the $IC_{50}$ values of variants A through D ? obtained in successive rounds of optimization increase, therefore the variants are less sensitive to the inhibitors present in human serum.

Example 5: Determination of $ICS_{50}$'s of individual inhibitors

Human serum contains a large number of different protease inhibitors which cannot be differentiated in the experiment described in example 3. To identify inhibitors to which the enzymes are particularly sensitive these can be tested individual. The concentrations of the different inhibitors in the serum vary considerably, between 70 μg/ml for anti-plasmin, 180 μg/ml C1-inhibitor, 230 μg/ml anti-thrombin and 1.5 mg/ml for anti-trypsin and alpha2-macroglobulin. These concentrations are defined as 100% and the enzymes are incubated with dilution series' of the individual inhibitors. Fluorescently labelled TNF-alpha is added and proteolytic cleavage is followed over time at 37 °C by measuring changes in fluorescence parameters. The residual activity is normalized to the uninhibited value and plotted against the concentration of the inhibitors. Figure 6 shows that the two variants C and E ? are both relatively insensitive to alpha2-macroglobulin even at the high concentration of about 1.5 mg/ml. However, sensitivity is clearly different towards anti-plasmin. While variant D is 50% inhibited at a concentration that corresponds to about 5% serum, the value for variant E is on the order of 40% serum equivalent. Figure 7 shows the insensitivity of variants G and F ? towards antiplasmin which is further increased compared to variant E. In addition, variants G anf F are fully insensitive to anti-thrombin at a concentration equivalent to 25% of the serum concentration. This demonstrates the success of the screening and selection procedure applied.

Table 1: Activity-modulating substances

| Code | Inhibitor name |
|---|---|
| I01.001 | ovomucoid inhibitor unit 1 |

(continued)

| Code | Inhibitor name |
|---|---|
| I01.002 | ovomucoid inhibitor unit 2 |
| I01.003 | ovomucoid inhibitor unit 3 |
| I01.004 | ovoinhibitor inhibitor unit 1 |
| I01.005 | ovoinhibitor inhibitor unit 2 |
| I01.006 | ovoinhibitor inhibitor unit 3 |
| I01.007 | ovoinhibitor inhibitor unit 4 |
| I01.008 | ovoinhibitor inhibitor unit 5 |
| I01.009 | ovoinhibitor inhibitor unit 6 |
| I01.010 | ovoinhibitor inhibitor unit 7 |
| I01.011 | SPINK1 |
| I01.012 | SPINK2 |
| I01.013 | SPINK5 inhibitor unit 1 |
| I01.014 | BUSI-I inhibitor |
| I01.015 | BUSI-II inhibitor |
| I01.016 | bikazin salivary inhibitor inhibitor unit 1 |
| I01.017 | bikazin salivary inhibitor inhibitor unit 2 |
| I01.018 | elastase inhibitor (Anemonia sulcata) |
| I01.019 | rhodniin inhibitor unit 1 |
| I01.020 | bdellin |
| I01.021 | tryptase inhibitor (Hirudo medicinalis) |
| I01.022 | dipetalogastin inhibitor unit 2 |
| I01.023 | dipetalogastin inhibitor unit 3 |
| I01.024 | TgPI inhibitor inhibitor unit 1 (Toxoplasma gondii) |
| I01.025 | TgPI inhibitor inhibitor unit 2 (Toxoplasma gondii) |
| I01.026 | TgPI inhibitor inhibitor unit 3 (Toxoplasma gondii) |
| I01.027 | TgPI inhibitor inhibitor unit 4 (Toxoplasma gondii) |
| I01.028 | SPINK5 inhibitor unit 2 |
| I01.029 | SPINK5 inhibitor unit 3 |
| I01.030 | SPINK5 inhibitor unit 4 |
| I01.031 | rhodniin inhibitor unit 2 |
| I01.032 | SPINK5 inhibitor unit 6 |
| I01.033 | NcPI-S protein (Neospora caninum) |
| I01.034 | EPI1 protein inhibitor domain (Phytophthora infestans) |
| I01.035 | skin protein 1 (Phyllomedusa sauvagii) |
| I01.036 | dipetalogastin inhibitor unit 1 |
| I01.037 | RECK protein inhibitor unit 1 |
| I01.038 | infestin 4 |
| I01.039 | PAPI I inhibitor unit (Pacifastacus leniusculus) |

(continued)

| Code | Inhibitor name |
|---|---|
| I02.001 | aprotinin |
| I02.002 | spleen trypsin inhibitor I (Bos taurus) |
| I02.003 | colostrum trypsin inhibitor (Bos taurus) |
| I02.004 | serum basic peptidase inhibitor (Bos taurus) |
| I02.005 | bikunin inhibitor unit 1 |
| I02.006 | bikunin inhibitor unit 2 |
| I02.007 | hepatocyte growth factor activator inhibitor 1 inhibitor unit 1 |
| I02.008 | hepatocyte growth factor activator inhibitor 1 inhibitor unit 2 |
| I02.009 | hepatocyte growth factor activator inhibitor 2 inhibitor unit 1 |
| I02.010 | hepatocyte growth factor activator inhibitor 2 inhibitor unit 2 |
| I02.011 | tissue factor pathway inhibitor-1 inhibitor unit K1 |
| I02.012 | tissue factor pathway inhibitor-1 inhibitor unit K2 |
| I02.013 | tissue factor pathway inhibitor-2 inhibitor unit K1 |
| I02.014 | tissue factor pathway inhibitor-2 inhibitor unit K2 |
| I02.015 | protease nexin II |
| I02.016 | amyloid-like protein 2 |
| I02.017 | peptidase inhibitor (Tachypleus) |
| I02.018 | chymotrypsin inhibitor SCI-I (Bombyx mori) |
| I02.019 | paragonial peptide D (Drosophila funebris) |
| I02.020 | boophilin inhibitor unit 1 |
| I02.021 | boophilin inhibitor unit 2 |
| I02.022 | chelonianin inhibitor unit 1 |
| I02.023 | carrapatin |
| I02.024 | ornithodorin inhibitor unit 1 |
| I02.025 | ixolaris inhibitor unit |
| I02.026 | peptidase inhibitor 5 (Anemonia sulcata) |
| I02.032 | ornithodorin inhibitor unit 2 |
| I02.033 | WFIKKN peptidase inhibitor inhibitor unit 2 |
| I02.034 | Ac-KPI-1 I (Ancylostoma caninum) inhibitor unit |
| I02.035 | savignin (Ornithodoros savignyi) |
| I02.037 | Kil-1 g.p. (Drosophila virilis) |
| I02.039 | amblin inhibitor unit (Amblyomma hebraeum) |
| I03.001 | soybean trypsin inhibitor |
| I03.002 | cathepsin D inhibitor (Solanum tuberosum) |
| I03.003 | trypsin/chymotrypsin inhibitor (Alocasia macrorrhiza) |
| I03.004 | alpha-amylase/subtilisin inhibitor (Hordeum vulgare) |
| I03.005 | chymotrypsin inhibitor ECI (Erythrina variegata) |
| I03.006 | proteinase inhibitor A (Sagittaria sagittifolia) inhibitor unit |

(continued)

| Code | Inhibitor name |
|---|---|
| I03.007 | proteinase inhibitor B (Sagittaria sagittifolia) inhibitor unit |
| I03.008 | trypsin inhibitor (Enterolobium contortisiliquum) |
| I03.009 | winged-bean chymotrypsin inhibitor |
| I03.010 | trypsin inhibitor (Acacia confusa) |
| I03.011 | erythrina trypsin/tissue plasminogen activator inhibitor |
| I03.012 | cruzipain inhibitor (Bauhinia bauhinioides) |
| I03.013 | sporamin |
| I03.015 | AtDr4 g.p. (Arabidopsis thaliana) |
| I03.016 | bauhinia trypsin/plasma kallikrein inhibitor |
| I03.017 | cysteine protease inhibitor 1 (potato) |
| I03.018 | trypsin inhibitor PtTI (Populus tremuloides) |
| I03.019 | papain inhibitor (Prosopis juliflora) |
| I03.020 | potato Kunitz-type trypsin inhibitor |
| I03.021 | Kunitz serine peptidase inhibitor (Delonix regia) |
| I03.022 | latex serine peptidase inhibitor (Papaya carica) |
| I04.001 | alpha-1-peptidase inhibitor |
| I04.002 | alpha-1-antichymotrypsin |
| I04.003 | kallistatin |
| I04.004 | protein C inhibitor |
| I04.005 | protein Z-dependent peptidase inhibitor |
| I04.006 | serpin B1 |
| I04.007 | plasminogen activator inhibitor 2 |
| I04.008 | squamous cell carcinoma antigen 1 |
| I04.009 | squamous cell carcinoma antigen 2 |
| I04.010 | maspin |
| I04.011 | serpin B6 |
| I04.012 | megsin |
| I04.013 | serpin B8 |
| I04.014 | serpin B9 |
| I04.015 | serpin B10 |
| I04.016 | serpin B12 |
| I04.017 | serpin B13 |
| I04.018 | antithrombin |
| I04.019 | heparin cofactor II |
| I04.020 | plasminogen activator inhibitor-1 |
| I04.021 | protease nexin I |
| I04.022 | pigment epithelium derived factor |
| I04.023 | alpha-2-plasmin inhibitor |

(continued)

| Code | Inhibitor name |
|---|---|
| I04.024 | C1 inhibitor |
| I04.025 | neuroserpin |
| I04.026 | serpin I2 |
| I04.027 | endopin 1 (Bos taurus) |
| I04.028 | viral serpin |
| I04.029 | contrapsin |
| I04.030 | peptidase inhibitor 3 (rodent) |
| I04.031 | alaserpin (Lepidoptera) |
| I04.032 | barley-type serpin |
| I04.033 | myeloid and erythroid nuclear termination stage-specific protein (Gallus gallus) |
| I04.034 | endopin 2 (Bos taurus) |
| I04.035 | colligin 1 |
| I04.036 | colligin 2 |
| I04.037 | thermopin (Thermobifida fusca) |
| I04.038 | PAP-regulating serpin (insect) |
| I04.039 | serpin SP6 (Drosophila melanogaster) |
| I04.040 | serpin Spn43Ac (Drosophila melanogaster) |
| I04.041 | serpin SRP-2 (Caenorhabditis elegans) |
| I04.042 | serpinb3b (Mus musculus) |
| I04.043 | serpin 4 (Drosophila melanogaster) |
| I04.044 | serpin SPI-C1 (Mus musculus) |
| I04.045 | serpin SPI2 (Mus musculus) |
| I04.952 | Homologue: serpin A2 |
| I04.953 | Homologue: angiotensinogen |
| I04.954 | Homologue: corticosteroid-binding globulin |
| I04.955 | Homologue: thyroxin-binding globulin |
| I04.955 | Homologue: Homologue: thyroxin-binding globulin |
| I04.955 | Homologue: Homologue: Homologue: thyroxin-binding globulin |
| I04.955 | Homologue: Homologue: Homologue: Homologue: thyroxin-binding globulin |
| I04.956 | Homologue: serpin B11 |
| I04.958 | Homologue: ovalbumin |
| I05.001 | ascidian trypsin inhibitor |
| I06.001 | maize trypsin/factor XIIa inhibitor |
| I06.002 | barley trypsin/factor XIIa inhibitor |
| I06.003 | ragi seed trypsin/alpha-amylase inhibitor |
| I06.004 | wheat trypsin/alpha-amylase inhibitor |
| I07.001 | trypsin inhibitor MCTI-1 (Momordica charantia) |
| I07.002 | elastase inhibitor MCEI (Momordica charantia) |

(continued)

| Code | Inhibitor name |
|---|---|
| I07.003 | trypsin inhibitor EETI-II (Ecballium elaterium) |
| I07.004 | macrocyclic trypsin inhibitor (Momordica cochinchinensis) |
| I07.005 | trypsin inhibitor CMTI-I (Cucurbita maxima) |
| I07.006 | trypsin inhibitor CPTI (Cucurbita pepo) |
| I07.007 | trypsin inhibitor CMTI-III (Cucurbita maxima) |
| I07.008 | trypsin inhibitor MCTI-II (Momordica charantia) |
| I07.009 | trypsin inhibitor CVTI-I (Cucurbitaceae) |
| I07.010 | trypsin inhibitor TGTI-I (Luffa cylindrica) |
| I07.011 | trypsin inhibitor TGTI-II (Luffa cylindrica) |
| I07.012 | trypsin inhibitor LCTI (Luffa cylindrica) |
| I07.013 | trypsin inhibitor CMTI-II (Cucumis melo) |
| I07.014 | trypsin inhibitor CSTI-IIb (Cucumis sativus) |
| I07.015 | trypsin inhibitor CSTI-IV (Cucumis sativus) |
| I07.016 | trypsin inhibitor BDTI-II (Bryonia dioica) |
| I07.017 | trypsin inhibitor MRTI-I (Momordica repens) |
| I07.018 | trypsin inhibitor MCTI-A (Momordica charantia) |
| I07.019 | trypsin inhibitor CMeTI-B (Cucumis melo) |
| I07.020 | trypsin inhibitor TTII (Trichosanthes kirilowii) |
| I07.021 | trypsin inhibitor MCTI-III (Momordica charantia) |
| I08.001 | chymotrypsin/elastase inhibitor (Ascaris-type) |
| I08.002 | Acp62F protein (Drosophila melanogaster) |
| I08.003 | Bombina trypsin inhibitor |
| I08.004 | hookworm coagulation inhibitor |
| I08.005 | coagulation inhibitor (Anisakis simplex) |
| I08.006 | inducible metallopeptidase inhibitor (Galleria mellonella) |
| I08.007 | Ascaris trypsin inhibitor |
| I08.008 | cathepsin G/chymotrypsin inhibitor (Apis mellifera) |
| I08.950 | Homologue: von Willebrand factor |
| I08.951 | Homologue: mucin |
| I08.952 | Homologue: mucin 6 |
| I08.953 | Homologue: mucin 5B |
| I09.001 | subtilisin propeptide |
| I09.002 | peptidase A inhibitor 1 (Pleurotus ostreatus) |
| I09.003 | endopeptidase B inhibitor (fungus) |
| I10.001 | marinostatin |
| I11.001 | ecotin |
| I12.001 | lima bean-type trypsin inhibitor |
| I12.002 | sunflower cyclic trypsin inhibitor |

(continued)

| Code | Inhibitor name |
|---|---|
| I12.003 | Bowman-Birk trypsin inhibitor (Medicago-type) |
| I12.004 | Bowman-Birk elastase and trypsin inhibitor (Phaseolus-type) |
| I12.005 | Bowman-Birk inhibitor (Glycine-type) unit 1 |
| I12.006 | Bowman-Birk trypsin/chymotrypsin inhibitor (Arachis hypogaea) |
| I12.007 | rice Bowman-Birk inhibitor inhibitor unit 1 |
| I12.008 | Bowman-Birk inhibitor (Glycine-type) unit 2 |
| I12.009 | Bowman-Birk inhibitor (Gramineae) inhibitor unit 1 |
| I12.010 | Bowman-Birk inhibitor (Gramineae) inhibitor unit 2 |
| I12.011 | rice Bowman-Birk trypsin inhibitor inhibitor unit 2 |
| I12.012 | rice Bowman-Birk trypsin inhibitor inhibitor unit 3 |
| I12.013 | rice Bowman-Birk trypsin inhibitor inhibitor unit 4 |
| I12.014 | bromelain inhibitor (Ananas comosus) |
| I12.015 | wheat-germ trypsin inhibitor (Triticum aestivum) inhibitor unit 1 |
| I12.016 | wheat-germ trypsin inhibitor (Triticum aestivum) inhibitor unit 2 |
| I13.001 | eglin C |
| I13.002 | potato peptidase inhibitor I |
| I13.003 | chymotrypsin inhibitor 2 |
| I13.004 | glutamyl endopeptidase II inhibitor (bitter gourd) |
| I13.005 | subtilisin-chymotrypsin inhibitor CI-1A (barley) |
| I13.006 | chymotrypsin inhibitor I (potato) |
| I13.007 | subtilisin inhibitor I (Fabaceae) |
| I13.008 | inhibitor of trypsin and Hageman factor (Cucurbita maxima) |
| I13.009 | trypsin/subtilisin inhibitor (Amaranthus sp.) |
| I13.010 | tomato peptidase inhibitor I |
| I13.011 | buckwheat peptidase inhibitor I |
| I13.012 | wheat subtilisin/chymotrypsin inhibitor |
| I13.013 | cytin B chain (Theromyzon tessulatum) |
| I14.001 | hirudin |
| I14.002 | haemadin |
| I15.001 | hirustasin |
| I15.002 | bdellastasin |
| I15.003 | theromin (Theromyzon tessulatum) |
| I15.004 | tessulin |
| I15.005 | guamerin (Hirudo nipponia) |
| I15.006 | therin |
| I15.007 | antistasin inhibitor unit 1 |
| I15.008 | antistasin inhibitor unit 2 |
| I15.009 | ghilanten inhibitor unit 1 |

(continued)

| Code | Inhibitor name |
|---|---|
| I15.010 | ghilanten inhibitor unit 2 |
| I15.011 | cytin A chain (Theromyzon tessulatum) |
| I16.001 | plasminostreptin |
| I16.002 | kexstatin I |
| I16.003 | streptomyces subtilisin inhibitor |
| I16.004 | SIL1 inhibitor (Streptomyces cacaoi) |
| I16.005 | SIL8 inhibitor |
| I16.006 | trypsin inhibitor STI1 (Streptomyces sp.) |
| I17.001 | mucus peptidase inhibitor inhibitor unit 2 |
| I17.002 | elafin |
| I17.003 | huWAP2 |
| I17.004 | chelonianin inhibitor unit 2 |
| I17.950 | Homologue: mucus peptidase inhibitor inhibitor unit 1 |
| I18.001 | mustard trypsin inhibitor |
| I18.002 | rape trypsin inhibitor |
| I19.001 | peptidase inhibitor LMPI (Orthoptera) inhibitor unit 1 |
| I19.002 | pacifastin inhibitor unit 1 |
| I19.003 | pacifastin inhibitor unit 2 |
| I19.004 | pacifastin inhibitor unit 3 |
| I19.005 | pacifastin inhibitor unit 4 |
| I19.006 | pacifastin inhibitor unit 5 |
| I19.007 | pacifastin inhibitor unit 6 |
| I19.008 | pacifastin inhibitor unit 7 |
| I19.009 | pacifastin inhibitor unit 8 |
| I19.010 | pacifastin inhibitor unit 9 |
| I19.011 | peptidase inhibitor LGPI (Orthoptera) inhibitor unit 2 |
| I20.001 | potato peptidase inhibitor II inhibitor unit |
| I20.002 | tobacco peptidase inhibitor II inhibitor unit |
| I20.003 | tomato peptidase inhibitor II inhibitor unit |
| I20.004 | serine peptidase inhibitor II (Capsicum-type) |
| I21.001 | secretogranin V |
| I24.001 | pinA Lon peptidase inhibitor (phage T4) |
| I25.001 | cystatin A |
| I25.003 | cystatin B |
| I25.004 | cystatin C |
| I25.005 | cystatin D |
| I25.006 | cystatin E/M |
| I25.007 | cystatin F |

(continued)

| Code | Inhibitor name |
|---|---|
| I25.008 | cystatin S |
| I25.009 | cystatin SA |
| I25.010 | cystatin SN |
| I25.011 | ovocystatin |
| I25.012 | snake venom cystatin (Bitis sp.) |
| I25.013 | sarcocystatin |
| I25.014 | phytocystatin |
| I25.015 | potato multicystatin inhibitor unit |
| I25.016 | kininogen inhibitor unit 2 |
| I25.017 | kininogen inhibitor unit 3 |
| I25.018 | T-kininogen inhibitor unit 2 |
| I25.019 | T-kininogen inhibitor unit 3 |
| 125.020 | alpha-2-HS-glycoprotein inhibitor unit 1 |
| I25.021 | alpha-2-HS-glycoprotein inhibitor unit 2 |
| I25.022 | histidine-rich glycoprotein inhibitor unit 1 |
| I25.023 | cystatin SC |
| I25.024 | cystatin TE-1 |
| I25.025 | histidine-rich glycoprotein inhibitor unit 2 |
| I25.026 | metallopeptidase inhibitor (snake venom) |
| I25.027 | cystatin G |
| I25.028 | oryzacystatin II |
| I25.029 | sunflower multicystatin inhibitor unit |
| I25.030 | papaya cystatin |
| I25.031 | onchocystatin |
| I25.950 | Homologue: kininogen inhibitor unit 1 |
| I27.001 | calpastatin inhibitor unit 1 |
| I27.002 | calpastatin inhibitor unit 2 |
| I27.003 | calpastatin inhibitor unit 3 |
| I27.004 | calpastatin inhibitor unit 4 |
| I29.001 | cathepsin L propeptide |
| I29.002 | cytotoxic T-lymphocyte antigen-2 alpha |
| I29.003 | cathepsin H propeptide |
| I29.004 | cathepsin S propeptide |
| I29.005 | Bombyx cysteine peptidase inhibitor |
| I29.006 | salarin inhibitor unit |
| I29.007 | cathepsin K propeptide |
| I29.008 | cytotoxic T-lymphocyte antigen-2 beta |
| I29.009 | cer g.p. (Drosophila melanogaster) |

(continued)

| Code | Inhibitor name |
|---|---|
| I31.001 | chum salmon egg cysteine peptidase inhibitor |
| I31.002 | MHC II invariant chain p41 form |
| I31.003 | equistatin (Actinia) inhibitor unit 1 |
| I31.004 | equistatin (Actinia) inhibitor unit 2 |
| I31.005 | equistatin (Actinia) inhibitor unit 3 |
| I31.006 | testican-1 |
| I31.950 | Homologue: thyroglobulin |
| I31.951 | Homologue: insulin-like growth factor binding protein |
| I31.952 | Homologue: insulin-like growth factor binding protein 3 |
| I31.953 | Homologue: insulin-like growth factor binding protein 2 |
| I32.001 | BIRC-1 protein |
| I32.002 | BIRC-2 protein |
| I32.003 | BIRC-3 protein |
| I32.004 | X-linked inhibitor of apoptosis protein |
| I32.005 | BIRC-5 protein |
| I32.006 | BIRC-6 protein |
| I32.007 | BIRC-7 protein |
| I32.008 | BIRC-8 protein |
| I32.009 | DIAPI (Drosophila melanogaster) |
| I33.001 | aspin |
| I34.001 | saccharopepsin inhibitor |
| I35.001 | timp-1 |
| I35.002 | timp-2 |
| I35.003 | timp-3 |
| I35.004 | timp-4 |
| I35.005 | timp-DM (Drosophila melanogaster) |
| I36.001 | Streptomyces metallopeptidase inhibitor |
| I37.001 | potato carboxypeptidase inhibitor |
| I38.001 | metallopeptidase inhibitor Erwinia |
| I38.002 | aprin |
| I38.003 | serralysin inhibitor (Serratia sp.) |
| I39.001 | alpha-2-macroglobulin |
| I39.002 | ovomacroglobulin |
| I39.003 | pregnancy-zone protein |
| I39.004 | murinoglobulin 1 |
| I39.005 | murinoglobulin 2 |
| I39.006 | antigen CD109 |
| I39.950 | Homologue: complement component C3 |

(continued)

| Code | Inhibitor name |
|---|---|
| I39.951 | Homologue: complement component C4 |
| I39.952 | Homologue: complement component C5 |
| I40.001 | Bombyx subtilisin inhibitor |
| I42.001 | chagasin |
| I43.001 | oprin |
| I44.001 | carboxypeptidase A inhibitor (Ascaris suum) |
| I46.001 | leech carboxypeptidase inhibitor |
| I47.001 | latexin |
| I48.001 | clitocypin |
| I49.001 | proSAAS |
| I50.001 | baculovirus p35 caspase inhibitor |
| I50.002 | baculovirus p49 caspase inhibitor |
| I51.001 | carboxypeptidase Y inhibitor (Saccharomyces cerevisiae) |
| I51.002 | phosphatidylethanolamine-binding protein |
| I52.001 | tick anticoagulant peptide (Ornithodorus sp.) |
| I57.001 | staphostatin B |
| I58.001 | staphostatin A |
| I59.001 | triabin |
| I63.001 | pro-eosinophil major basic protein |
| I64.001 | thrombostasin (Haematobia irritans) |
| LI01-001 | ovomucoid |
| LI01-002 | ovoinhibitor |
| LI01-003 | bikazin |
| LI01-004 | SPINK5 g.p. (Homo sapiens) |
| LI01-005 | inhibitor TgPI (Toxoplasma gondii) |
| LI01-006 | dipetalogastin |
| LI01-007 | rhodniin |
| LI01-008 | infestin (Triatoma infestans) |
| LI01-009 | PAPI I inhibitor (Pacifastacus leniusculus) |
| LI02-001 | bikunin |
| LI02-002 | tissue factor pathway inhibitor 1 |
| LI02-003 | tissue factor pathway inhibitor 2 |
| LI02-004 | hepatocyte growth factor activator inhibitor type 1 |
| LI02-005 | hepatocyte growth factor activator inhibitor type 2 |
| LI02-006 | boophilin |
| LI02-007 | ixolaris (Ixodes scapularis) |
| LI02-010 | amblin (Amblyomma hebraeum) |
| LI03-001 | proteinase inhibitor B (Sagittaria sagittifolia) |

(continued)

| Code | Inhibitor name |
|---|---|
| LI12-001 | compound inhibitor: 112.005, 112.008 |
| LI12-002 | compound inhibitor: 112.009, 112.010, 112.010, I12 unassigned |
| LI12-004 | rice Bowman-Birk inhibitor |
| LI12-005 | compound inhibitor: I12.015, I12.016 |
| LI12-UPW | unassigned compound peptidase inhibitor containing family I12 units |
| LI15-002 | antistasin |
| LI15-003 | ghilanten |
| LI17-001 | mucus peptidase inhibitor |
| LI19-001 | pacifastin |
| LI20-002 | tobacco type 2 peptidase inhibitor |
| LI20-003 | tomato type 2 peptidase inhibitor |
| LI25-001 | multicystatin (potato) |
| LI25-002 | L-kininogen |
| LI25-003 | T-kininogen |
| LI25-005 | histidine-rich glycoprotein |
| LI25-006 | sunflower multicystatin |
| LI27-001 | calpastatin |
| LI31-001 | equistatin |
| LI90-001 | WFIKKN peptidase inhibitor |
| LI90-002 | WFIKKNRP putative peptidase inhibitor |
| LI90-004 | eppin |

Table 2: Proteasen

| CODE | Protease name |
|---|---|
| A01.001 | pepsin A |
| A01.002 | pepsin B |
| A01.003 | gastricsin |
| A01.004 | memapsin-2 |
| A01.006 | chymosin |
| A01.007 | renin |
| A01.008 | renin-2 |
| A01.009 | cathepsin D |
| A01.010 | cathepsin E |
| A01.011 | penicillopepsin |
| A01.012 | rhizopuspepsin |
| A01.013 | mucorpepsin |
| A01.014 | candidapepsin SAP1 |
| A01.015 | barrierpepsin |

(continued)

| CODE | Protease name |
|---|---|
| A01.016 | aspergillopepsin I |
| A01.017 | endothiapepsin |
| A01.018 | saccharopepsin |
| A01.019 | polyporopepsin |
| A01.020 | phytepsin |
| A01.021 | plasmepsin (Plasmodium sp.) |
| A01.022 | plasmepsin 1 |
| A01.023 | plasmepsin 2 |
| A01.025 | peptidase E |
| A01.026 | peptidase F |
| A01.027 | trichodermapepsin |
| A01.028 | embryonic pepsin (Gallus gallus) |
| A01.029 | neurosporapepsin |
| A01.030 | yapsin 1 |
| A01.031 | yapsin 2 |
| A01.035 | yapsin 3 |
| A01.036 | acid peptidase (Yarrowia lipolytica) |
| A01.037 | canditropsin |
| A01.038 | candiparapsin |
| A01.040 | nepenthesin |
| A01.041 | memapsin-1 |
| A01.042 | syncephapepsin |
| A01.043 | histoaspartic peptidase (Plasmodium falciparum) |
| A01.044 | podosporapepsin |
| A01.045 | nothepsin |
| A01.046 | napsin A (human-type) |
| A01.049 | napsin A (mouse-type) |
| A01.050 | CND41 peptidase |
| A01.051 | pepsin F |
| A01.053 | nemepsin-3 |
| A01.056 | Yps1 protein (Schizosaccharomyces pombe) |
| A01.058 | eimepsin |
| A01.059 | plasmepsin 4 |
| A01.060 | candidapepsin SAP2 |
| A01.061 | candidapepsin SAP3 |
| A01.062 | candidapepsin SAP4 |
| A01.063 | candidapepsin SAP5 |
| A01.064 | candidapepsin SAP6 |

(continued)

| CODE | Protease name |
|---|---|
| A01.065 | candidapepsin SAP7 |
| A01.066 | candidapepsin SAP8 |
| A01.067 | candidapepsin SAP9 |
| A01.068 | nemepsin-2 |
| A01.069 | CDR1 g.p. (Arabidopsis thaliana) |
| A01.070 | pepsin A4 (Homo sapiens) |
| A01.071 | pepsin A5 (Homo sapiens) |
| A01.072 | oryzepsin |
| A01.073 | nucellin |
| A01.074 | AtASP38 peptidase (Arabidopsis thaliana) |
| A02.001 | HIV-1 retropepsin |
| A02.002 | HIV-2 retropepsin |
| A02.003 | simian immunodeficiency virus retropepsin |
| A02.004 | equine infectious anaemia virus retropepsin |
| A02.005 | bovine immunodeficiency virus retropepsin |
| A02.006 | Visna lentivirus-type retropepsin |
| A02.007 | feline immunodeficiency virus retropepsin |
| A02.008 | Moloney murine leukemia virus-type retropepsin |
| A02.009 | Mason-Pfizer leukemia virus retropepsin |
| A02.010 | mouse mammary tumor virus retropepsin |
| A02.011 | human endogenous retrovirus K retropepsin |
| A02.012 | retropepsin (human T-cell leukemia virus) |
| A02.013 | bovine leukemia virus retropepsin |
| A02.015 | Rous sarcoma virus retropepsin |
| A02.016 | intracisternal A-particle retropepsin |
| A02.018 | simian T-cell lymphotropic virus retropepsin |
| A02.019 | multiple-sclerosis-associated retrovirus retropepsin |
| A02.020 | porcine endogenous retrovirus endopeptidase |
| A02.021 | Gypsy transposon (Drosophila sp.) endopeptidase |
| A02.022 | Ty3 transposon (Saccharomyces cerevisiae) endopeptidase |
| A02.024 | rabbit endogenous retrovirus endopeptidase |
| A02.051 | retrotransposon peptidase (fungus) |
| A02.052 | retrotransposon 17.6 peptidase |
| A02.053 | S71-related human endogenous retropepsin |
| A02.054 | Osvaldo retrotransposon peptidase (Drosophila sp.) |
| A02.055 | RTVL-H-like putative peptidase |
| A02.056 | human endogenous retrovirus retropepsin homologue 1 |
| A02.057 | human endogenous retrovirus retropepsin homologue 2 |

(continued)

| CODE | Protease name |
|---|---|
| A02.060 | type D-like endogenous retrovirus endopeptidase (Mus musculus) |
| A02.061 | Ulysses retrotransposon peptidase (Drosophila virilis) retropepsin |
| A02.062 | TED retrotransposon peptidase (Trichoplusia ni) |
| A02.063 | Walleye dermal sarcoma virus retropepsin |
| A03.001 | cauliflower mosaic virus-type endopeptidase |
| A03.002 | bacilliform virus endopeptidase |
| A03.003 | banana streak virus endopeptidase |
| A03.004 | Commelina yellow mottle virus endopeptidase |
| A03.005 | cassava vein mosaic virus-type endopeptidase |
| A03.006 | retrotransposon peptidase (Nicotiana tabacum) |
| A05.001 | thermopsin |
| A06.001 | nodavirus endopeptidase |
| A08.001 | signal peptidase II |
| A09.001 | spumapepsin |
| A11.001 | Copia transposon (Drosophila sp.) peptidase |
| A11.002 | Tnt1 retrotransposon (plant) endopeptidase |
| A11.003 | Ty1 transposon (Saccharomyces sp.) endopeptidase |
| A11.004 | Evelknievel retrotransposon endopeptidase |
| A11.005 | Melmoth transposon endopeptidase |
| A11.051 | SIRE-1 (Glycine max) peptidase |
| A21.001 | tetravirus endopeptidase |
| A22.001 | presenilin 1 |
| A22.002 | presenilin 2 |
| A22.003 | impas 1 endopeptidase |
| A22.004 | impas 4 endopeptidase |
| A22.005 | impas 2 endopeptidase |
| A22.006 | impas 5 endopeptidase |
| A22.007 | impas 3 endopeptidase |
| A22.008 | YKL100c protein (Saccharomyces cerevisiae) |
| A22.009 | SEL-12 protein (Caenorhabditis elegans) |
| A22.010 | hop-1 g.p. (Caenorhabditis elegans) |
| A24.001 | type 4 prepilin peptidase 1 |
| A24.003 | type 4 prepilin peptidase 2 |
| A24.016 | preflagellin peptidase |
| A24.017 | PibD g.p. (Sulfolobus sp.) |
| A26.001 | omptin |
| A26.002 | OmpP (Escherichia coli) |
| A26.003 | plasminogen activator Pla |

(continued)

| CODE | Protease name |
|---|---|
| A26.004 | protein E (Salmonella sp.) |
| A26.005 | peptidase SopA |
| A9G.001 | aspartic endopeptidase, plasma |
| A9G.008 | rhodotorulapepsin |
| A9G.010 | pycnoporopepsin |
| A9G.011 | scytalidopepsin A |
| A9G.017 | acid endopeptidase (Cladosporium) |
| A9G.018 | acid endopeptidase (Paecilomyces) |
| A9G.019 | acrocylindropepsin |
| A9G.020 | yapsin A |
| C01.001 | papain |
| C01.002 | chymopapain |
| C01.003 | caricain |
| C01.004 | glycyl endopeptidase |
| C01.005 | stem bromelain |
| C01.006 | ficain |
| C01.007 | actinidain |
| C01.008 | asclepain |
| C01.009 | cathepsin V |
| C01.010 | vignain |
| C01.011 | calotropin |
| C01.013 | cathepsin X |
| C01.014 | cathepsin L-like peptidase 2 |
| C01.015 | cathepsin L-like peptidase 3 |
| C01.016 | cathepsin-1 |
| C01.017 | zingipain |
| C01.018 | cathepsin F |
| C01.019 | CC-I endopeptidase (Carica sp.) |
| C01.020 | CC-III endopeptidase (Carica candamarcensis) |
| C01.021 | brassicain |
| C01.022 | glycinain |
| C01.023 | cathepsin M |
| C01.024 | endopeptidase B (barley-type) |
| C01.026 | ananain |
| C01.027 | comosain |
| C01.028 | fruit bromelain |
| C01.029 | pseudotzain |
| C01.030 | crustapain |

(continued)

| CODE | Protease name |
|---|---|
| C01.031 | cathepsin-2 |
| C01.032 | cathepsin L |
| C01.033 | cathepsin L-like endopeptidase (Fasciola sp.) |
| C01.034 | cathepsin S |
| C01.035 | cathepsin O |
| C01.036 | cathepsin K |
| C01.037 | cathepsin W |
| C01.038 | cathepsin P |
| C01.039 | cathepsin Q |
| C01.040 | cathepsin H |
| C01.041 | aleurain |
| C01.042 | cathepsin R |
| C01.044 | SmCL2-like peptidase |
| C01.045 | cathepsin-6 |
| C01.046 | falcipain-2 |
| C01.047 | granulovirus cathepsin |
| C01.049 | cathepsin B, plant form |
| C01.050 | histolysain |
| C01.053 | cathepsin-3 |
| C01.054 | 2310051m13rik protein |
| C01.055 | papain homologue (nematode) |
| C01.056 | Rcr3 peptidase (Lycopersicon sp.) |
| C01.057 | vinckepain-2 |
| C01.058 | peptidase similar to cathepsin 7 |
| C01.059 | peptidase similar to cathepsin 8 (Mus musculus) |
| C01.060 | cathepsin B |
| C01.061 | SmCB2 peptidase (Schistosoma sp.) |
| C01.062 | cathepsin B-like endopeptidase (platyhelminth) |
| C01.063 | falcipain-3 |
| C01.064 | RD21 endopeptidase |
| C01.065 | XCP1 peptidase (Arabidopsis-type) |
| C01.066 | cpl-1 endopeptidase |
| C01.067 | insect 26/29 kDa peptidase |
| C01.068 | vitellogenic cathepsin B |
| C01.070 | dipeptidyl-peptidase I |
| C01.071 | toxopain-1 |
| C01.072 | rhodesain |
| C01.073 | endopeptidase 1 (mite) |

(continued)

| CODE | Protease name |
|---|---|
| C01.074 | CPB endopeptidase |
| C01.075 | cruzipain |
| C01.076 | CPA endopeptidase |
| C01.077 | falcipain-1 |
| C01.079 | papain homologue (Theileria-type) |
| C01.081 | papain homologue (Dictyostelium-type) |
| C01.082 | papain homologue (trichomonad) |
| C01.083 | V-cath endopeptidase |
| C01.084 | bleomycin hydrolase (animal) |
| C01.085 | bleomycin hydrolase (yeast) |
| C01.086 | aminopeptidase C |
| C01.088 | oligopeptidase E |
| C01.089 | peptidase G |
| C01.091 | peptidase W |
| C01.093 | miltpain |
| C01.094 | giardain |
| C01.095 | papain homologue (Archaeoglobus) |
| C01.096 | melain G |
| C01.097 | phytolacain |
| C01.098 | CPC endopeptidase |
| C01.099 | ervatamin B |
| C01.100 | cruzipain 2 |
| C01.101 | cathepsin B-like peptidase, nematode |
| C01.102 | encystation-specific endopeptidase (Giardia sp.) |
| C01.104 | SPG31-like peptidase |
| C01.105 | mir1 g.p. (Zea mays) |
| C01.107 | papain homologue (Rattus norvegicus) |
| C01.108 | peptidase similar to cathepsin 8 (Rattus norvegicus) |
| C01.110 | similar to cathepsin M (Mus musculus) |
| C01.111 | cathepsin Q2 (Rattus norvegicus) |
| C01.112 | similar to cathepsin M (Rattus norvegicus) |
| C01.113 | tetrain |
| C01.114 | testin-3 |
| C01.115 | fascipain B |
| C01.116 | ervatamin C |
| C01.117 | senescence-associated gene 12 |
| C01.118 | allergen Blo t 1 (Blomia tropicalis) |
| C01.119 | EhCP112 peptidase (Entamoeba histolytica) |

(continued)

| CODE | Protease name |
|---|---|
| C01.120 | p48h-17 g.p. (Zinnia-type) |
| C01.121 | XCP2 peptidase |
| C01.122 | SERA5 peptidase (Plasmodium falciparum) |
| C01.123 | EhCP-B peptidase (Entamoeba histolytica) |
| C01.124 | dipeptidylpeptidase I (Plasmodium-type) |
| C01.125 | Cwp84 g.p. (Clostridium difficile) |
| C02.001 | calpain-1 |
| C02.002 | calpain-2 |
| C02.003 | calpain C |
| C02.004 | calpain-3 |
| C02.006 | calpain-9 |
| C02.007 | calpain-8 |
| C02.008 | calpain-7 |
| C02.009 | calpain tra-3 (Caenorhabditis elegans) |
| C02.010 | calpain-15 |
| C02.011 | calpain-5 |
| C02.013 | calpain-11 |
| C02.014 | calpain A |
| C02.015 | calpain B |
| C02.017 | calpain-12 |
| C02.018 | calpain-10 |
| C02.019 | phytocalpain |
| C02.020 | calpain-13 |
| C02.021 | calpain-14 |
| C02.022 | Tpr peptidase (Porphyromonas gingivalis) |
| C02.023 | calpain (Schistosoma sp.) |
| C03.001 | poliovirus-type picornain 3C |
| C03.003 | cowpea mosaic-type comovirus picornain 3C |
| C03.004 | grapevine fanleaf-type nepovirus picornain 3C |
| C03.005 | hepatitis A virus-type picornain 3C |
| C03.007 | rhinovirus picornain 3C |
| C03.008 | foot-and-mouth disease virus picornain 3C |
| C03.009 | cardiovirus picornain 3C |
| C03.010 | Theiler’s murine encephalomyelitis virus picornain 3C |
| C03.011 | coxsackievirus-type picornain 3C |
| C03.012 | tomato ringspot nepovirus picornain 3C |
| C03.013 | rhinovirus 14 3C peptidase |
| C03.014 | human enterovirus 71 3C peptidase |

(continued)

| CODE | Protease name |
|---|---|
| C03.020 | poliovirus-type picornain 2A |
| C03.021 | rhinovirus picornain 2A |
| C03.022 | coxsackievirus-type picornain 2A |
| C03.023 | parechovirus picornain 3C |
| C03.024 | rice tungro spherical virus-type endopeptidase |
| C03.025 | tomato black ring virus-type picornain |
| C04.001 | nuclear-inclusion-a endopeptidase (plum pox virus) |
| C04.002 | potato virus Y-type NIa endopeptidase |
| C04.003 | tobacco vein mottling virus-type NIa endopeptidase |
| C04.004 | tobacco etch virus NIa endopeptidase |
| C04.005 | Ornithogalum mosaic virus NIa endopeptidase |
| C04.006 | yam mosaic virus NIa endopeptidase |
| C04.007 | shallot potyvirus NIa endopeptidase |
| C04.008 | bean yellow mosaic virus-type NIa endopeptidase |
| C04.009 | papaya ringspot virus NIa endopeptidase |
| C04.010 | pea seed-borne mosaic virus NIa endopeptidase |
| C04.011 | Johnson grass mosaic virus NIa endopeptidase |
| C04.012 | rye grass mosaic virus NIa endopeptidase |
| C04.013 | sweet potato mild mottle virus NIa endopeptidase |
| C04.014 | potato virus A NIa endopeptidase |
| C05.001 | adenain |
| C06.001 | potato virus Y-type helper component peptidase |
| C06.002 | barley yellow mosaic virus-type helper component peptidase |
| C07.001 | chestnut blight fungus virus p29 peptidase |
| C08.001 | chestnut blight fungus virus p48 peptidase |
| C09.001 | sindbis virus-type nsP2 peptidase |
| C10.001 | streptopain |
| C10.002 | PrtT peptidase |
| C10.003 | periodontain |
| C11.001 | clostripain |
| C12.001 | ubiquitinyl hydrolase-L1 (mammal) |
| C12.002 | ubiquitinyl hydrolase-YUH1 |
| C12.003 | ubiquitinyl hydrolase-L3 |
| C12.004 | ubiquitinyl hydrolase-BAP1 |
| C12.005 | ubiquitinyl hydrolase-UCH37 |
| C12.006 | ubiquitinyl hydrolase B40085 |
| C12.007 | ubiquitinyl hydrolase isozyme L4 (Mus musculus) |
| C12.008 | ubiquitinyl hydrolase UCH-D (Drosophila melanogaster) |

(continued)

| CODE | Protease name |
|---|---|
| C12.009 | Uch2 peptidase (Schizosaccharomyces pombe) |
| C13.001 | legumain (plant beta form) |
| C13.002 | legumain (plant alpha form) |
| C13.003 | legumain (non-chordate) |
| C13.004 | legumain (chordate) |
| C13.005 | glycosylphosphatidylinositol:protein transamidase |
| C13.006 | legumain (plant gamma form) |
| C14.001 | caspase-1 |
| C14.002 | CED-3 endopeptidase |
| C14.003 | caspase-3 |
| C14.004 | caspase-7 |
| C14.005 | caspase-6 |
| C14.006 | caspase-2 |
| C14.007 | caspase-4 |
| C14.008 | caspase-5 |
| C14.009 | caspase-8 |
| C14.010 | caspase-9 |
| C14.011 | caspase-10 |
| C14.012 | caspase-11 |
| C14.013 | caspase-12 |
| C14.015 | caspase (insect 1) |
| C14.016 | caspase (insect 2) |
| C14.017 | caspase-13 |
| C14.018 | caspase-14 |
| C14.019 | caspase DRONC (Drosophila melanogaster) |
| C14.021 | ICEY peptidase |
| C14.023 | STRICA g.p. (Drosophila melanogaster) |
| C14.025 | caspase DAMM (Drosophila melanogaster) |
| C14.026 | paracaspase |
| C14.030 | Caspy g.p. (Danio rerio) |
| C14.031 | Caspy2 g.p. (Danio rerio) |
| C14.032 | putative caspase (Homo sapiens) |
| C14.033 | metacaspase-4 (Arabidopsis thaliana) |
| C14.034 | metacaspase-9 (Arabidopsis thaliana) |
| C14.035 | yeast metacaspase-1 |
| C15.001 | pyroglutamyl-peptidase I (prokaryote) |
| C15.010 | pyroglutamyl-peptidase I (eukaryote) |
| C16.001 | murine hepatitis coronavirus papain-like endopeptidase 1 |

(continued)

| CODE | Protease name |
|---|---|
| C16.002 | human coronavirus 229E papain-like endopeptidase 1 |
| C16.003 | porcine epidemic diarrhea virus papain-like endopeptidase 1 |
| C16.004 | porcine transmissible gastroenteritis coronavirusvirus papain-like endopeptidase 1 |
| C16.005 | avian infectious bronchitis coronavirus papain-like endopeptidase 1 |
| C16.006 | murine hepatitis coronavirus papain-like endopeptidase 2 |
| C16.008 | porcine transmissible gastroenteritis coronavirus papain-like endopeptidase 2 |
| C16.009 | SARS coronavirus papain-like endopeptidase |
| C18.001 | hepatitis C virus endopeptidase 2 |
| C19.001 | ubiquitin-specific peptidase 5 |
| C19.002 | Ubp1 ubiquitin peptidase |
| C19.003 | Ubp2 ubiquitin peptidase |
| C19.004 | Ubp3 ubiquitin peptidase |
| C19.005 | Doa4 ubiquitin peptidase |
| C19.006 | Ubp5 ubiquitin peptidase |
| C19.007 | Fat facets protein |
| C19.008 | ubiquitin-specific peptidase (plant) |
| C19.009 | ubiquitin-specific peptidase 6 |
| C19.010 | ubiquitin-specific peptidase 4 |
| C19.011 | ubiquitin-specific peptidase 8 |
| C19.012 | ubiquitin-specific peptidase 13 |
| C19.013 | ubiquitin-specific peptidase 2 |
| C19.014 | ubiquitin-specific peptidase 11 |
| C19.015 | ubiquitin-specific peptidase 14 |
| C19.016 | ubiquitin-specific peptidase 7 |
| C19.017 | ubiquitin-specific peptidase 9X |
| C19.018 | ubiquitin-specific peptidase 10 |
| C19.019 | ubiquitin-specific peptidase 1 |
| C19.020 | ubiquitin-specific peptidase 12 |
| C19.021 | ubiquitin-specific peptidase 16 |
| C19.022 | ubiquitin-specific peptidase 15 |
| C19.023 | ubiquitin-specific peptidase 17 |
| C19.024 | ubiquitin-specific peptidase 19 |
| C19.025 | ubiquitin-specific peptidase 20 |
| C19.026 | ubiquitin-specific peptidase 3 |
| C19.028 | ubiquitin-specific peptidase 9Y |
| C19.030 | ubiquitin-specific peptidase 18 |
| C19.031 | DUB-1 ubiquitin-specific peptidase |
| C19.032 | DUB-2 ubiquitin-specific peptidase |

(continued)

| CODE | Protease name |
|---|---|
| C19.034 | ubiquitin-specific peptidase 21 |
| C19.035 | ubiquitin-specific peptidase 22 |
| C19.037 | ubiquitin-specific peptidase 33 |
| C19.040 | ubiquitin-specific peptidase 29 |
| C19.041 | ubiquitin-specific peptidase 25 |
| C19.042 | ubiquitin-specific peptidase 36 |
| C19.044 | ubiquitin-specific peptidase 32 |
| C19.045 | ubiquitin-specific peptidase 26 (mouse-type) |
| C19.046 | ubiquitin-specific peptidase 26 (human-type) |
| C19.047 | ubiquitin-specific peptidase 24 |
| C19.048 | ubiquitin-specific peptidase 42 |
| C19.051 | Usp9y g.p. (Mus musculus) |
| C19.052 | ubiquitin-specific peptidase 46 |
| C19.053 | ubiquitin-specific peptidase 37 |
| C19.054 | ubiquitin-specific peptidase 28 |
| C19.055 | ubiquitin-specific peptidase 47 |
| C19.056 | ubiquitin-specific peptidase 38 |
| C19.057 | ubiquitin-specific peptidase 44 |
| C19.058 | ubiquitin-specific peptidase 50 |
| C19.059 | ubiquitin-specific peptidase 35 |
| C19.060 | ubiquitin-specific peptidase 30 |
| C19.064 | ubiquitin-specific peptidase 45 |
| C19.065 | ubiquitin-specific peptidase 51 |
| C19.067 | ubiquitin-specific peptidase 34 |
| C19.068 | ubiquitin-specific peptidase 48 |
| C19.069 | ubiquitin-specific peptidase 40 |
| C19.071 | ubiquitin-specific peptidase 31 |
| C19.073 | ubiquitin-specific peptidase 49 |
| C19.076 | protein similar to high mobility group protein |
| C19.077 | Dub5 peptidase (Mus musculus) |
| C19.078 | USP17-like peptidase |
| C19.079 | ubiquitin-specific peptidase 6 (Saccharomyces cerevisiae) |
| C19.080 | ubiquitin-specific peptidase 54 |
| C19.081 | ubiquitin-specific peptidase 53 |
| C19.082 | deubiquitinating enzyme 6 (Mus musculus) |
| C19.083 | deubiquitinating enzyme 14 (Saccharomyces cerevisiae) |
| C19.084 | deubiquitinating enzyme 14 (plant) |
| C19.085 | DUB-1A peptidase (Mus musculus) |

(continued)

| CODE | Protease name |
|---|---|
| C19.086 | DUB-2A peptidase (Mus musculus) |
| C19.087 | ubiquitin-specific peptidase 8 (Saccharomyces cerevisiae) |
| C19.088 | ubiquitin-specific peptidase 10 (Saccharomyces cerevisiae) |
| C21.001 | tymovirus endopeptidase |
| C23.001 | carlavirus endopeptidase |
| C24.001 | rabbit hemorrhagic disease virus 3C-like endopeptidase |
| C24.002 | feline calicivirus 3C-like endopeptidase |
| C25.001 | gingipain R |
| C25.002 | gingipain K |
| C25.003 | gingipain R2 |
| C26.001 | gamma-glutamyl hydrolase |
| C27.001 | rubella virus endopeptidase |
| C28.001 | foot-and-mouth disease virus L-peptidase |
| C28.002 | equine rhinovirus L-peptidase |
| C30.001 | hepatitis coronavirus picornain 3C-like endopeptidase |
| C30.002 | avian infectious bronchitis coronavirus 3C-like endopeptidase |
| C30.003 | human coronavirus 229E main endopeptidase |
| C30.004 | porcine transmissible gastroenteritis virus-type main endopeptidase |
| C30.005 | SARS coronavirus picornain 3C-like endopeptidase |
| C31.001 | porcine respiratory and reproductive syndrome arterivirus-type cysteine peptidase alpha |
| C32.001 | equine arteritis virus-type cysteine peptidase |
| C33.001 | equine arterivirus Nsp2-type cysteine peptidase |
| C36.001 | beet necrotic yellow vein furovirus-type papain-like endopeptidase |
| C37.001 | calicivirin |
| C39.001 | bacteriocin-processing peptidase |
| C39.003 | streptococcin SA-FF22 processing peptidase (Streptococcus pyogenes) |
| C39.004 | mersacidin lantibiotic processing peptidase (Bacillus sp.) |
| C39.005 | colicin V processing peptidase |
| C39.006 | mutacin II processing peptidase (Streptococcus mutans) |
| C39.007 | lacticin 481 processing peptidase (Lactococcus lactis) |
| C40.001 | dipeptidyl-peptidase VI (bacteria) |
| C40.002 | murein endopeptidase lytF (Bacillus sp.) |
| C40.003 | lytE g.p. (Bacillus-type) |
| C40.004 | spr g.p. (Escherichia-type) |
| C42.001 | beet yellows virus-type papain-like peptidase |
| C42.002 | papain-like peptidase 2 (citrus tristeza virus) |
| C42.003 | L1 peptidase (citrus tristeza virus) |
| C44.001 | amidophosphoribosyltransferase precursor |

(continued)

| CODE | Protease name |
|---|---|
| C45.001 | acyl-coenzyme A:6-aminopenicillanic acid acyl-transferase precursor |
| C46.001 | hedgehog protein |
| C46.002 | Sonic hedgehog protein |
| C46.003 | Indian hedgehog protein |
| C46.004 | Desert hedgehog protein |
| C46.005 | Tiggy-winkle protein |
| C47.001 | staphopain A |
| C47.002 | staphopain B |
| C47.003 | ecp g.p. (Staphylococcus epidermidis) |
| C48.001 | Ulp1 endopeptidase |
| C48.002 | SENP1 peptidase |
| C48.003 | SENP3 peptidase |
| C48.004 | SENP6 peptidase |
| C48.005 | Ulp2 endopeptidase |
| C48.007 | SENP2 peptidase |
| C48.008 | SENP5 peptidase |
| C48.009 | SENP7 peptidase |
| C48.011 | SENP8 peptidase |
| C48.012 | SENP4 peptidase |
| C48.018 | peptidase similar to SUMO-1-specific peptidase (Rattus norvegicus) |
| C48.020 | LOC297623 peptidase (Rattus norvegicus) |
| C48.021 | similar to SUMO-1-specific peptidase (Mus musculus) |
| C48.022 | esd4 g.p. (Arabidopsis thaliana) |
| C48.023 | XopD peptidase |
| C48.024 | Ulp1 g.p. (Drosophila melanogaster) |
| C50.001 | separase |
| C51.001 | D-alanyl-glycyl endopeptidase (staphylococcal phage phi11) |
| C53.001 | pestivirus Npro endopeptidase |
| C54.001 | ATG4 peptidase (Saccharomyces cerevisiae) |
| C54.002 | autophagin-2 |
| C54.003 | autophagin-1 |
| C54.004 | autophagin-3 |
| C54.005 | autophagin-4 |
| C55.001 | YopJ endopeptidase |
| C55.003 | AvrA g.p. (Salmonella sp.) |
| C55.004 | PopP1 g.p. (Ralstonia solanacearum) |
| C55.005 | AvrPpiG1 g.p. (Pseudomonas syringae) |
| C55.006 | AvrXv4 (Xanthomonas campestris) |

(continued)

| CODE | Protease name |
|---|---|
| C55.007 | VopA g.p. (Vibrio parahaemolyticus) |
| C56.001 | PfpI endopeptidase |
| C56.002 | DJ-1 putative peptidase |
| C56.004 | YDR533C peptidase |
| C56.006 | Hsp31 g.p. (Escherichia coli) |
| C57.001 | vaccinia virus I7L processing peptidase |
| C58.001 | YopT peptidase (Yersinia-type) |
| C58.002 | AvrPphB g.p. (Pseudomonas syringae) |
| C59.001 | penicillin V acylase (Bacillus-type) |
| C60.001 | sortase A |
| C60.002 | sortase B |
| C60.003 | sortase C2 |
| C62.001 | gill-associated virus 3C-like peptidase |
| C63.001 | African swine fever virus processing peptidase |
| C64.001 | Cezanne deubiquitinating peptidase |
| C64.002 | Cezanne-2 peptidase |
| C64.003 | tumor necrosis factor alpha-induced protein 3 |
| C64.004 | TRABID protein |
| C65.001 | otubain-1 |
| C65.002 | otubain-2 |
| C65.003 | otubain-3 |
| C66.001 | IdeS peptidase (Streptococcus pyogenes) |
| C67.001 | CylD protein |
| C69.001 | dipeptidase A |
| C69.002 | arginine aminopeptidase (Streptococcus sp.) |
| C70.001 | AvrRpt2 g.p. (Pseudomonas syringae) |
| C71.001 | pseudomurein endoisopeptidase Pei |
| C72.001 | HopPtoN g.p. (Pseudomonas syringae) |
| C9B.001 | lysosomal dipeptidase II |
| C9C.001 | dipeptidyl-dipeptidase |
| C9G.001 | cathepsin N |
| C9G.002 | leucoegresin-generating endopeptidase |
| C9G.003 | ATP-dependent cysteine endopeptidase |
| C9G.004 | mitochondrial cysteine endopeptidase |
| C9G.005 | cathepsin T |
| C9G.006 | nuclear cysteine endopeptidase |
| C9G.009 | cathepsin M (old) |
| C9G.012 | cancer procoagulant |

(continued)

| CODE | Protease name |
|---|---|
| C9G.013 | prohormone thiol peptidase |
| C9G.020 | archaean cysteine endopeptidase |
| C9G.021 | lobster muscle calpain-like peptidase |
| C9G.022 | cysteine endopeptidase (Micrococcus sp. INIA 528) |
| C9G.024 | alanyl aminopeptidase (cysteine type) (Pseudomonas aeruginosa) |
| C9G.025 | cysteine peptidase 1 (Vibrio harveyi) |
| C9G.026 | avian infectious bronchitis coronavirus papain-like endopeptidase 2 |
| G01.001 | scytalidoglutamic peptidase |
| G01.002 | aspergilloglutamic peptidase |
| G01.003 | endopeptidase EapB |
| G01.004 | endopeptidase EapC |
| M01.001 | aminopeptidase N |
| M01.002 | lysyl aminopeptidase (bacteria) |
| M01.003 | aminopeptidase A |
| M01.004 | leukotriene A4 hydrolase |
| M01.005 | alanyl aminopeptidase (proteobacteria) |
| M01.006 | Ape2 aminopeptidase |
| M01.007 | Aap1’ aminopeptidase |
| M01.008 | pyroglutamyl-peptidase II |
| M01.009 | aminopeptidase N (actinomycete-type) |
| M01.010 | cytosol alanyl aminopeptidase |
| M01.011 | cystinyl aminopeptidase |
| M01.012 | aminopeptidase G (Streptomyces sp.) |
| M01.013 | aminopeptidase N (insect) |
| M01.014 | aminopeptidase B |
| M01.015 | aminopeptidase H11 (nematode) |
| M01.016 | aminopeptidase Ey |
| M01.017 | Yin7 g.p. (Saccharomyces cerevisiae) |
| M01.018 | aminopeptidase PILS |
| M01.020 | tricorn interacting factor F2 (Thermoplasma sp.) |
| M01.021 | tricorn interacting factor F3 (Thermoplasma sp.) |
| M01.024 | leukocyte-derived arginine aminopeptidase |
| M01.025 | aminopeptidase-1 (Caenorhabditis elegans) |
| M01.027 | laeverin |
| M01.028 | aminopeptidase O |
| M01.029 | PfA-M1 aminopeptidase (Plasmodium falciparum-type) |
| M02.001 | angiotensin-converting enzyme peptidase unit 1 |
| M02.002 | peptidyl-dipeptidase Acer |

(continued)

| CODE | Protease name |
|---|---|
| M02.003 | peptidyl-dipeptidase Ance |
| M02.004 | angiotensin-converting enzyme peptidase unit 2 |
| M02.005 | peptidyl-dipeptidase A (Theromyzon) |
| M02.006 | angiotensin-converting enzyme 2 |
| M03.001 | thimet oligopeptidase |
| M03.002 | neurolysin |
| M03.003 | saccharolysin |
| M03.004 | oligopeptidase A |
| M03.005 | peptidyl-dipeptidase Dcp |
| M03.006 | mitochondrial intermediate peptidase |
| M03.007 | oligopeptidase F |
| M03.009 | oligopeptidase MepB |
| M04.001 | thermolysin |
| M04.003 | vibriolysin |
| M04.005 | pseudolysin |
| M04.006 | Msp peptidase (Legionella sp.) |
| M04.007 | coccolysin |
| M04.008 | thermolysin homologue (Listeria sp.) |
| M04.009 | aureolysin |
| M04.010 | vimelysin (Vibrio str. T1800) |
| M04.011 | lambda toxin (Clostridium sp.) |
| M04.012 | neutral peptidase B (Bacillus sp.) |
| M04.014 | bacillolysin |
| M04.016 | PA peptidase (Aeromonas-type) |
| M04.017 | griselysin |
| M04.018 | stearolysin |
| M04.019 | MprIII (Alteromonas sp. strain O-7) |
| M04.020 | pap6 endopeptidase |
| M04.021 | neutral endopeptidase (Thermoactinomyces sp. 27a) |
| M05.001 | mycolysin |
| M06.001 | immune inhibitor A (Bacillus sp.) |
| M06.004 | inhA2 g.p. (Bacillus sp.) |
| M07.001 | snapalysin |
| M08.001 | leishmanolysin |
| M08.002 | invadolysin |
| M08.003 | leishmanolysin-2 |
| M09.001 | microbial collagenase (Vibrio sp.) |
| M09.002 | collagenase colA |

(continued)

| CODE | Protease name |
|---|---|
| M09.003 | collagenase colH |
| M09.004 | endopeptidase VMC (Vibrio sp.) |
| M10.001 | collagenase 1 |
| M10.002 | collagenase 2 |
| M10.003 | gelatinase A |
| M10.004 | gelatinase B |
| M10.005 | stromelysin 1 |
| M10.006 | stromelysin 2 |
| M10.007 | stromelysin 3 |
| M10.008 | matrilysin |
| M10.009 | macrophage elastase |
| M10.010 | envelysin |
| M10.012 | plant matrixin |
| M10.013 | collagenase 3 |
| M10.014 | membrane-type matrix metallopeptidase 1 |
| M10.015 | membrane-type matrix metallopeptidase 2 |
| M10.016 | membrane-type matrix metallopeptidase 3 |
| M10.017 | membrane-type matrix metallopeptidase 4 |
| M10.018 | collagenase 4 |
| M10.019 | enamelysin |
| M10.020 | fragilysin |
| M10.021 | matrix metallopeptidase 19 |
| M10.022 | matrix metallopeptidase 23B |
| M10.023 | membrane-type matrix metallopeptidase 5 |
| M10.024 | membrane-type matrix metallopeptidase 6 |
| M10.025 | HMMP peptidase (Hydra vulgaris) |
| M10.026 | matrix metallopeptidase 21 |
| M10.027 | matrix metallopeptidase 22 |
| M10.029 | matrilysin-2 |
| M10.030 | epilysin |
| M10.031 | Dm1 matrix matallopeptidase (Diptera) |
| M10.032 | matrixin V |
| M10.033 | collagenase-like A peptidase (rodent) |
| M10.034 | collagenase-like B peptidase (rodent) |
| M10.035 | S-layer-associated peptidase (Caulobacter crescentus) |
| M10.036 | Dm2-MMP peptidase (Drosophila melanogaster) |
| M10.037 | matrix metallopeptidase 23A |
| M10.051 | serralysin |

(continued)

| CODE | Protease name |
|---|---|
| M10.052 | peptidase A (Erwinia-type) |
| M10.053 | peptidase B (Erwinia-type) |
| M10.054 | peptidase C (Erwinia-type) |
| M10.055 | peptidase G (Erwinia-type) |
| M10.056 | aeruginolysin |
| M10.057 | mirabilysin |
| M10.060 | epralysin |
| M10.062 | psychrophilic alkaline metallopeptidase (Pseudomonas sp.) |
| M11.001 | gametolysin |
| M11.002 | VMP peptidase (Volvox carteri) |
| M11.003 | mmp2 g.p. (Chlamydomonas reinhardtii) |
| M12.001 | astacin |
| M12.002 | meprin alpha subunit |
| M12.003 | myosinase |
| M12.004 | meprin beta subunit |
| M12.005 | procollagen C-peptidase |
| M12.006 | choriolysin L |
| M12.007 | choriolysin H |
| M12.008 | nephrosin |
| M12.010 | tolloid |
| M12.011 | tolkin |
| M12.013 | SpAN g.p. (Strongylocentrotus purpuratus) |
| M12.014 | hatching enzyme (Xenopus) |
| M12.015 | xolloid |
| M12.016 | mammalian tolloid-like 1 protein |
| M12.017 | metallopeptidase 1 (Hydra) |
| M12.018 | mammalian tolloid-like 2 protein |
| M12.019 | MIG-17 endopeptidase (Caenorhabditis elegans) |
| M12.020 | ADAM28 endopeptidase (mouse-type) |
| M12.021 | ADAMTS9 endopeptidase |
| M12.022 | brevilysin H6 |
| M12.024 | ADAMTS14 endopeptidase |
| M12.025 | ADAMTS15 endopeptidase |
| M12.026 | ADAMTS16 endopeptidase |
| M12.027 | ADAMTS17 endopeptidase |
| M12.028 | ADAMTS18 endopeptidase |
| M12.029 | ADAMTS19 endopeptidase |
| M12.030 | peptidase similar to ADAMTS-1 endopeptidase (Mus musculus) |

(continued)

| CODE | Protease name |
|---|---|
| M12.031 | peptidase similar to ADAMTS-9 endopeptidase (Rattus norvegicus) |
| M12.066 | flavastacin |
| M12.131 | acutolysin A |
| M12.132 | bilitoxin (Agkistrodon bilineatus) |
| M12.133 | fibrolase (Agkistrodon contortrix) |
| M12.134 | halylysin a |
| M12.135 | gon-1 g.p. (Caenorhabditis elegans) |
| M12.136 | leucolysin |
| M12.137 | BHRa hemorrhagin (Bitis arietans) |
| M12.138 | jararhagin |
| M12.139 | bothrolysin |
| M12.140 | bothropasin |
| M12.141 | adamalysin |
| M12.142 | atrolysin A |
| M12.143 | atrolysin B |
| M12.144 | atrolysin C |
| M12.145 | atrolysin E |
| M12.146 | atrolysin F |
| M12.147 | atroxase |
| M12.148 | basilysin |
| M12.149 | horrilysin |
| M12.150 | ruberlysin |
| M12.151 | ecarin |
| M12.152 | ophiolysin |
| M12.153 | fibrinolytic endopeptidase (Philodryas olfershii) |
| M12.154 | trimerelysin I |
| M12.155 | trimerelysin II |
| M12.156 | trimerelysin IIA |
| M12.157 | mucrolysin |
| M12.158 | russellysin |
| M12.159 | cobrin |
| M12.160 | venom metalloendopeptidase PREH |
| M12.161 | kistomin (Calloselasma rhodostoma) |
| M12.162 | mutalysin II |
| M12.163 | graminelysin (Trimeresurus gramineus) |
| M12.164 | lebetase |
| M12.166 | BaH1 endopeptidase (Bothrops asper) |
| M12.167 | najalysin |

(continued)

| CODE | Protease name |
|---|---|
| M12.168 | alpha peptidase (Crotalus atrox) |
| M12.169 | metalloendopeptidase (Bothrops moojeni) |
| M12.170 | jararafibrase II (Bothrops jararaca) |
| M12.171 | HT-1 endopeptidase (Crotalus viridis) |
| M12.172 | carinactivase |
| M12.173 | mocarhagin |
| M12.176 | fibrinolytic peptidase M5 (Crotalus molossus) |
| M12.177 | multactivase |
| M12.178 | brevilysin L6 |
| M12.179 | bilitoxin 2 (Agkistrodon bilineatus) |
| M12.180 | Mde10 metalloendopeptidase (Schizosaccharomyces) |
| M12.184 | mutalysin I |
| M12.185 | moojeni peptidase B |
| M12.186 | vascular apoptosis-inducing protein 1 |
| M12.187 | similar to ADAM 21 preproprotein (Rattus norvegicus) |
| M12.188 | ADAMTS20 endopeptidase (Mus musculus) |
| M12.201 | ADAM1 endopeptidase |
| M12.202 | Adam1A g.p. (Mus musculus) |
| M12.203 | Adam1B g.p. (Mus musculus) |
| M12.208 | ADAM8 endopeptidase |
| M12.209 | ADAM9 endopeptidase |
| M12.210 | ADAM10 endopeptidase |
| M12.211 | Kuzbanian protein (non-mammalian) |
| M12.212 | ADAM12 endopeptidase |
| M12.213 | ADAM13 endopeptidase |
| M12.214 | adamalysin-19 |
| M12.215 | ADAM 15 endopeptidase |
| M12.217 | ADAM 17 endopeptidase |
| M12.218 | ADAM20 endopeptidase |
| M12.219 | ADAMDEC1 endopeptidase |
| M12.220 | ADAMTS3 endopeptidase |
| M12.221 | ADAMTS4 endopeptidase |
| M12.222 | ADAMTS1 endopeptidase |
| M12.224 | ADAM28 endopeptidase (human-type) |
| M12.225 | ADAMTS5 endopeptidase |
| M12.226 | ADAMTS8 endopeptidase |
| M12.227 | ADAM24 endopeptidase |
| M12.228 | ADAM25 endopeptidase |

(continued)

| CODE | Protease name |
|---|---|
| M12.229 | ADAM26 endopeptidase |
| M12.230 | ADAMTS6 endopeptidase |
| M12.231 | ADAMTS7 endopeptidase |
| M12.232 | ADAM30 endopeptidase |
| M12.233 | ADAM31 endopeptidase (rodent) |
| M12.234 | ADAM21 endopeptidase (Homo sapiens) |
| M12.235 | ADAMTS10 endopeptidase |
| M12.236 | kaouthiagin |
| M12.237 | ADAMTS12 endopeptidase |
| M12.238 | membrane-anchored metallopeptidase (Xenopus laevis) |
| M12.241 | ADAMTS13 endopeptidase |
| M12.242 | TM-3 peptidase (Trimeresurus mucrosquamatus) |
| M12.243 | testase 4 (Mus musculus) |
| M12.244 | ADAM33 endopeptidase |
| M12.245 | ovastacin |
| M12.246 | ADAMTS20 endopeptidase (Homo sapiens) |
| M12.247 | peptidase similar to ADAM 21 endopeptidase (Mus musculus) |
| M12.248 | peptidase similar to ADAMTS-6 endopeptidase (Mus musculus) |
| M 12.249 | testase-7 |
| M12.250 | testase-6 |
| M12.251 | testase-8 |
| M12.252 | testase-9 |
| M12.301 | procollagen I N-endopeptidase |
| M12.302 | ADAMTS adt-1 endopeptidase (Caenorhabditis elegans) |
| M12.303 | acutolysin C |
| M12.304 | jararafibrase III (Bothrops jararaca) |
| M12.305 | jararafibrase IV (Bothrops jararaca) |
| M12.306 | BHRb haemorrhagin (Bitis arietans) |
| M12.307 | halylysin b |
| M12.308 | halylysin c |
| M12.309 | hemorrhagic toxin I (Gloydius halys blomhoffii) |
| M12.310 | metallopeptidase MTP-1 (Ancylostoma caninum) |
| M12.311 | BaP1 endopeptidase (Bothrops asper) |
| M12.312 | neuwiedase (Bothrops neuwiedi) |
| M12.313 | jerdonitin (Trimeresurus jerdonii) |
| M12.314 | EBR1 peptidase (Strongylocentrotus sp.) |
| M12.315 | halysase (Gloydius halys) |
| M12.316 | triflamp (Trimeresurus flavoviridis) |

(continued)

| CODE | Protease name |
|---|---|
| M12.317 | acutolysin D |
| M12.318 | C17G1.6 g.p. (Caenorhabditis elegans) |
| M12.319 | C26C6.3 gene (Caenorhabditis elegans) |
| M12.321 | TOH-2 g.p. (Caenorhabditis elegans) |
| M13.001 | neprilysin |
| M13.002 | endothelin-converting enzyme 1 |
| M13.003 | endothelin-converting enzyme 2 |
| M13.004 | oligopeptidase O1 |
| M13.005 | oligopeptidase O3 |
| M13.007 | DINE peptidase |
| M13.008 | neprilysin-2 |
| M13.009 | PgPepO oligopeptidase |
| M13.010 | oligopeptidase O2 |
| M13.011 | nematode neprilysin homologue |
| M13.012 | Nep2 peptidase (Drosophila melanogaster) |
| M13.090 | Kell blood-group protein |
| M13.091 | PHEX endopeptidase |
| M14.001 | carboxypeptidase A1 |
| M14.002 | carboxypeptidase A2 |
| M14.003 | carboxypeptidase B |
| M14.004 | carboxypeptidase N |
| M14.005 | carboxypeptidase E |
| M14.006 | carboxypeptidase M |
| M14.007 | carboxypeptidase T |
| M14.008 | gamma-D-glutamyl-(L)-meso-diaminopimelate peptidase I |
| M14.009 | carboxypeptidase U |
| M14.010 | carboxypeptidase A3 |
| M14.011 | metallocarboxypeptidase D peptidase unit 1 |
| M14.012 | metallocarboxypeptidase Z |
| M14.014 | carboxypeptidase MeCPA |
| M14.016 | metallocarboxypeptidase D peptidase unit 2 |
| M14.017 | carboxypeptidase A4 |
| M14.018 | carboxypeptidase A6 |
| M14.020 | carboxypeptidase A5 |
| M14.021 | metallocarboxypeptidase O |
| M14.023 | CPG70 carboxypeptidase (Porphyromonas gingivalis) |
| M14.024 | insect gut carboxypeptidase-1 |
| M14.027 | hypothetical protein flj14442 (Homo sapiens) |

(continued)

| CODE | Protease name |
|---|---|
| M14.029 | A430081C19RIK protein (Mus musculus) |
| M14.030 | hypothetical Zn-dependent exopeptidase (Mus musculus) |
| M14.031 | insect gut carboxypeptidase-2 |
| M15.001 | zinc D-Ala-D-Ala carboxypeptidase (Streptomyces sp.) |
| M15.002 | DD-carboxypeptidase pdcA (Myxococcus xanthus) |
| M15.003 | van XYc peptidase |
| M15.010 | vanY D-Ala-D-Ala carboxypeptidase |
| M15.011 | vanX D-Ala-D-Ala dipeptidase |
| M15.020 | ply endolysin |
| M16.001 | pitrilysin |
| M16.002 | insulysin |
| M16.003 | mitochondrial processing peptidase beta-subunit |
| M16.004 | chloroplast (stromal) processing peptidase |
| M16.005 | nardilysin |
| M16.006 | pqqF protein |
| M16.007 | Axl1 peptidase |
| M16.008 | Ste23 peptidase |
| M16.009 | eupitrilysin |
| M16.011 | falcilysin |
| M16.012 | PreP peptidase |
| M 16.013 | CYM1 peptidase (Saccharomyces cerevisiae) |
| M17.001 | leucyl aminopeptidase (animal) |
| M17.002 | leucyl aminopeptidase (plant) |
| M17.003 | aminopeptidase A (bacteria) |
| M17.004 | PepB aminopeptidase |
| M18.001 | aminopeptidase I |
| M18.002 | aspartyl aminopeptidase |
| M19.001 | membrane dipeptidase |
| M19.003 | dipeptidase AC |
| M19.007 | thermostable dipeptidase (Brevibacillus-type) |
| M20.001 | glutamate carboxypeptidase |
| M20.002 | Gly-X carboxypeptidase |
| M20.003 | peptidase T |
| M20.004 | peptidase V |
| M20.005 | cytosolic nonspecific dipeptidase |
| M20.006 | carnosinase |
| M20.007 | Xaa-His dipeptidase |
| M20.008 | carboxypeptidase Ss1 |

(continued)

| CODE | Protease name |
|---|---|
| M20.010 | DapE peptidase |
| M20.012 | Pep581 peptidase (Prevotella albensis) |
| M22.001 | O-sialoglycoprotein endopeptidase |
| M22.002 | yeaZ protein |
| M22.005 | Pgp1 peptidase |
| M23.001 | beta-lytic metalloendopeptidase (myxobacteria) |
| M23.002 | staphylolysin |
| M23.003 | fibrinolytic endopeptidase (Aeromononas) |
| M23.004 | lysostaphin |
| M23.005 | zoocin A |
| M23.006 | YibP peptidase |
| M23.007 | enterolysin A |
| M24.001 | methionyl aminopeptidase 1 |
| M24.002 | methionyl aminopeptidase 2 |
| M24.003 | Xaa-Pro dipeptidase (bacteria) |
| M24.004 | aminopeptidase P (bacteria) |
| M24.005 | aminopeptidase P2 |
| M24.007 | Xaa-Pro dipeptidase (eukaryote) |
| M24.008 | Xaa-Pro dipeptidase (archaea) |
| M24.009 | aminopeptidase P1 |
| M24.026 | aminopeptidase P homologue |
| M24.031 | leucine aminopeptidase (Thermotoga maritima) |
| M26.001 | IgA1-specific metalloendopeptidase |
| M26.002 | ZmpB metallopeptidase (Streptococcus sp.) |
| M26.003 | ZmpC metallopeptidase (Streptococcus pneumoniae) |
| M27.001 | tentoxilysin |
| M27.002 | bontoxilysin |
| M28.001 | aminopeptidase Y |
| M28.002 | aminopeptidase Ap1 |
| M28.003 | aminopeptidase S |
| M28.004 | aminopeptidase apAC (Aeromonas caviae) |
| M28.005 | IAP aminopeptidase |
| M28.007 | AMP1 putative carboxypeptidase |
| M28.008 | PA2939 g.p. (Pseudomonas aeruginosa) |
| M28.010 | glutamate carboxypeptidase II |
| M28.011 | NAALADASE L peptidase |
| M28.012 | glutamate carboxypeptidase III |
| M28.014 | plasma glutamate carboxypeptidase |

(continued)

| CODE | Protease name |
|---|---|
| M28.015 | aminopeptidase ES-62 (Acanthocheilonema viteae) |
| M28.019 | aminopeptidase SSAP (Streptomyces septatus) |
| M29.001 | aminopeptidase T |
| M29.002 | aminopeptidase II (Bacillus-type) |
| M29.004 | PepS aminopeptidase |
| M30.001 | hyicolysin |
| M32.001 | carboxypeptidase Taq |
| M32.002 | carboxypeptidase Pfu |
| M34.001 | anthrax lethal factor |
| M35.001 | penicillolysin |
| M35.002 | deuterolysin |
| M35.003 | extracellular endopeptidase (Aeromonas-type) |
| M35.004 | peptidyl-Lys metalloendopeptidase |
| M36.001 | fungalysin |
| M38.001 | beta-aspartyl dipeptidase |
| M38.002 | Pro-Hyp dipeptidase |
| M41.001 | FtsH endopeptidase |
| M41.002 | Afg3 g.p. (Saccharomyces cerevisiae) |
| M41.003 | m-AAA peptidase |
| M41.004 | i-AAA peptidase |
| M41.005 | FtsH endopeptidase homologue, chloroplast |
| M41.006 | paraplegin |
| M41.007 | Afg3-like protein 2 |
| M41.009 | FtsH-2 peptidase |
| M41.010 | Afg3-like protein 1 |
| M41.016 | ATP-dependent zinc metallopeptidase (Mus musculus) |
| M42.001 | glutamyl aminopeptidase (bacterium) |
| M42.002 | bacillus aminopeptidase I (Geobacillus/Bacillus stearothermophilus) |
| M42.003 | PTET aminopeptidase (Pyrococcus sp.) |
| M42.004 | PTET2 aminopeptidase (Pyrococcus sp.) |
| M42.005 | TET aminopeptidase (Halobacterium sp.) |
| M43.001 | cytophagalysin |
| M43.002 | metallopeptidase MEP1 (Metarhizium) |
| M43.004 | pappalysin-1 |
| M43.005 | pappalysin-2 |
| M44.001 | pox virus metalloendopeptidase |
| M48.001 | Ste24 endopeptidase |
| M48.002 | HtpX endopeptidase |

(continued)

| CODE | Protease name |
|---|---|
| M48.003 | farnesylated-protein converting enzyme 1 |
| M48.004 | HtpX-2 endopeptidase |
| M48.009 | YhfN protein (Bacillus sp.) |
| M48.010 | PAB0555 protein (Pyrococcus abyssi) |
| M48.011 | small heat-shock protein (Plasmodium vivax) |
| M48.018 | Oma1 endopeptidase (Saccharomyces cerevisiae) |
| M49.001 | dipeptidyl-peptidase III |
| M50.001 | S2P peptidase |
| M50.002 | sporulation factor SpoIVFB |
| M50.003 | YUP8H12.25 protein (Arabidopsis thaliana) |
| M50.004 | RseP peptidase |
| M52.001 | HybD endopeptidase |
| M52.002 | HyaD endopeptidase |
| M52.003 | HycI endopeptidase |
| M55.001 | D-aminopeptidase DppA |
| M56.001 | BlaR1 peptidase |
| M56.002 | MecR1 g.p. (Staphylococcus sp.) |
| M56.003 | PenR1 g.p. (Bacillus licheniformis) |
| M57.001 | prtB g.p. (Myxococcus xanthus) |
| M60.001 | enhancin |
| M61.001 | glycyl aminopeptidase |
| M63.001 | gpr peptidase |
| M64.001 | IgA peptidase (Clostridium ramosum) |
| M66.001 | StcE peptidase |
| M67.001 | Poh1 peptidase |
| M67.002 | Jab1/MPN domain metalloenzyme |
| M67.006 | AMSH deubiquitinating peptidase |
| M67.007 | C6.1A-like putative peptidase |
| M67.008 | putative peptidase (Homo sapiens chromosome 2) |
| M67.010 | JAMM-like protein (Archaeoglobus-type) |
| M72.001 | peptidyl-Asp metalloendopeptidase |
| M73.001 | camelysin |
| M74.001 | murein endopeptidase |
| M75.001 | imelysin |
| M9A.002 | tripeptide aminopeptidase |
| M9A.005 | clostridial aminopeptidase |
| M9A.007 | Xaa-Trp aminopeptidase |
| M9A.008 | tryptophanyl aminopeptidase |

(continued)

| CODE | Protease name |
|---|---|
| M9A.009 | aminopeptidase X |
| M9A.010 | aminopeptidase yscCo-II |
| M9A.011 | neuron-specific aminopeptidase |
| M9A.012 | glycyl aminopeptidase (Actinomucor elegans) |
| M9B.001 | Xaa-Arg dipeptidase |
| M9B.004 | Met-Xaa dipeptidase |
| M9D.001 | peptidyl-dipeptidase B |
| M9D.002 | proline-specific peptidyl-dipeptidase (Streptomyces) |
| M9E.002 | alanine carboxypeptidase |
| M9E.003 | mitochondrial carboxypeptidase |
| M9E.004 | membrane Pro-X carboxypeptidase |
| M9E.007 | carboxypeptidase G3 |
| M9G.003 | acrolysin |
| M9G.005 | succinyl-tri-alanyl-p-nitroaniline hydrolase |
| M9G.008 | plant metalloendopeptidase |
| M9G.009 | neutral endopeptidase (Aspergillus oryzae) |
| M9G.018 | neutral endopeptidase (Micrococcus caseolyticus) |
| M9G.021 | metalloendopeptidase QG (Escherichia coli) |
| M9G.022 | peptidase Ci (Escherichia coli) |
| M9G.025 | magnolysin |
| M9G.026 | dactylysin |
| M9G.028 | magaininase |
| M9G.029 | MAP1 peptidase (Myxococcus xanthus) |
| M9G.030 | dynorphin-processing endopeptidase (metallo-type) |
| M9G.031 | cyclic peptidase (Lactocobacillus) |
| M9G.034 | metallopeptidase ShpII (Staphylococcus hyicus) |
| M9G.035 | endopeptidase ECP 32 (Escherichia coli) |
| M9G.036 | gonadotropin beta-subunit nicking enzyme |
| M9G.037 | dithiothreitol-sensitive tetrameric peptidase |
| M9G.039 | procollagen II N-peptidase |
| M9G.040 | hepatitis B virus binding factor |
| M9G.041 | aharin |
| M9G.043 | collagenase (Empedobacter collagenolyticum) |
| M9G.044 | endopeptidase Thr-N |
| M9G.047 | insulin-cleaving periplasmic peptidase (Acinetobacter calcoaceticus) |
| M9G.049 | procollagen III N-peptidase |
| M9G.051 | ZPA-processing enzyme |
| S01.001 | chymotrypsin A (cattle-type) |

(continued)

| CODE | Protease name |
|---|---|
| S01.003 | mast cell peptidase 2 (Mus musculus) |
| S01.004 | Cma2 g.p. (Mus musculus) |
| S01.005 | mast cell peptidase 4 (Rattus) |
| S01.008 | mast cell peptidase 10 (Rattus) |
| S01.009 | mast cell peptidase 8 (Rattus) |
| S01.010 | granzyme B, human-type |
| S01.011 | testisin |
| S01.012 | mast cell peptidase 3 (Rattus) |
| S01.013 | Nudel peptidase |
| S01.015 | tryptase beta (Homo sapiens) |
| S01.017 | kallikrein hK5 |
| S01.018 | scolexin |
| S01.019 | corin |
| S01.020 | kallikrein hK12 |
| S01.021 | DESC1 peptidase |
| S01.022 | ovotryptase (Xenopus laevis) |
| S01.023 | flavoxobin |
| S01.024 | ovotryptase 2 (Xenopus laevis) |
| S01.025 | mast cell peptidase 6 (mouse numbering) |
| S01.026 | mast cell peptidase 7 (mouse numbering) |
| S01.028 | tryptase gamma 1 |
| S01.029 | kallikrein hK14 |
| S01.030 | granzyme N |
| S01.031 | peptidase 9 (Dermatophagoides-type) |
| S01.033 | hyaluronan-binding peptidase |
| S01.034 | transmembrane peptidase, serine 4 |
| S01.035 | brachyurin-T |
| S01.036 | granzyme O |
| S01.037 | kallikrein mK5 (Mus sp.) |
| S01.038 | kallikrein mK21 (Mus musculus) |
| S01.039 | kallikrein mK22 (Mus musculus) |
| S01.040 | chymotrypsin-like enzyme (Lepidoptera) |
| S01.041 | kallikrein mK11 (Mus musculus) |
| S01.042 | intestinal serine peptidase (rodent) |
| S01.045 | TESP2 peptidase (Mus musculus) |
| S01.047 | adrenal secretory serine peptidase |
| S01.048 | Xesp-1 g.p. (Xenopus laevis) |
| S01.049 | Xesp-2 g.p. (Xenopus laevis) |

(continued)

| CODE | Protease name |
|---|---|
| S01.050 | XMT-SP1 g.p. (Xenopus laevis) |
| S01.052 | kallidin-releasing enzyme (Bitis arietans) |
| S01.054 | tryptase delta 1 (Homo sapiens) |
| S01.055 | trypsin 5 (mouse numbering) |
| S01.057 | trypsin 8 (mouse numbering) |
| S01.058 | trypsin 9 (mouse numbering) |
| S01.059 | trypsin 10 (mouse numbering) |
| S01.060 | trypsin 11 (mouse numbering) |
| S01.061 | trypsin 12 (mouse numbering) |
| S01.062 | trypsin 15 (mouse numbering) |
| S01.063 | trypsin 16 (mouse numbering) |
| S01.064 | trypsin 20 (mouse numbering) |
| S01.065 | kallikrein mK2 (Mus musculus) |
| S01.066 | kallikrein mGk4 (Mus musculus) |
| S01.067 | kallikrein mK8 (Mus musculus) |
| S01.068 | kallikrein mK14 (Mus musculus) |
| S01.069 | kallikrein mK24 (Mus musculus) |
| S01.070 | kallikrein mK26 (Mus musculus) |
| S01.071 | kallikrein mK9 (Mus musculus) |
| S01.072 | matriptase-3 |
| S01.073 | mouse glandular kallikrein 27 |
| S01.074 | marapsin |
| S01.075 | tryptase homologue 2 (Homo sapiens) |
| S01.076 | tryptase homologue 3 (Homo sapiens) |
| S01.079 | transmembrane peptidase, serine 3 |
| S01.081 | kallikrein hK15 (Homo sapiens) |
| S01.082 | spermosin (Halocynthia roretzi) |
| S01.084 | mouse kallikrein 10 |
| S01.086 | 30kP peptidase A (Bombyx-type) |
| S01.087 | mosaic serine peptidase long-form |
| S01.090 | hypodermin B |
| S01.091 | natural killer cell peptidase 1 (Rattus norvegicus) |
| S01.092 | trypsin Va (rodent) |
| S01.093 | trypsin Vb (Rattus norvegicus) |
| S01.094 | trypsin 1 (Rattus-type) |
| S01.095 | vascular chymase (Rattus norvegicus) |
| S01.097 | granzyme-like protein 1 (Rattus norvegicus) |
| S01.099 | testis serine peptidase 4 |

(continued)

| CODE | Protease name |
|---|---|
| S01.100 | tryptase-6 (Mus musculus) |
| S01.101 | trypsin (Streptomyces sp.) |
| S01.102 | trypsin (Streptomyces erythreaus) |
| S01.103 | trypsin (fungal) |
| S01.104 | 1700007n14rik protein (Mus musculus) |
| S01.108 | tryptase-5 (Mus musculus) |
| S01.109 | astrovirus serine peptidase |
| S01.110 | trypsin alpha (insect) |
| S01.111 | hypodermin A |
| S01.112 | trypsin (invertebrate) |
| S01.113 | vitellin-degrading endopeptidase (Bombyx-type) |
| S01.114 | trypsin theta (insect) |
| S01.115 | trypsin iota (insect) |
| S01.116 | trypsin zeta (insect) |
| S01.117 | trypsin eta (insect) |
| S01.118 | tryptase (mammalian, non-human) |
| S01.119 | trypsin 2 (anionic) (Rattus norvegicus) |
| S01.120 | trypsin 2 (mammalian, non-human, non-rodent) |
| S01.121 | hypodermin C |
| S01.122 | brachyurin-C |
| S01.123 | euphauserase |
| S01.124 | trypsin (fish) |
| S01.125 | trypsin X (fish) |
| S01.127 | cationic trypsin (Homo sapiens-type) |
| S01.128 | trypsin (Petromyzon-type) |
| S01.129 | trypsin 4 (Mus musculus) |
| S01.130 | trypsin (mosquito type) |
| S01.131 | neutrophil elastase |
| S01.132 | mannan-binding lectin-associated serine peptidase-3 |
| S01.133 | cathepsin G |
| S01.134 | myeloblastin |
| S01.135 | granzyme A |
| S01.136 | granzyme B, rodent-type |
| S01.137 | granzyme C |
| S01.139 | granzyme M |
| S01.140 | chymase (human-type) |
| S01.141 | mast cell peptidase 1 (rodent) |
| S01.142 | duodenase |

(continued)

| CODE | Protease name |
|---|---|
| S01.143 | tryptase alpha |
| S01.144 | cercarial elastase (Schistosoma) |
| S01.145 | mastin |
| S01.146 | granzyme K |
| S01.147 | granzyme H |
| S01.148 | mast cell peptidase 9 (Rattus norvegicus) |
| S01.149 | mast cell peptidase 4 (mouse numbering) |
| S01.150 | mast cell peptidase 5 (mouse numbering) |
| S01.151 | trypsin 1 |
| S01.152 | chymotrypsin B |
| S01.153 | pancreatic elastase |
| S01.154 | pancreatic endopeptidase E |
| S01.155 | pancreatic elastase II |
| S01.156 | enteropeptidase |
| S01.157 | chymotrypsin C |
| S01.159 | prostasin |
| S01.160 | kallikrein hK1 |
| S01.161 | kallikrein hK2 (Homo sapiens) |
| S01.162 | kallikrein hK3 |
| S01.163 | kallikrein mK16 (Mus musculus) |
| S01.164 | mouse kallikrein 1 |
| S01.165 | kallikrein rK10 (Rattus norvegicus) |
| S01.166 | chymotrypsin m-type 1 (insect) |
| S01.167 | mouse kallikrein 6 |
| S01.168 | chymotrypsin m-type 2 (insect) |
| S01.170 | 7S nerve growth factor gamma subunit (Mus sp.) |
| S01.171 | kallikrein 1 (Equus caballus) |
| S01.172 | tonin |
| S01.173 | kallikrein 13 (Mus musculus) |
| S01.174 | mesotrypsin |
| S01.176 | batroxobin |
| S01.177 | crotalase |
| S01.178 | Ancrod |
| S01.179 | bothrombin |
| S01.180 | platelet-aggregating venom endopeptidase |
| S01.181 | bilineobin |
| S01.183 | trypsin IV (Rattus norwegicus) |
| S01.184 | factor V activator (Daboia russellii) |

(continued)

| CODE | Protease name |
|---|---|
| S01.186 | venom plasminogen activator (Trimeresurus sp.) |
| S01.187 | peptidase 6 (Dermatophagoides sp.) |
| S01.188 | capillary permeability-increasing enzyme-2 (Gloydius-type) |
| S01.189 | complement component C1r-like peptidase |
| S01.190 | tissue kallikrein (Mastomys natalensis) |
| S01.191 | complement factor D |
| S01.192 | complement component activated C1r |
| S01.193 | complement component activated C1s |
| S01.194 | complement component 2 |
| S01.196 | complement factor B |
| S01.198 | mannan-binding lectin-associated serine peptidase 1 |
| S01.199 | complement factor I |
| S01.200 | Snake endopeptidase (Insecta) |
| S01.201 | Easter endopeptidase |
| S01.202 | Gastrulation-defective g.p. (Drosophila melanogaster) |
| S01.203 | CG3066 protein (Drosophila melanogaster) |
| S01.204 | prophenoloxidase-activating endopeptidase (Holotrichia diomphalia) |
| S01.205 | pancreatic endopeptidase E form B |
| S01.206 | pancreatic elastase II form B (Homo sapiens) |
| S01.207 | 9930019B18Rik protein |
| S01.209 | complement component C1rB (Mus musculus) |
| S01.210 | complement component C1sB (Mus musculus) |
| S01.211 | coagulation factor XIIa |
| S01.212 | plasma kallikrein |
| S01.213 | coagulation factor XIa |
| S01.214 | coagulation factor IXa |
| S01.215 | coagulation factor VIIa |
| S01.216 | coagulation factor Xa |
| S01.217 | thrombin |
| S01.218 | protein C (activated) |
| S01.219 | coagulation factor C (horseshoe crab), activated |
| S01.220 | coagulation factor B (Limulus, Tachypleus), activated |
| S01.221 | clotting enzyme (Tachypleus) |
| S01.222 | coagulation factor G (Tachypleus), activated |
| S01.223 | acrosin |
| S01.224 | hepsin |
| S01.225 | Stubble endopeptidase (Insecta) |
| S01.228 | hepatocyte growth factor activator |

(continued)

| CODE | Protease name |
|---|---|
| S01.229 | mannan-binding lectin-associated serine peptidase 2 |
| S01.231 | u-plasminogen activator |
| S01.232 | t-plasminogen activator |
| S01.233 | plasmin |
| S01.234 | peptidase 3 (Dermatophagoides-type) |
| S01.235 | acutobin |
| S01.236 | neurosin |
| S01.237 | neurotrypsin |
| S01.239 | plasminogen activator (Desmodus-type) |
| S01.240 | oviductin |
| S01.243 | lumbrokinase |
| S01.244 | neuropsin |
| S01.245 | ovochymase |
| S01.246 | kallikrein hK10 (Homo sapiens) |
| S01.247 | epitheliasin |
| S01.248 | putative peptidase similar to natural killer cell peptidase 1 (Rattus norwegicus) |
| S01.250 | testis serine peptidase 6 |
| S01.251 | prostase |
| S01.252 | brain-specific serine peptidase 4 |
| S01.253 | halystase |
| S01.254 | mast cell peptidase 8 (Mus musculus) |
| S01.255 | mekratin |
| S01.256 | chymopasin |
| S01.257 | kallikrein hK11 |
| S01.258 | trypsin-2 (Homo sapiens) |
| S01.260 | B1598 endopeptidase |
| S01.261 | streptogrisin A |
| S01.262 | streptogrisin B |
| S01.263 | SAM-P20 peptidase (Streptomyces sp.) |
| S01.265 | streptogrisin C |
| S01.266 | streptogrisin D |
| S01.267 | streptogrisin E |
| S01.268 | alpha-lytic endopeptidase |
| S01.269 | glutamyl endopeptidase I |
| S01.270 | exfoliatin A |
| S01.271 | glutamyl endopeptidase BL |
| S01.272 | glutamyl endopeptidase BS |
| S01.273 | peptidase Do |

(continued)

| CODE | Protease name |
|---|---|
| S01.274 | DegQ |
| S01.275 | DegS |
| S01.276 | yeast-lytic endopeptidase (Rarobacter) |
| S01.277 | HtrA1 peptidase |
| S01.278 | HtrA2 peptidase |
| S01.279 | DegP2 peptidase (chloroplast) |
| S01.280 | lysyl endopeptidase (bacteria) |
| S01.281 | arginyl endopeptidase |
| S01.282 | SplB g.p. (Staphylococcus aureus) |
| S01.283 | SplC g.p. (Staphylococcus aureus) |
| S01.284 | HtrA3 peptidase |
| S01.285 | HtrA4 peptidase |
| S01.286 | 1300019n10rik protein (Mus musculus) |
| S01.287 | kallikrein rK12 (Rattus norvegicus) |
| S01.288 | kallikrein rK8 (Rattus norvegicus) |
| S01.289 | arginine esterase (Canis familiaris) |
| S01.290 | renal kallikrein (Mastomys natalensis) |
| S01.291 | LOC144757 peptidase (Homo sapiens) |
| S01.292 | HAT-like putative peptidase 2 |
| S01.294 | HAT-like putative peptidase 3 |
| S01.297 | mouse kallikrein 15 |
| S01.298 | trypsin C |
| S01.300 | stratum corneum chymotryptic enzyme |
| S01.302 | matriptase |
| S01.303 | mast cell peptidase-11 (rodent) |
| S01.304 | mast cell peptidase-9 (Mus musculus) |
| S01.305 | prophenoloxidase-activating endopeptidase (Bombyx-type) |
| S01.306 | kallikrein hK13 |
| S01.307 | kallikrein hK9 |
| S01.308 | matriptase-2 |
| S01.309 | umbelical vein peptidase |
| S01.311 | LCLP peptidase |
| S01.313 | spinesin |
| S01.314 | strypsin-1 |
| S01.315 | strypsin-2 |
| S01.316 | 26 kDa endopeptidase (Sarcophaga peregrina) |
| S01.317 | testis serine peptidase 2 (Mus musculus) |
| S01.318 | marapsin-2 |

(continued)

| CODE | Protease name |
|---|---|
| S01.319 | complement factor D-like putative peptidase |
| S01.325 | epidermis-specific SP-like putative peptidase |
| S01.326 | testis serine peptidase 5 |
| S01.327 | testis serine peptidase 1 |
| S01.328 | Try10-like trypsinogen |
| S01.330 | catroxase I |
| S01.331 | pallabin |
| S01.332 | pallabin 2 |
| S01.333 | pallase |
| S01.334 | alpha-fibrinogenase (Vipera lebetina) |
| S01.335 | calobin (Gloydius sp.) |
| S01.336 | catroxobin I |
| S01.337 | cerastobin |
| S01.338 | salmobin |
| S01.339 | cerastotin |
| S01.340 | serpentokallikrein-1 (Trimeresurus mucrosquamatus) |
| S01.341 | brevinase |
| S01.342 | cerastocytin |
| S01.343 | mucofibrase 1 |
| S01.344 | mucofibrase 4 |
| S01.345 | mucofibrase 5 |
| S01.346 | okinaxobin I |
| S01.347 | contortrixobin |
| S01.348 | acubin |
| S01.349 | acubin2 |
| S01.350 | salmonase |
| S01.352 | elegaxobin (Trimeresurus elegans) |
| S01.353 | KN-BJ endopeptidase 1 (Bothrops jararaca) |
| S01.354 | KN-BJ endopeptidase 2 (Bothrops jararaca) |
| S01.355 | elegaxobin II (Trimeresurus elegans) |
| S01.356 | afaacytin (Cerastes cerastes) |
| S01.357 | polyserase-IA protein (unit 1) |
| S01.358 | polyserase-IA protein (unit 2) |
| S01.360 | complement component C1sA (Mus musculus) |
| S01.361 | peptidase similar to prostasin (Mus musculus) |
| S01.362 | testis serine peptidase 2 (Homo sapiens) |
| S01.363 | hypothetical acrosin-like peptidase (Homo sapiens) |
| S01.364 | htrA-like peptidase (Listeria-type) |

(continued)

| CODE | Protease name |
|---|---|
| S01.366 | HISP peptidase (Haemaphysalis longicornis) |
| S01.398 | granzyme D (Mus musculus) |
| S01.399 | granzyme E (Mus sp.) |
| S01.401 | granzyme F (Mus musculus) |
| S01.402 | granzyme G (Mus musculus) |
| S01.404 | granzyme RNKP-7 (Rattus) |
| S01.405 | kallikrein rK1 (Rattus) |
| S01.406 | kallikrein rK7 (Rattus) |
| S01.407 | kallikrein rK9 (Rattus) |
| S01.410 | kallikrein K-32 (Rattus norvegicus) |
| S01.412 | CHY1 peptidase (Metarhizium anisopliae) |
| S01.413 | prophenoloxidase-activating endopeptidase (Pacifastacus leniusculus) |
| S01.417 | testis-specific serine peptidase-1 |
| S01.418 | kallikrein 5-like peptidase |
| S01.419 | plasma kallikrein-like peptidase |
| S01.420 | prothrombin activator (Lonomia sp.) |
| S01.421 | Persephone endopeptidase (Drosophila melanogaster) |
| S01.422 | fibrinolytic enzyme A (Annelida) |
| S01.423 | SprE glutamyl peptidase (Enterococcus faecalis) |
| S01.424 | serine peptidase SP-28 (Ctenocephalides felis) |
| S01.425 | trocarin D |
| S01.426 | hopsarin D (Hoplocephalus stephensi) |
| S01.427 | prophenoloxidase-activating endopeptidase (Manduca-type) |
| S01.428 | LV-Ka endopeptidase |
| S01.429 | factor V activating enzyme (Vipera lebetina) |
| S01.430 | gabonase (Bitis gabonica) |
| S01.431 | SFase-2 endopeptidase (Streptomyces fradiae) |
| S01.432 | gyroxin (Crotalus durissus terrificus) |
| S01.433 | Bothrops peptidase A (Bothrops jararaca) |
| S01.434 | Nma111 endopeptidase (Saccharomyces cerevisiae) |
| S01.435 | gilatoxin (Heloderma horridum) |
| S01.436 | DESC4 peptidase |
| S01.437 | cod chymotrypsin B |
| S01.438 | fire ant chymotrypsin |
| S01.439 | cortical granule serine peptidase 1 (Strongylocentrotus sp.) |
| S01.440 | Vn50 peptidase (Cotesia rubecula) |
| S01.441 | SppA1 peptidase (Arabidopsis thaliana) |
| S01.442 | HtrA stress response protease (Brucella-type) |

(continued)

| CODE | Protease name |
|---|---|
| S01.443 | glutamyl endopeptidase BI |
| S01.444 | hemolymph proteinase 14 (Manduca sexta) |
| S03.001 | togavirin |
| S06.001 | IgA1-specific serine endopeptidase (Neisseria sp.) |
| S06.002 | EspP g.p. (Escherichia coli) |
| S06.003 | Tsh peptidase (Escherichia coli) |
| S06.004 | Pet peptidase |
| S06.005 | Pic peptidase (Shigella flexneri) |
| S06.006 | Hap serine peptidase |
| S06.007 | IgA1-specific serine peptidase type 1 (Haemophilus sp.) |
| S06.008 | IgA1-specific serine peptidase type 2 (Haemophilus sp.) |
| S06.009 | EatA peptidase (Escherichia coli) |
| S06.010 | EspC peptidase |
| S07.001 | flavivirin |
| S08.001 | subtilisin Carlsberg |
| S08.002 | mesentericopeptidase |
| S08.003 | subtilisin lentus |
| S08.004 | wprA g.p. (Bacillus-type) |
| S08.005 | peptidase Q (Bacillus pumilis) |
| S08.006 | P69 endopeptidase |
| S08.007 | thermitase |
| S08.009 | subtilisin Ak1 |
| S08.010 | M-peptidase (Bacillus sp. KSM-K16) |
| S08.011 | kexin-like peptidase (Pneumocystis carinii) |
| S08.012 | subtilisin-like peptidase 1 (Plasmodium sp.) |
| S08.013 | subtilisin-like peptidase 2 (Plasmodium-type) |
| S08.014 | ALE1 endopeptidase (Arabidopsis thaliana) |
| S08.016 | WF146 peptidase (Bacillus sp. WF146) |
| S08.017 | bacillopeptidase F |
| S08.018 | cell envelope-associated peptidase (Lactobacillus sp.) |
| S08.019 | lactocepin I |
| S08.020 | C5a peptidase |
| S08.021 | fervidolysin |
| S08.022 | basic serine peptidase (Dichelobacter) |
| S08.023 | acidic serine peptidase V5 (Dichelobacter) |
| S08.024 | trepolisin |
| S08.025 | antigen Pen ch 13 (Penicillium chrysogenum) |
| S08.026 | nasp g.p. (Dermatophilus congolensis) |

(continued)

| CODE | Protease name |
|---|---|
| S08.030 | IspA peptidase |
| S08.031 | blisterase |
| S08.032 | Psp3 protein (Schizosaccharomyces pombe) |
| S08.034 | subtilisin BPN’ |
| S08.035 | subtilisin J |
| S08.036 | subtilisin E |
| S08.037 | subtilisin DY |
| S08.038 | alkaline peptidase (Bacillus alcalophilus) |
| S08.039 | proprotein convertase 9 |
| S08.042 | subtilisin amylosacchariticus |
| S08.043 | HreP peptidase (Yersinia enterocolitica) |
| S08.044 | subtilisin NAT |
| S08.045 | subtilisin ALP 1 |
| S08.046 | subtilisin aprM |
| S08.047 | kpc-1 proprotein convertase |
| S08.048 | furin-1 (insect) |
| S08.049 | Furin-2 g.p. (Drosophila melanogaster) |
| S08.050 | exopeptidase A |
| S08.051 | aqualysin 1 |
| S08.052 | cerevisin |
| S08.053 | oryzin |
| S08.054 | endopeptidase K |
| S08.055 | alkaline endopeptidase (Yarrowia lipolytica) |
| S08.056 | cuticle-degrading endopeptidase |
| S08.057 | thermomycolin |
| S08.058 | subtilisin-like peptidase (Ophiostoma sp.) |
| S08.059 | NisP lantibiotic leader peptidase (Lactococcus lactis) |
| S08.060 | EpiP lantibiotic leader peptidase (Staphylococcus epidermidis) |
| S08.061 | peptidase T |
| S08.062 | antigen Pen c 1-type peptidase |
| S08.063 | site-1 peptidase |
| S08.064 | PrtA g.p. (Streptococcus pneumoniae) |
| S08.065 | MutP lantibiotic leader peptidase (Streptococcus mutans) |
| S08.067 | Apr g.p. (Alteromonas sp. O-7) |
| S08.068 | SphB1 autotransporter (Bordetella sp.) |
| S08.069 | SAM-P45 peptidase (Streptomyces sp.) |
| S08.070 | kexin |
| S08.071 | furin |

(continued)

| CODE | Protease name |
|---|---|
| S08.072 | proprotein convertase 1 |
| S08.073 | proprotein convertase 2 |
| S08.074 | proprotein convertase 4 |
| S08.075 | PACE4 proprotein convertase |
| S08.076 | proprotein convertase 5 |
| S08.077 | proprotein convertase 7 |
| S08.078 | vitellogenin convertase (Diptera) |
| S08.079 | PrcA peptidase |
| S08.080 | kexin-like peptidase (Tachypleus) |
| S08.083 | CP70 cold-active peptidase (Flavobacterium balustinum) |
| S08.084 | SDD1 peptidase |
| S08.085 | PepP lantibiotic leader peptidase (Staphylococcus epidermidis) |
| S08.086 | CylP/CylA lantibiotic leader peptidase (Enterococcus faecalis) |
| S08.087 | EciP lantibiotic leader peptidase (Staphylococcus epidermidis) |
| S08.090 | tripeptidyl-peptidase II |
| S08.091 | tripeptidyl-peptidase S |
| S08.092 | cucumisin |
| S08.093 | LasP lantibiotic leader peptidase (Lactobacillus sakei) |
| S08.094 | subtilisin extracellular homologue (Serratia) |
| S08.095 | ElkP lantibiotic leader peptidase (Staphylococcus epidermidis) |
| S08.096 | subtilisin homologue (Staphylothermus) |
| S08.097 | peptidase C1 (Glycine max) |
| S08.098 | subtilisin sendai |
| S08.100 | pyrolysin |
| S08.101 | halolysin 1 |
| S08.102 | halolysin R4 |
| S08.104 | AF70 peptidase (Picea abies) |
| S08.105 | aerolysin |
| S08.106 | stetterlysin |
| S08.107 | peptidase MprA (Burkholderia-type) |
| S08.108 | GSP peptidase (Clostridium sp.) |
| S08.109 | C51E3.7B protein (Caenorhabditis elegans) |
| S08.110 | StmPr1 endopeptidase (Stenotrophomonas-type) |
| S08.111 | AprP peptidase (Pseudomonas aeruginosa) |
| S08.112 | ARA12 g.p. (Arabidopsis thaliana) |
| S08.113 | sfericase (Bacillus sphaericus) |
| S08.114 | endopeptidase Vpr (Bacillus-type) |
| S08.115 | subtilisin-like peptidase 3 (Microsporum-type) |

(continued)

| CODE | Protease name |
|---|---|
| S08.116 | lactocepin III |
| S08.117 | FT peptidase |
| S08.118 | PrtB peptidase (Lactobacillus delbrueckii subsp. bulgaricus) |
| S08.119 | AIR3 peptidase |
| S08.120 | Aoz1 peptidase (Arthrobotrys oligospora) |
| S08.121 | cytotoxin SubA |
| S08.122 | subtilisin-like peptidase 3 (Plasmodium sp.) |
| S08.123 | KP-43 peptidase (Bacillus sp.) |
| S09.001 | prolyl oligopeptidase |
| S09.002 | prolyl oligopeptidase homologue (Pyrococcus sp.) |
| S09.003 | dipeptidyl-peptidase IV (eukaryote) |
| S09.004 | acylaminoacyl-peptidase |
| S09.005 | dipeptidyl aminopeptidase A |
| S09.006 | dipeptidyl aminopeptidase B (fungus) |
| S09.007 | fibroblast activation protein alpha subunit |
| S09.008 | dipeptidyl peptidase IV (Aspergillus sp.) |
| S09.010 | oligopeptidase B |
| S09.012 | dipeptidyl-peptidase V |
| S09.013 | dipeptidyl-peptidase IV (bacteria) |
| S09.014 | dipeptidyl aminopeptidase B1 (Pseudomonas sp.) |
| S09.016 | S9 homologue (invertebrate) |
| S09.017 | prolyl tripeptidyl peptidase |
| S09.018 | dipeptidyl-peptidase 8 |
| S09.019 | dipeptidyl-peptidase 9 |
| S09.021 | glutamyl endopeptidase (plant) |
| S09.051 | FLJ1 putative peptidase |
| S09.056 | dipeptidyl-peptidase IV, membrane-type (Giardia intestinalis) |
| S09.057 | apsC g.p. (Aspergillus niger N400) |
| S09.061 | C14orf29 protein |
| S09.062 | hypothetical protein |
| S09.063 | hypothetical esterase/lipase/thioesterase (Mus musculus) |
| S09.065 | protein bat5 |
| S09.067 | D230019K24Rik protein (Mus musculus) |
| S10.001 | carboxypeptidase Y |
| S10.002 | serine carboxypeptidase A |
| S10.003 | vitellogenic carboxypeptidase-like protein |
| S10.004 | serine carboxypeptidase C |
| S10.005 | serine carboxypeptidase D |

(continued)

| CODE | Protease name |
|---|---|
| S10.007 | kex carboxypeptidase |
| S10.008 | carboxypeptidase S1 (Penicillium janthinellum) |
| S10.009 | carboxypeptidase III (plant) |
| S10.010 | serine carboxypeptidase Z (Absidia zachae) |
| S10.011 | serine carboxypeptidase P |
| S10.012 | Sxa2 carboxypeptidase |
| S10.013 | RISC peptidase |
| S11.001 | D-Ala-D-Ala carboxypeptidase A |
| S11.002 | murein-DD-endopeptidase |
| S11.003 | penicillin-binding protein 6 |
| S11.004 | K15 DD-transpeptidase (Streptomyces sp.) |
| S11.005 | D-Ala-D-Ala carboxypeptidase DacF |
| S11.006 | D,D-carboxypeptidase PBP3 (Streptococcus sp.) |
| S12.001 | D-Ala-D-Ala carboxypeptidase B |
| S12.002 | aminopeptidase DmpB |
| S12.003 | alkaline D-peptidase (Bacillus sp.) |
| S12.004 | LACT-1 peptidase |
| S13.001 | D-Ala-D-Ala peptidase C |
| S13.002 | D-Ala-D-Ala carboxypeptidase (Actinomadura-type) |
| S13.003 | D-Ala-D-Ala carboxypeptidase PBP3 (Neisseria sp.) |
| S14.001 | endopeptidase Clp (type 1) |
| S14.002 | endopeptidase Clp (type 2) |
| S14.003 | endopeptidase Clp (type 3) |
| S14.004 | endopeptidase Clp (type 4) |
| S14.005 | endopeptidase Clp (type 5) |
| S14.006 | endopeptidase Clp (type 6) |
| S14.007 | endopeptidase Clp (type 7) |
| S14.008 | ClpP1 endopeptidase (Streptomyces-type) |
| S14.009 | ClpP2 endopeptidase (Streptomyces-type) |
| S15.001 | Xaa-Pro dipeptidyl-peptidase |
| S16.001 | Lon-A peptidase |
| S16.002 | PIM1 endopeptidase |
| S16.003 | endopeptidase La homologue (type 3) |
| S16.004 | Lon peptidase (type 4) |
| S16.005 | Lon-B peptidase |
| S21.001 | assemblin |
| S21.002 | cytomegalovirus assemblin |
| S21.003 | Epstein-Barr virus-type assemblin |

(continued)

| CODE | Protease name |
|---|---|
| S21.004 | herpesvirus 6-type assemblin |
| S21.005 | Varicella zoster assemblin |
| S21.006 | herpesvirus 8-type assemblin |
| S24.001 | repressor LexA |
| S24.002 | phage lambda repressor protein |
| S24.003 | UmuD protein |
| S24.004 | RvuZ homologue protein (Rattus) |
| S26.001 | signal peptidase I |
| S26.002 | mitochondrial inner membrane peptidase 1 |
| S26.003 | signal peptidase SipS |
| S26.004 | signal peptidase SipT |
| S26.005 | signal peptidase SipU |
| S26.006 | signal peptidase SipV |
| S26.007 | signal peptidase SipP |
| S26.008 | thylakoidal processing peptidase |
| S26.009 | signalase (eukaryote) 18 kDa component |
| S26.010 | signalase (eukaryote) 21 kDa component |
| S26.011 | signal peptidase SipW (Bacillus-type) |
| S26.012 | mitochondrial inner membrane peptidase 2 |
| S26.013 | mitochondrial signal peptidase (metazoa) |
| S26.014 | TraF peptidase |
| S26.015 | Streptococcus-type signal peptidase |
| S26.016 | signal peptidase SpsB (Staphylococcus aureus) |
| S26.017 | archaean signal peptidase (Methanococcus voltae) |
| S26.018 | signal peptidase SipM (Bacillus megaterium) |
| S26.019 | similar to type-I signal peptidase |
| S28.001 | lysosomal Pro-Xaa carboxypeptidase |
| S28.002 | dipeptidyl-peptidase II |
| S28.003 | thymus-specific serine peptidase |
| S29.001 | hepacivirin |
| S29.002 | hepatitis G virus NS3 endopeptidase |
| S30.001 | potyvirus P1 peptidase |
| S31.001 | pestivirus NS3 polyprotein peptidase |
| S32.001 | equine arteritis virus serine endopeptidase |
| S33.001 | prolyl aminopeptidase |
| S33.002 | tripeptidyl-peptidase A (Streptomyces sp.) |
| S33.003 | leucine aminopeptidase pepL |
| S33.004 | prolinase (Lactobacillus sp.) |

(continued)

| CODE | Protease name |
|---|---|
| S33.005 | tricorn interacting factor F1 |
| S33.006 | tripeptidyl-peptidase B |
| S33.007 | tripeptidyl-peptidase C (Streptomyces sp.) |
| S33.008 | prolyl aminopeptidase 2 |
| S33.010 | SCO7095 endopeptidase (Streptomyces coelicolor A3(2)) |
| S33.011 | epoxide hydrolase-like putative peptidase |
| S33.012 | Loc328574-like protein |
| S37.001 | PS-10 peptidase |
| S39.001 | sobemovirus peptidase |
| S39.002 | luteovirus peptidase |
| S41.001 | C-terminal processing peptidase-1 |
| S41.002 | C-terminal processing peptidase-2 |
| S41.004 | C-terminal processing peptidase-3 |
| S41.005 | tricorn core peptidase (archaea) |
| S41.006 | tricorn core peptidase (bacteria) |
| S41.007 | ctpB peptidase (Bacillus subtilis) |
| S45.001 | penicillin G acylase precursor |
| S45.002 | cephalosporin acylase precursor |
| S46.001 | dipeptidyl-peptidase 7 |
| S48.001 | HetR endopeptidase |
| S49.001 | signal peptide peptidase A |
| S49.002 | sohB endopeptidase |
| S49.003 | protein C (bacteriophage lambda) |
| S49.004 | peptidase IV (Arabidopsis thaliana) |
| S49.005 | protein 1510-N (Pyrococcus horikoshii) |
| S50.001 | infectious pancreatic necrosis birnavirus Vp4 peptidase |
| S50.002 | avian infectious bursal disease birnavirus Vp4 endopeptidase |
| S50.003 | Drosophila X virus Vp4 peptidase |
| S50.004 | blotched snakehead birnavirus Vp4 peptidase |
| S51.001 | dipeptidase E |
| S51.002 | alpha-aspartyl dipeptidase (eukaryote) |
| S51.003 | cyanophycinase |
| S53.001 | sedolisin |
| S53.002 | sedolisin-B |
| S53.003 | tripeptidyl-peptidase I |
| S53.004 | kumamolisin |
| S53.005 | kumamolisin-B |
| S53.006 | physarolisin |

(continued)

| CODE | Protease name |
|---|---|
| S53.007 | aorsin |
| S53.008 | physarolisin II |
| S53.009 | kumamolisin-As |
| S54.001 | Rhomboid-1 (Diptera) |
| S54.002 | rhomboid-like protein 2 |
| S54.004 | aarA protein (Providencia stuartii) |
| S54.005 | rhomboid-like protein 1 |
| S54.006 | ventrhoid transmembrane protein |
| S54.007 | Pcp1 protein (Saccharomyces cereviseae) |
| S54.008 | rhomboid-like protein 5 |
| S54.009 | PARL peptidase |
| S54.010 | Rhomboid-2 (Drosophila-type) |
| S54.011 | Rhomboid-3 (Drosophila melanogaster) |
| S54.012 | Rhomboid-4 (Drosophila melanogaster) |
| S54.013 | ROM-1 peptidase (Caenorhabditis elegans) |
| S54.014 | rhomboid YqgP (Bacillus subtilis) |
| S55.001 | SpoIVB peptidase |
| S58.001 | aminopeptidase DmpA |
| S59.001 | nucleoporin 145 |
| S60.001 | lactoferrin |
| S62.001 | influenza A PA endopeptidase |
| S63.001 | EGF-like module containing mucin-like hormone receptor-like 2 |
| S63.002 | CD97 antigen |
| S63.003 | EGF-like module containing mucin-like hormone receptor-like 3 |
| S63.004 | EGF-like module containing mucin-like hormone receptor-like 1 (Homo sapiens) |
| S63.005 | FLJ00015 protein (Homo sapiens) |
| S63.006 | FLJ00046 protein (Homo sapiens) |
| S63.008 | EGF-like module containing mucin-like hormone receptor-like 4 |
| S64.001 | Ssy5 endopeptidase (Sacchaomyces cerevisiae) |
| S9C.001 | glycylprolyl peptidase (Bacteroides gingivalis) |
| S9F.001 | peptidyl-glycinamidase |
| S9G.002 | dog pancreatic collagenase |
| S9G.005 | elastase-like enzyme, platelet |
| S9G.006 | tissue elastase |
| S9G.009 | macrophage chymotrypsin-like endopeptidase |
| S9G.012 | tryase |
| S9G.013 | guanidinobenzoatase |
| S9G.014 | clipsin |

(continued)

| CODE | Protease name |
|---|---|
| S9G.016 | thymus chromatin endopeptidase |
| S9G.018 | nuclear histone endopeptidase |
| S9G.023 | ingobsin snake venom coagulation factor X activator, serine-type (Bungarus |
| S9G.025 | fasciatus, Cerastes vipera, Ophiophagus hannah) |
| S9G.027 | scutelarin (Oxyuranus scutellatus) |
| S9G.031 | leucyl endopeptidase (Spinacia oleracea) |
| S9G.034 | metridin |
| S9G.035 | serine endopeptidase (Alternaria) |
| S9G.036 | collagenolytic endopeptidase (Entomophthora) |
| S9G.038 | endopeptidase So |
| S9G.040 | serine endopeptidase (Pseudomonas) |
| S9G.041 | peptidase V (Escherichia coli) |
| S9G.042 | peptidase Mi (Escherichia coli) |
| S9G.043 | peptidase Fa (Escherichia coli) |
| S9G.049 | extracellular serine endopeptidase (Arthrobacter) |
| S9G.050 | peptidase VI (Escherichia coli) |
| S9G.054 | profilaggrin endopeptidase 1 |
| S9G.055 | thermostable serine endopeptidase (Sulfolobus) |
| S9G.056 | sporangin |
| S9G.058 | beta-secretase Matsumoto |
| S9G.060 | amelopeptidase |
| S9G.061 | peptidase gp76 (Plasmodium falciparum) |
| S9G.062 | thrombocytin |
| S9G.063 | peptidase In (Escherichia coli) |
| S9G.064 | archealysin |
| S9G.065 | fish muscle prokallikrein |
| S9G.066 | mole salivary kallikrein |
| S9G.069 | apoptotic serine peptidase AP24 |
| S9G.072 | erythrocyte membrane high molecular mass peptidase |
| S9G.075 | LasD g.p. (Pseudononas aeruginosa) |
| S9G.077 | serine endopeptidase (Perkinsus marinus) |
| S9G.079 | plan Asp/Glu serine endopeptidase |
| S9G.081 | SP220K peptidase |
| S9G.082 | tryptase Clara |
| S9G.083 | soluble dipeptidyl-peptidase IV |
| S9G.084 | dipeptidyl-peptidase IV beta |
| S9G.087 | jerdonobin (Trimeresurus jerdonii) |
| S9G.088 | jerdofibrase (Trimeresurus jerdonii) |

(continued)

| CODE | Protease name |
|---|---|
| S9G.089 | flavovilase (Trimeresurus flavoviridis) |
| S9G.092 | M003 endopeptidase (Bothrops moojeni) |
| S9G.093 | MSP 1 endopeptidase (Bothrops moojeni) |
| S9G.094 | MSP 2 endopeptidase (Bothrops moojeni) |
| S9G.099 | pseutarin C (Pseudonaja textilis) |
| S9G.100 | okinaxobin II (Trimeresurus okinavensis) |
| S9G.101 | habutobin |
| S9G.103 | PofibS endopeptidase (Philodryas olfersii) |
| T01.002 | archaean proteasome, beta component |
| T01.005 | bacterial proteasome, beta component |
| T01.006 | HslV component of HsIUV peptidase |
| T01.007 | CodW component of CodWX peptidase |
| T01.010 | proteasome catalytic subunit 1 |
| T01.011 | proteasome catalytic subunit 2 |
| T01.012 | proteasome catalytic subunit 3 |
| T01.013 | proteasome catalytic subunit 1i |
| T01.014 | proteasome catalytic subunit 2i |
| T01.015 | proteasome catalytic subunit 3i |
| T01.016 | RIKEN cDNA 5830406J20 |
| T01.017 | protein serine kinase c17 (Homo sapiens) |
| T02.001 | glycosylasparaginase precursor |
| T02.002 | asparaginase |
| T02.004 | taspase-1 |
| T02.005 | asparaginase-like sperm autoantigen homolog |
| T02.006 | hypothetical protein flj22316 |
| T03.001 | gamma-glutamyltransferase 1 (bacterial) |
| T03.002 | gamma-glutamyltransferase 5 (mammalian) |
| T03.005 | gamma-glutamyltransferase (Drosophila melanogaster) |
| T03.006 | gamma-glutamyltransferase 1 (mammalian) |
| T03.007 | gamma-glutamyltransferase CG4829 (Drosophila melanogaster) |
| T03.008 | gamma-glutamyltransferase (plant) |
| T03.009 | gamma-glutamyltransferase (nematode) |
| T03.010 | gamma-glutamyltransferase CG1492 (Drosophila melanogaster) |
| T03.011 | gamma-glutamyltransferase (Schizosaccharomyces) |
| T03.012 | gamma-glutamyltransferase (Saccharomyces) |
| T03.013 | gamma-glutamyltransferase (Synechocystis-type) |
| T03.014 | gamma-glutamyltransferase 2 (bacterial) |
| T03.015 | gamma-glutamyltransferase 2 (Homo sapiens) |

(continued)

| CODE | Protease name |
|---|---|
| T03.016 | gamma-glutamyltransferase-like protein 4 |
| T03.017 | gamma-glutamyltransferase-like protein 3 |
| T03.018 | similar to gamma-glutamyltransferase 1 precursor (Homo sapiens) |
| T03.020 | gamma-glutamyltransferase-like protein A4 |
| T03.022 | 9030405D14Rik protein (Mus musculus) |
| T05.001 | ornithine acetyltransferase precursor |

**Sequence Listing, Free Text**

| | |
|---|---|
| SEQ ID NOs: 1 TO 4 | PRIIMER P1 TO P4 |
| SEQ ID NO: 5 | human cationic trypsin |
| SEQ ID NO: 6 | human Anionic trypsin (Trypsin-2 precursor) |
| SEQ ID NO: 7 | human Mesotrypsin (Trypsin-3 precursor) |

A protease with reduced sensitivity towards activity-modulating substances being derived from a serine protease of the structural class S1 and having one or more mutations at positions selected from the group of positions that correspond structurally or by amino acid sequence homology to the regions or positions 18-28, 34-41, 46-68, 78, 90-102, 110-120, 123-137, 162-186, 195 or 214 in wild-type human cationic trypsin with the amino acid sequence shown in SEQ ID NO:5, or a modified form thereof.

The protease as defined above, which is derived from a trypsin-like protease, more preferably is derived from a human trypsin, most preferably is derived from human cationic trypsin with the amino acid sequence shown in SEQ ID NO: 5.

The protease as defined above, having one or more mutations at one or more positions from the group of positions that correspond structurally or by amino acid sequence homology to the regions 20-26, 36-39, 51-59, 63-67, 78, 92-99, 112-118, 124-128, 131-134, 172-184, 195 or 214 in human trypsin, preferably at one or more of the following positions 21, 22, 23, 24, 28, 37, 39, 46, 52, 55, 56, 57, 64, 66, 67, 78, 92, 93, 98, 99, 112, 115, 118, 124, 125, 128, 131, 133, 163, 172, 174, 181, 183, 184, 195 and 214, and more preferably at one or more of the following positions 22, 23, 24, 37, 52, 57, 64 and 133 numbered according to the amino acid sequence shown in SEQ ID NO:5.

The protease as defined above, which has at least one substitution or any combination of substitutions selected from the group of substitutions:

G at position 21 is substituted by A, D, S or V, preferably D or V;
Y at position 22 is substituted by T, H, Q, S, W, G or A, preferably by T or H;
H at position 23 is substituted by T, N, G, D, R or Y, preferably by T or N;
F at position 24 is substituted by I, V, Q, T, L or A, preferably by I or V;
S at position 28 is substituted by A;
S at position 37 is substituted by T;
G at position 39 is substituted by S;
I at position 46 is substituted by V, N, L or T, preferably by V;
E at position 52 is substituted by V or M, preferably by V;
N at position 54 is substituted by S;
I at position 55 is substituted by T, N or R, preferably by T or N;
E at position 56 is substituted by G or R, preferably by G;
V at position 57 is substituted by A, T or G, preferably by A;
F at position 64 is substituted by I or T, preferably by I;
N at position 66 is substituted by D;
A at position 67 is substituted by V;
R at position 78 is substituted by W;
S at position 92 is substituted by T;
R at position 93 is substituted by P;
A at position 98 is substituted by D;

R at position 99 is substituted by H;
T at position 112 is substituted by A or P, preferably by A;
K at position 115 is substituted by M;
I at position 118 is substituted by V;
T at position 124 is substituted by K or I, preferably by K;
A at position 125 is substituted by P or S, preferably by P;
G at position 128 is substituted by R, K or T, preferably by R;
Y at position 131 is substituted by F, N or H, preferably by F;
D at position 133 is substituted by G;
V at position 163 is substituted by A;
S at position 172 is substituted by T;
Q at position 174 is substituted by R;
V at position 181 is substituted by A;
C at position 183 is substituted by H, Q or R, preferably by H;
N at position 184 is substituted by K or D;
D at position 195 is substituted by E; and
K at position 214 is substituted by E, D, R, T, or V, preferably by E.

A protease as defined above, which has at least one group of substitutions selected from the group of substitutions:

Y22T, H23T, F24I or F24V, S37T, E52V, V57A, F64I, D133G;
S37T, E52V, V57A, F64I, D133G;
Y22T, H23T, F24I, S37T, E52V, V57A, F64I, D133G;
Y22T, F24V, S37T, E52V, V57A, F64I, D133G;
S37T, E52V, E56G, V57A, F64I, R78W, D133G, C183H;
Y22T, H23T, F24I, S37T, E52V, E56G, V57A, F64I, R78W, D133G, C183H;
Y22H, F24V, S37T, E52V, E56G, V57A, F64I, R78W, D133G, C183H;
Y22T, H23T, F24I, S37T, E52V, I55N, E56G, V57A, L58A, E59Q, F64T, R78W, R93P, T124K, A125P, G128R, Y131H, D133G, L135V, D139N, V163A, C183H, D195E, D214E;
G21D, Y22T, H23T, F24I, S28A, S37T, E52V, N54S, I55T, E56G, V57A, F64I, R78W, R93P, R99H, T124K, A125P, D133G, V163A, C183H, D195E, K214E;
G21V, Y22T, H23T, F24I, S28A, S37T, E52M, N54S, I55T, E56R, V57A, F64I, R78W, S92T, R93P, A98D, R99H, T112A, T124K, A125P, D133G, V163A, S172T, C183Q, D195E, K214E; and
G21D, Y22T, H23T, F24I, S28A, S37T, G39S, I46T, E52M, N54S, I55T, E56G, V57A, F64I, A67V, R78W, S92T, R93P, A98D, R99H, T112A, K115M, I118V, T124K, A125P, D133G, V163A, S172T, V181A, C183Q, N184D, D195E, K214E;

where the numbering of the described substitutions refers to wild-type human cationic trypsin with the amino acid sequence shown in SEQ ID NO:5.
The protease as defined above, which is covalently linked to

(i) at least one further proteinacious component, preferably said proteinacious component is fused to the protease and being selected from the group consisting of binding domains, receptors, antibodies, regulation domains, pro-sequences, and fragments thereof, and/or
(ii) at least one further functional component, preferably said further functional component being selected from the group consisting of polyethylenglycols, carbohydrates, lipids, fatty acids, nucleic acids, metals, metal chelates, and fragments or derivatives thereof.

The protease as defined above, wherein the protease has a reduced sensitivity towards activity-modulating substances present within an application matrix as compared to the wild type serine protease of the structural class S1, preferably

(i) the application matrix is derived from a human or animal body fluid selected from the group consisting of blood, digestive fluids, preferably intestinal and gastric juice, mucosa, synovial fluid, interstitial fluid, mucosal fluid, cerebrospinal fluid, peritoneal fluid, or from the extracellular matrix; and/or
(ii) the activity-modulating substance is selected from table 1, and/or is a human protease inhibitor, preferably a serpin selected from the group consisting of alphal-antitrypsin, alpha1-antichymotrypsin, kallistatin, protein C-inhibitor, leucocyte elastase inhibitor, plasminogen activator inhibitor, maspin, serpin B6, megsin, serpin B9, serpin B10, serpin B11, serpin B12, serpin B13, antithrombin, heparin cofactor, plasminogen activator inhibitor, alpha-2-plasmin inhibitor, C1-inhibitor, neuroserpin, serpin I2 and thyroxin-binding globulin; a cystein protease inhibitor selected from the group consisting of cystatin A, cystatin B, cystatin C, cystatin D, cystatin E/M, cystatin F, cystatin S, cystatin SA, cystatin SN, cystatin G, kininogen inhibitor unit 2 and/or kininogen inhibitor unit 3; a metallo protease inhibitor selected from the group consisting of TIMP-1, TIMP-2, TIMP-3 and/or TIMP-4; macroglobulins, preferably alpha2-macroglobulin; BIRC-1; BIRC-2; BIRC-3; BIRC-4; BIRC-5; BIRC-6; BIRC-7 or BIRC-8.

A DNA encoding the protease as defined above.

A vector comprising the DNA as defined above.

A cell transformed/transfected with the vector as defined above and/or containing the DNA as defined above.

A method for preparing the protease according to any one of claims 1 to 7, which method comprises cuturing the cell as defined above and isolating the protease from the culture broth and/or the cell culture.

A pharmaceutical, diagnostic or cosmetic composition comprising the protease as defined above.

Use of the protease as defined above for preparing a medicament for preventing or treating a desease curable by protease therapy.

A method for generating a protease, preferably a protease as defined above, having reduced sensitivity towards activity-modulating substances present within an application matrix, comprising

(a) providing a library of one or more proteases derived from one or more parent proteases,
(b) contacting the proteases with at least one activity-modulating substance, and
(c) selecting one or more protease variants with reduced sensitivity towards activity-modulating substances as compared to the parent protease(s).

## Claims

1. A protease with reduced sensitivity towards activity-modulating substances being derived from a serine protease of the structural class S1 and having one or more mutations at positions selected from the group of positions that correspond structurally or by amino acid sequence homology to the positions 24, 22, 37, 21, 23, 28, 39, 46, 52, 55, 56, 57, 64, 66, 67, 78, 92, 93, 98, 99, 112, 115, 118, 124, 125, 128, 131, 133, 163, 172, 174, 181, 183, 184, 195 and 214 in wild-type human cationic trypsin with the amino acid sequence shown in SEQ ID NO:5, or a modified form thereof.

2. The protease according to claim 1 having one or more mutations at one or more of the following positions 24, 22, 37, 23, 52, 57, 64 and 133 numbered according to the amino acid sequence shown in SEQ ID NO:5.

3. The protease of claim 1 or 2, which is derived from a trypsin-like protease, more preferably is derived from a human trypsin, most preferably is derived from human cationic trypsin with the amino acid sequence shown in SEQ ID NO: 5.

4. The protease according to any of claims 1 to 3, which has at least one substitution or any combination of substitutions selected from the group of substitutions:

   F at position 24 is substituted by I, V, Q, T, L or A, preferably by I or V;
   Y at position 22 is substituted by T, H, Q, S, W, G or A, preferably by T or H;
   S at position 37 is substituted by T;
   G at position 21 is substituted by A, D, S or V, preferably D or V;
   H at position 23 is substituted by T, N, G, D, R or Y, preferably by T or N;
   S at position 28 is substituted by A;
   G at position 39 is substituted by S;
   I at position 46 is substituted by V, N, L or T, preferably by V;
   E at position 52 is substituted by V or M, preferably by V;
   N at position 54 is substituted by S;
   I at position 55 is substituted by T, N or R, preferably by T or N;
   E at position 56 is substituted by G or R, preferably by G;
   V at position 57 is substituted by A, T or G, preferably by A;
   F at position 64 is substituted by I or T, preferably by I;
   N at position 66 is substituted by D;
   A at position 67 is substituted by V;
   R at position 78 is substituted by W;
   S at position 92 is substituted by T;
   R at position 93 is substituted by P;
   A at position 98 is substituted by D;
   R at position 99 is substituted by H;
   T at position 112 is substituted by A or P, preferably by A;
   K at position 115 is substituted by M;
   I at position 118 is substituted by V;
   T at position 124 is substituted by K or I, preferably by K;

A at position 125 is substituted by P or S, preferably by P;
G at position 128 is substituted by R, K or T, preferably by R;
Y at position 131 is substituted by F, N or H, preferably by F;
D at position 133 is substituted by G;
V at position 163 is substituted by A;
S at position 172 is substituted by T;
Q at position 174 is substituted by R;
V at position 181 is substituted by A;
C at position 183 is substituted by H, Q or R, preferably by H;
N at position 184 is substituted by K or D;
D at position 195 is substituted by E; and
K at position 214 is substituted by E, D, R, T, or V, preferably by E.

**5.** A protease according to any of claims 1 to 3, which has at least one group of substitutions selected from the group of substitutions:

Y22T, H23T, F24I or F24V, S37T, E52V, V57A, F64I, D133G;
S37T, E52V, V57A, F64I, D133G;
Y22T, H23T, F24I, S37T, E52V, V57A, F64I, D133G;
Y22T, F24V, S37T, E52V, V57A, F64I, D133G;
S37T, E52V, E56G, V57A, F64I, R78W, D133G, C183H;
Y22T, H23T, F24I, S37T, E52V, E56G, V57A, F64I, R78W, D133G, C183H;
Y22H, F24V, S37T, E52V, E56G, V57A, F64I, R78W, D133G, C183H;
Y22T, H23T, F24I, S37T, E52V, I55N, E56G, V57A, L58A, E59Q, F64T, R78W, R93P, T124K, A125P, G128R, Y131H, D133G, L135V, D139N, V163A, C183H, D195E, D214E;
G21D, Y22T, H23T, F24I, S28A, S37T, E52V, N54S, I55T, E56G, V57A, F64I, R78W, R93P, R99H, T124K, A125P, D133G, V163A, C183H, D195E, K214E;
G21V, Y22T, H23T, F24I, S28A, S37T, E52M, N54S, I55T, E56R, V57A, F64I, R78W, S92T, R93P, A98D, R99H, T112A, T124K, A125P, D133G, V163A, S172T, C183Q, D195E, K214E; and
G21D, Y22T, H23T, F24I, S28A, S37T, G39S, I46T, E52M, N54S, I55T, E56G, V57A, F64I, A67V, R78W, S92T, R93P, A98D, R99H, T112A, K115M, I118V, T124K, A125P, D133G, V163A, S172T, V181A, C183Q, N184D, D195E, K214E;

where the numbering of the described substitutions refers to wild-type human cationic trypsin with the amino acid sequence shown in SEQ ID NO:5.

**6.** The protease according to any of claims 1 to 5, which is covalently linked to

(i) at least one further proteinacious component, preferably said proteinacious component is fused to the protease and being selected from the group consisting of binding domains, receptors, antibodies, regulation domains, pro-sequences, and fragments thereof, and/or
(ii) at least one further functional component, preferably said further functional component being selected from the group consisting of polyethylenglycols, carbohydrates, lipids, fatty acids, nucleic acids, metals, metal chelates, and fragments or derivatives thereof.

**7.** The protease according to any of claims 1 to 5, wherein the protease has a reduced sensitivity towards activity-modulating substances present within an application matrix as compared to the wild type serine protease of the structural class S1, preferably

(i) the application matrix is derived from a human or animal body fluid selected from the group consisting of blood, digestive fluids, preferably intestinal and gastric juice, mucosa, synovial fluid, interstitial fluid, mucosal fluid, cerebrospinal fluid, peritoneal fluid, or from the extracellular matrix; and/or
(ii) the activity-modulating substance is selected from table 1, and/or is a human protease inhibitor, preferably a serpin selected from the group consisting of alphal-antitrypsin, alphai-antichymotrypsin, kallistatin, protein C-inhibitor, leucocyte elastase inhibitor, plasminogen activator inhibitor, maspin, serpin B6, megsin, serpin B9, serpin B10, serpin B11, serpin B12, serpin B13, antithrombin, heparin cofactor, plasminogen activator inhibitor, alpha-2-plasmin inhibitor, C1-inhibitor, neuroserpin, serpin 12 and thyroxin-binding globulin; a cystein protease inhibitor selected from the group consisting of cystatin A, cystatin B, cystatin C, cystatin D, cystatin E/M, cystatin

F, cystatin S, cystatin SA, cystatin SN, cystatin G, kininogen inhibitor unit 2 and/or kininogen inhibitor unit 3; a metallo protease inhibitor selected from the group consisting of TIMP-1, TIMP-2, TIMP-3 and/or TIMP-4; macroglobulins, preferably alpha2-macroglobulin; BIRC-1; BIRC-2; BIRC-3; BIRC-4; BIRC-5; BIRC-6; BIRC-7 or BIRC-8.

**8.** A DNA encoding the protease according to any one of claims 1 to 7.

**9.** A vector comprising the DNA of claim 8.

**10.** A cell transformed/transfected with the vector of claim 9 and/or containing the DNA of claim 8.

**11.** A method for preparing the protease according to any one of claims 1 to 7, which method comprises cuturing the cell of claim 10 and isolating the protease from the culture broth and/or the cell culture.

**12.** A pharmaceutical, diagnostic or cosmetic composition comprising the protease according to any one of claims 1 to 7.

**13.** Use of the protease according to any one of claims 1 to 7 for preparing a medicament for preventing or treating a desease curable by protease therapy.

**14.** A method for generating a protease according to any one of claims 1 to 7, having reduced sensitivity towards activity-modulating substances present within an application matrix, comprising

(a) providing a library of one or more proteases derived from one or more parent proteases,
(b) contacting the proteases with at least one activity-modulating substance, and
(c) selecting one or more protease variants with reduced sensitivity towards activity-modulating substances as compared to the parent protease(s).

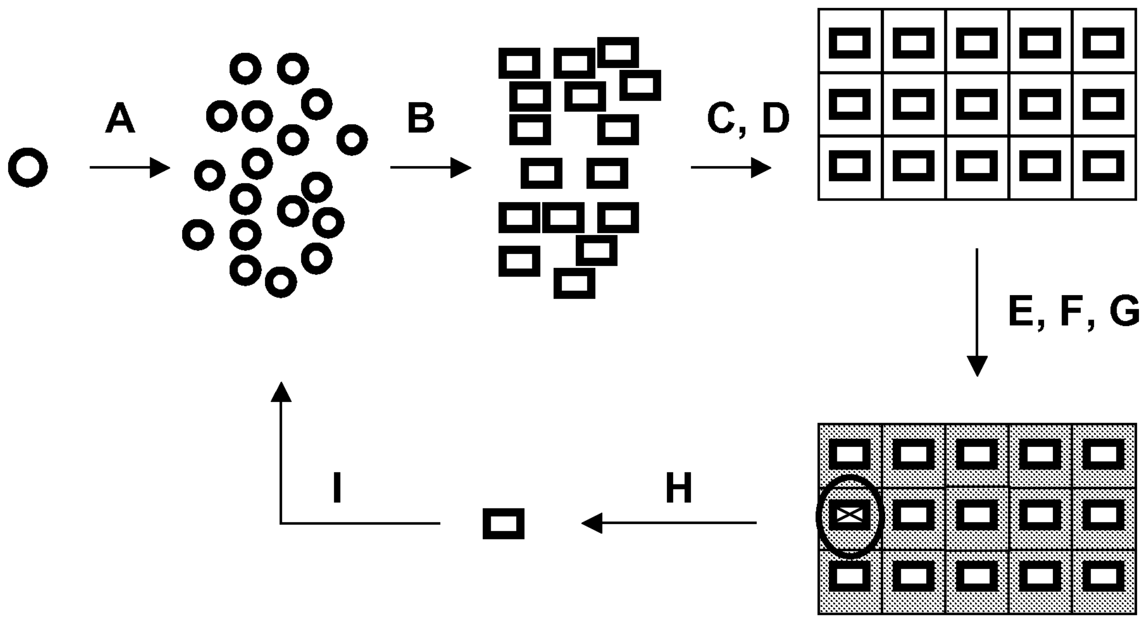


Fig. 1

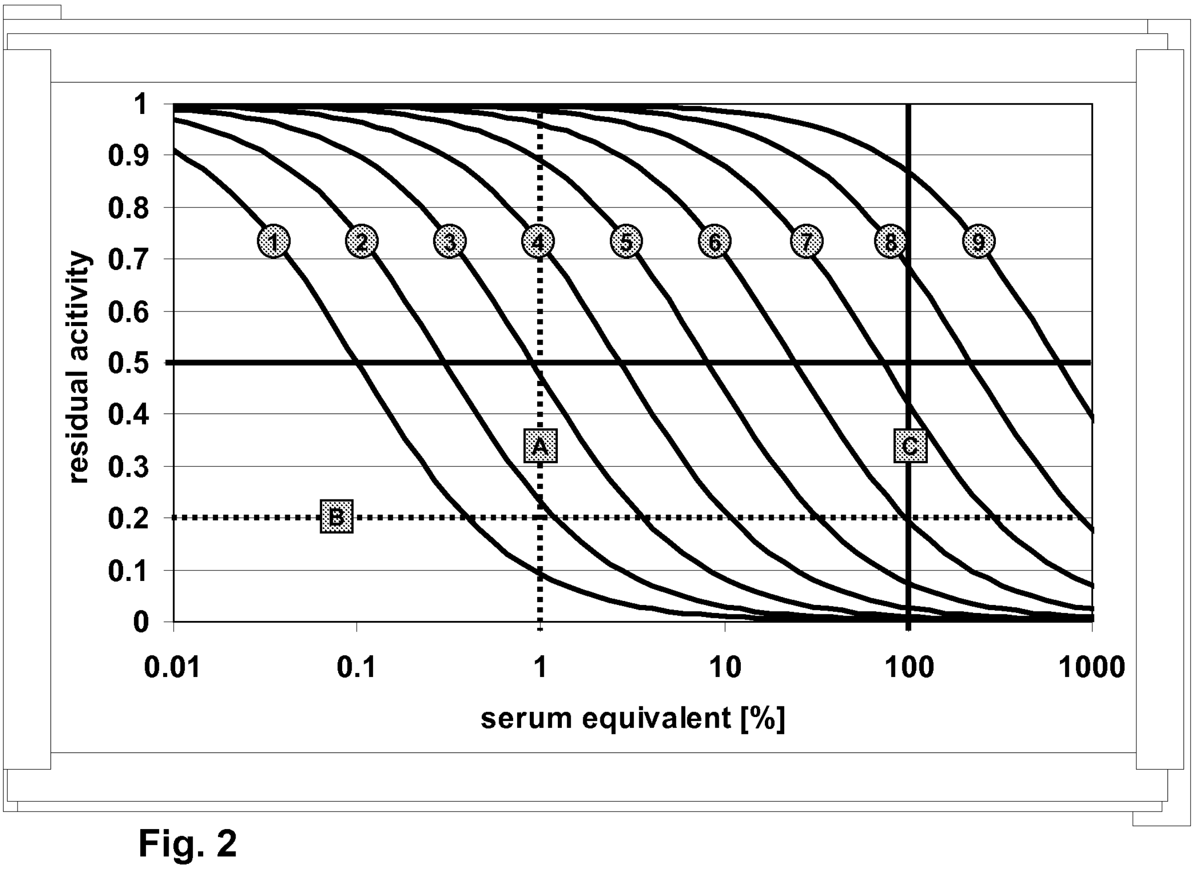
1
0.9
0.8
0.7
0.6
0.5
0.4
0.3
0.2
0.1
0
residual acitivity
0.01
0.1
1
10
100
1000
serum equivalent [%]
1
2
3
4
5
6
7
8
9
A
B
C

Fig. 2

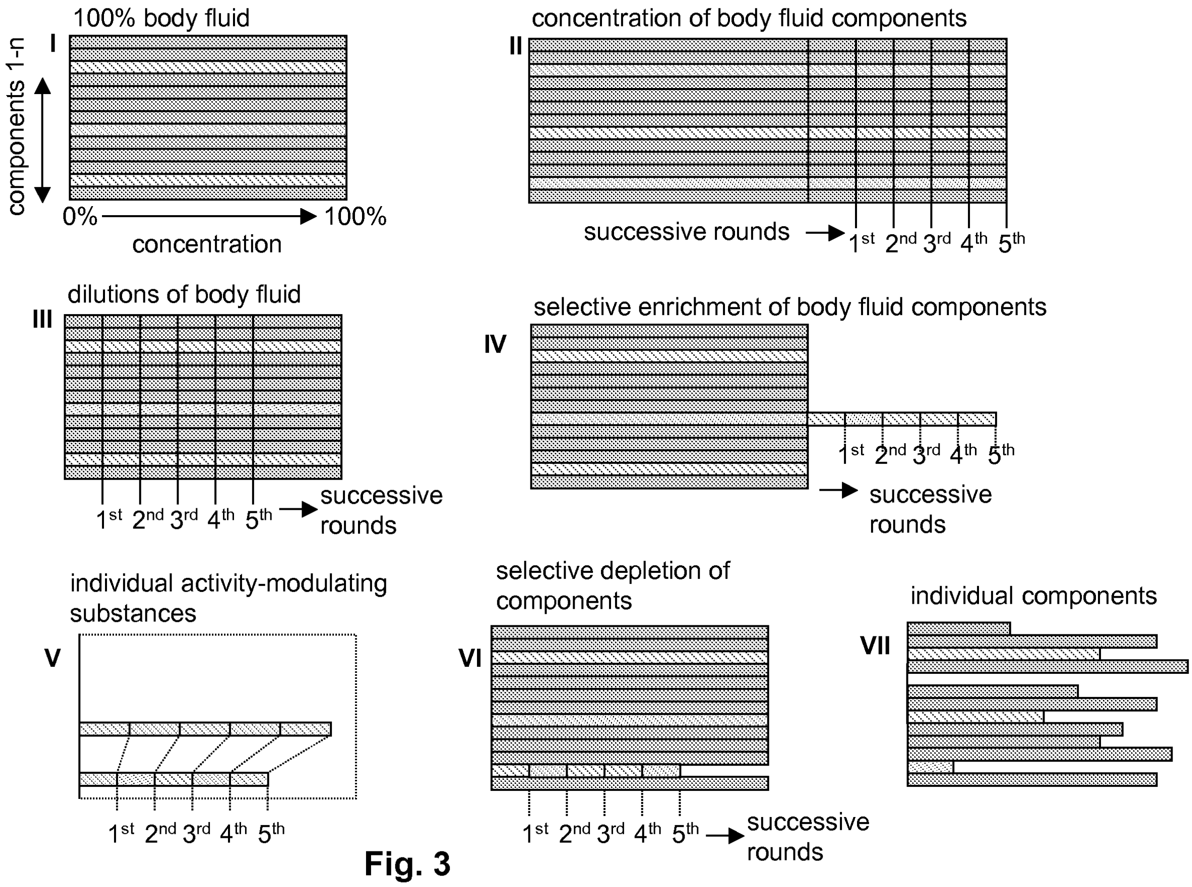

100% body fluid
I
components 1-n
0%
100%
concentration
concentration of body fluid components
II
successive rounds
1st 2nd 3rd 4th 5th
dilutions of body fluid
III
1st 2nd 3rd 4th 5th
successive rounds
selective enrichment of body fluid components
IV
1st 2nd 3rd 4th 5th
successive rounds
individual activity-modulating substances
V
1st 2nd 3rd 4th 5th
selective depletion of components
VI
1st 2nd 3rd 4th 5th
successive rounds
individual components
VII


Fig. 3

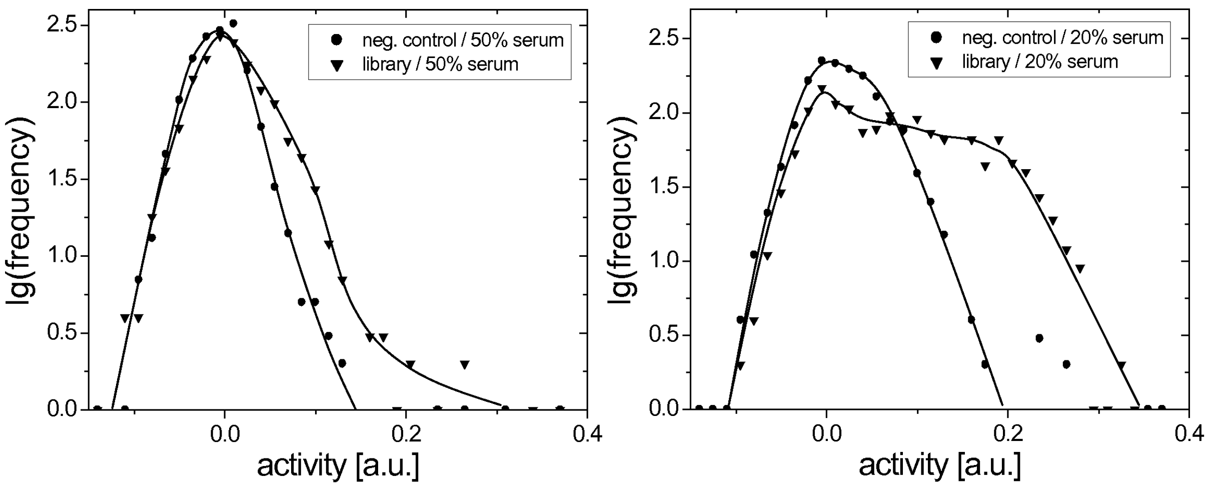
2.5
2.0
1.5
1.0
0.5
0.0
lg(frequency)
neg. control / 50% serum
library / 50% serum
0.0
0.2
0.4
activity [a.u.]
2.5
2.0
1.5
1.0
0.5
0.0
lg(frequency)
neg. control / 20% serum
library / 20% serum
0.0
0.2
0.4
activity [a.u.]

**Fig. 4**

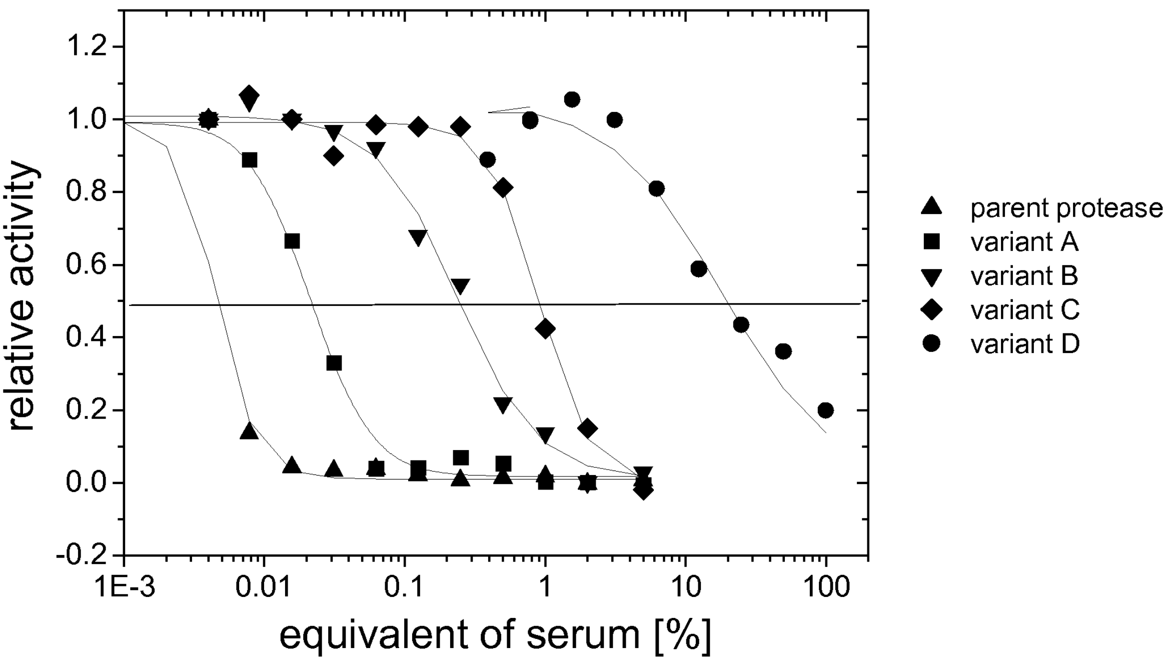
1.2
1.0
0.8
0.6
0.4
0.2
0.0
-0.2
relative activity
1E-3
0.01
0.1
1
10
100
equivalent of serum [%]
parent protease
variant A
variant B
variant C
variant D

**Fig. 5**

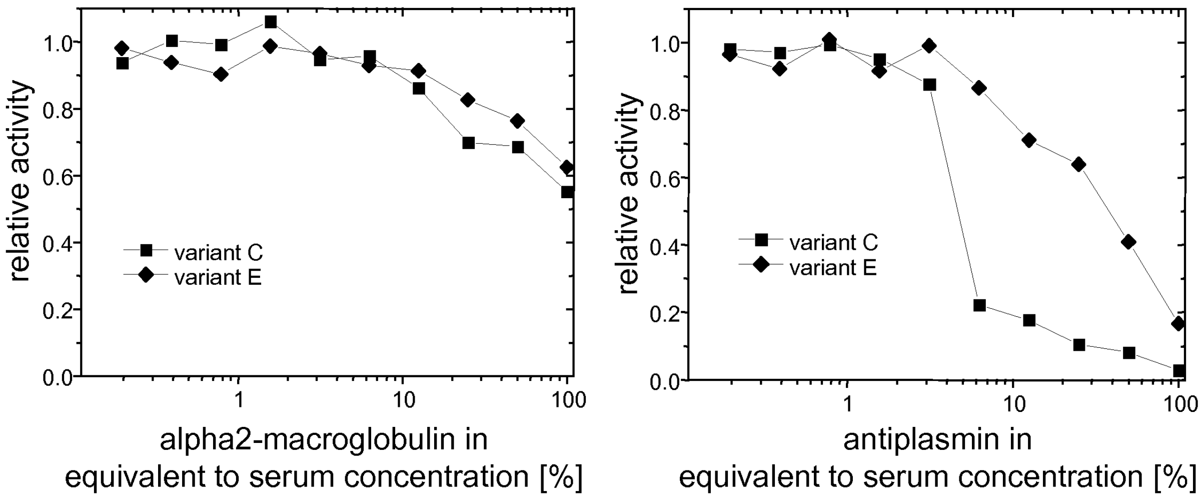
relative activity
1.0
0.8
0.6
0.4
0.2
0.0
1
10
100
variant C
variant E
alpha2-macroglobulin in
equivalent to serum concentration [%]
relative activity
1.0
0.8
0.6
0.4
0.2
0.0
1
10
100
variant C
variant E
antiplasmin in
equivalent to serum concentration [%]

**Fig. 6**

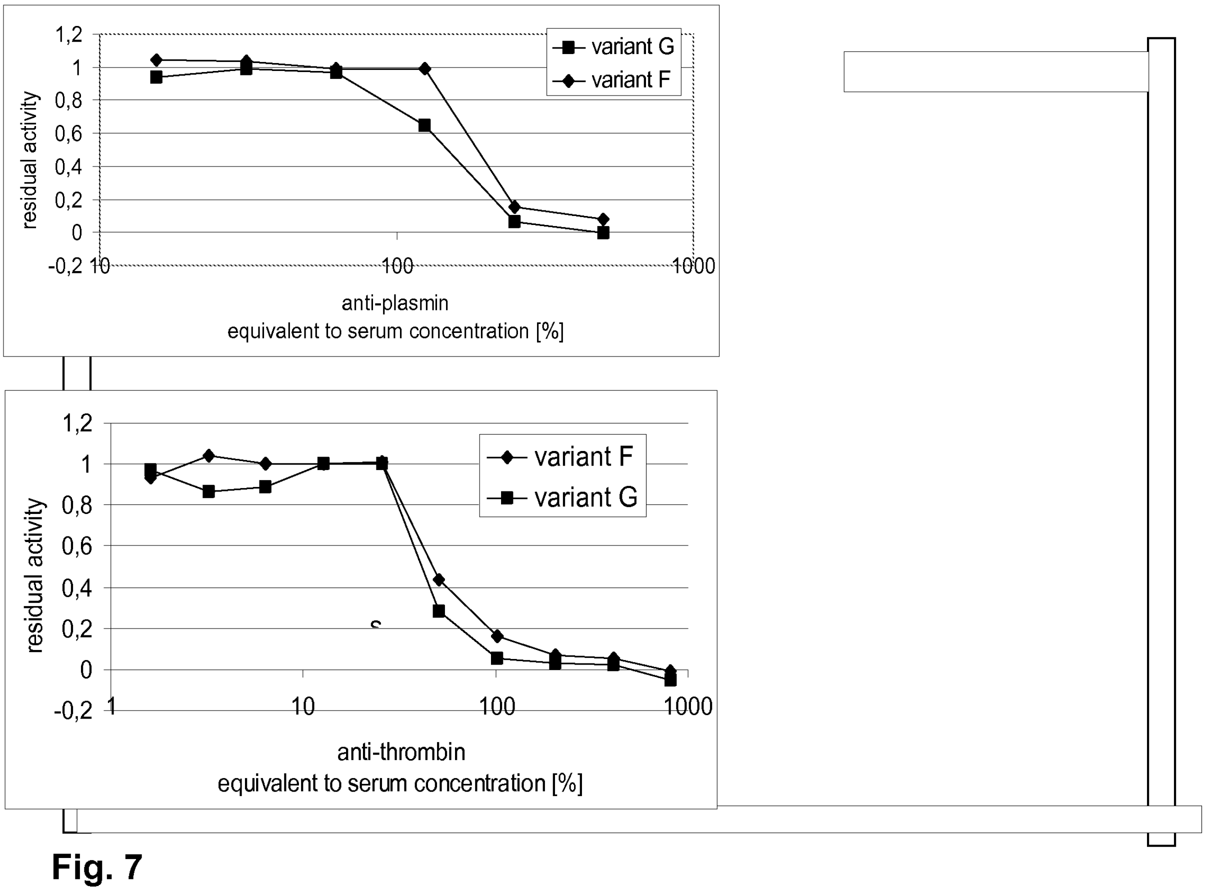
variant G
variant F
residual activity
1,2
1
0,8
0,6
0,4
0,2
0
-0,2
10
100
1000
anti-plasmin
equivalent to serum concentration [%]
variant F
variant G
residual activity
1
10
100
1000
anti-thrombin
equivalent to serum concentration [%]

**Fig. 7**

**Sequence Alignment Human Trypsins**

```
TRY1_HUMAN       IVGGYNCEENSVPYQVSLNSGYHFCGGSLINEQWVVSAGHCYKSRIQVRLGEHNIEVLEG
TRY2_HUMAN       IVGGYICEENSVPYQVSLNSGYHFCGGSLISEQWVVSAGHCYKSRIQVRLGEHNIEVLEG
TRY3_HUMAN       IVGGYTCEENSLPYQVSLNSGSHFCGGSLISEQWVVSAAHCYKTRIQVRLGEHNIKVLEG
                 ***** ***** ********* ******** ******* **** *********** ****

TRY1_HUMAN       NEQFINAAKIIRHPQYDRKTLNNDIMLIKLSSRAVINARVSTISLPTAPPATGTKCLISG
TRY2_HUMAN       NEQFINAAKIIRHPKYNSRTLDNDILLIKLSSPAVINSRVSAISLPTAPPAAGTESLISG
TRY3_HUMAN       NEQFINAAKIIRHPKYNRDTLDNDIMLIKLSSPAVINARVSTISLPTAPPAAGTECLISG
                 ************** *   ** *** ****** **** *** ********* **  ****

TRY1_HUMAN       WGNTASSGADYPDELQCLDAPVLSQAKCEASYPGKITSNMFCVGFLEGGKDSCQGDSGGP
TRY2_HUMAN       WGNTLSSGADYPDELQCLDAPVLSQAECEASYPGKITNNMFCVGFLEGGKDSCQGDSGGP
TRY3_HUMAN       WGNTLSFGADYPDELKCLDAPVLTQAECKASYPGKITNSMFCVGFLEGGKDSCQRDSGGP
                 **** * ******** ******* ** * ********  *************** *****

TRY1_HUMAN       VVCNGQLQGVVSWGDGCAQKNKPGVYTKVYNYVKWIKNTIAANS
TRY2_HUMAN       VVSNGELQGIVSWGYGCAQKNRPGVYTKVYNYVDWIKDTIAANS
TRY3_HUMAN       VVCNGQLQGVVSWGHGCAWKNRPGVYTKVYNYVDWIKDTIAANS
                 ** ** *** **** *** ** *********** *** ******
```

# Fig. 8

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 1419248 B1
- EP 1230390 B1
- DE 19646372 C1
- EP 1307482 A
- WO 2004113521 A
- WO 9416313 A


### Non-patent literature cited in the description


- **RINDERKNECHT H. et al.** Mesotrypsin: A new inhibitor-resistant protease from a zymogen in human pancreatic tissue and fluid. *Gastroenterology,* 1984, vol. 86, 681-92
- **KURSCHUS et al.** Killing of target cells by redirected granzyme B in the absence of perforin. *FEBS Letters,* 2004, vol. 562, 87-92
- **CADWELL RC ; JOYCE GF.** *Mutagenic PCR PCR Methods and Applications,* 1994, vol. 3, 136-140
- **CADWELL RC ; JOYCE GF.** *Randomization of Genes by PCR Mutagenesis PCR Methods and Applications,* 1992, vol. 2, 28-33
- **SPEE JH et al.** Efficient random mutagenesis method with adjustable mutation frequency by use of PCR and dITP. *Nucleic Acid Research,* 1993, vol. 3, 777-778
- **HO SN et al.** Site-directed mutagenesis by overlap extension using the polymerase chain reaction. *Gene,* 1989, vol. 77, 51-59
- **HORTON RM et al.** Engineering hybrid genes without the use of restriction proteases: gene splicing by overlap extension. *Gene,* 1989, vol. 77, 61-68
- Introduction to phage biology and phage display. **RUSSEL M ; LOWMAN HB ; CLACKSON T.** Phage display - a practical approach. Oxford University Press, 2004, 1-26
- **TAWFIK DS ; GRIFFITHS AD.** Man-made cell-like compartments for molecular evolution. *Nature Biotechnology,* 1998, vol. 16, 652-656
- **BERNATH K et al.** In vitro compartmentalization by double emulsions: sorting and gene enrichment by fluorescence activated cell sorting. *Analytical Biochemistry,* 2004, vol. 325, 151-157
- **RAWLING ND et al.** Evolutionary families of peptidase inhibitors. *Biochemistry Journal,* 2004, vol. 378, 705-716
- **LESLY et al.** *Methods in Molecular Biology,* 1995, vol. 37, 265-278
- **PALVA I. et al.** Secretion of Eschirichia coli beta-lactamase from Bacillus subtilis by the aid of alpha-amylase signal sequence. *Cell Biology,* 1982, vol. 79, 5582-5586
- **SPIZIZEN J.** Transformation of biochemically deficient strains of Bacillus subtilis by deoxyribonucleate. *Proc. Natl. Acad. Sci. US,* 1958, vol. 44, 1072-1978

SEQUENCE LISTING

<110> Direvo Biotech AG

<120> Serine Proteases with Altered Sensitivity to Activity-Modulating Substances

<130> 061290wo

<150> EP 06763303.2
<151> 2006-05-26

<150> EP 05104543.3
<151> 2005-05-27

<160> 7

<170> PatentIn version 3.3

<210> 1
<211> 29
<212> DNA
<213> artificial

<220>
<223> Primer P1

<400> 1
tggcaggagg ggccactcag gcctttgca 29

<210> 2
<211> 26
<212> DNA
<213> artificial

<220>
<223> Primer P2

<400> 2
cacctagtgg cctagtcggc cttagc 26

<210> 3
<211> 54
<212> DNA
<213> artificial

<220>
<223> Primer P3

<220>
<221> misc_feature
<222> (31)..(36)
<223> N=A, C, G or T

M=C or A

<400> 3
gatgatctgc tcattcccct ccaaggctcc mnnmnngtgc actcccagtc tcac 54

```
<210>  4
<211>  18
<212>  DNA
<213>  artificial

<220>
<223>  Primer P4

<400>  4
gggaatgagc agatcatc                                                       18


<210>  5
<211>  224
<212>  PRT
<213>  Homo sapiens

<400>  5

Ile Val Gly Gly Tyr Asn Cys Glu Glu Asn Ser Val Pro Tyr Gln Val
1               5                   10                  15

Ser Leu Asn Ser Gly Tyr His Phe Cys Gly Gly Ser Leu Ile Asn Glu
            20                  25                  30

Gln Trp Val Val Ser Ala Gly His Cys Tyr Lys Ser Arg Ile Gln Val
        35                  40                  45

Arg Leu Gly Glu His Asn Ile Glu Val Leu Glu Gly Asn Glu Gln Phe
    50                  55                  60

Ile Asn Ala Ala Lys Ile Ile Arg His Pro Gln Tyr Asp Arg Lys Thr
65                  70                  75                  80

Leu Asn Asn Asp Ile Met Leu Ile Lys Leu Ser Ser Arg Ala Val Ile
                85                  90                  95

Asn Ala Arg Val Ser Thr Ile Ser Leu Pro Thr Ala Pro Pro Ala Thr
            100                 105                 110

Gly Thr Lys Cys Leu Ile Ser Gly Trp Gly Asn Thr Ala Ser Ser Gly
        115                 120                 125

Ala Asp Tyr Pro Asp Glu Leu Gln Cys Leu Asp Ala Pro Val Leu Ser
    130                 135                 140

Gln Ala Lys Cys Glu Ala Ser Tyr Pro Gly Lys Ile Thr Ser Asn Met
145                 150                 155                 160

Phe Cys Val Gly Phe Leu Glu Gly Gly Lys Asp Ser Cys Gln Gly Asp
                165                 170                 175
```

```
Ser Gly Gly Pro Val Val Cys Asn Gly Gln Leu Gln Gly Val Val Ser
            180                 185                 190

Trp Gly Asp Gly Cys Ala Gln Lys Asn Lys Pro Gly Val Tyr Thr Lys
        195                 200                 205

Val Tyr Asn Tyr Val Lys Trp Ile Lys Asn Thr Ile Ala Ala Asn Ser
    210                 215                 220

<210>  6
<211>  224
<212>  PRT
<213>  Homo sapiens

<400>  6

Ile Val Gly Gly Tyr Ile Cys Glu Glu Asn Ser Val Pro Tyr Gln Val
1               5                   10                  15

Ser Leu Asn Ser Gly Tyr His Phe Cys Gly Gly Ser Leu Ile Ser Glu
            20                  25                  30

Gln Trp Val Val Ser Ala Gly His Cys Tyr Lys Ser Arg Ile Gln Val
        35                  40                  45

Arg Leu Gly Glu His Asn Ile Glu Val Leu Glu Gly Asn Glu Gln Phe
    50                  55                  60

Ile Asn Ala Ala Lys Ile Ile Arg His Pro Lys Tyr Asn Ser Arg Thr
65                  70                  75                  80

Leu Asp Asn Asp Ile Leu Leu Ile Lys Leu Ser Ser Pro Ala Val Ile
                85                  90                  95

Asn Ser Arg Val Ser Ala Ile Ser Leu Pro Thr Ala Pro Pro Ala Ala
            100                 105                 110

Gly Thr Glu Ser Leu Ile Ser Gly Trp Gly Asn Thr Leu Ser Ser Gly
        115                 120                 125

Ala Asp Tyr Pro Asp Glu Leu Gln Cys Leu Asp Ala Pro Val Leu Ser
    130                 135                 140

Gln Ala Glu Cys Glu Ala Ser Tyr Pro Gly Lys Ile Thr Asn Asn Met
145                 150                 155                 160

Phe Cys Val Gly Phe Leu Glu Gly Gly Lys Asp Ser Cys Gln Gly Asp
                165                 170                 175
```

```
Ser Gly Gly Pro Val Val Ser Asn Gly Glu Leu Gln Gly Ile Val Ser
            180                 185                 190

Trp Gly Tyr Gly Cys Ala Gln Lys Asn Arg Pro Gly Val Tyr Thr Lys
        195                 200                 205

Val Tyr Asn Tyr Val Asp Trp Ile Lys Asp Thr Ile Ala Ala Asn Ser
    210                 215                 220


<210>  7
<211>  224
<212>  PRT
<213>  Homo sapiens

<400>  7

Ile Val Gly Gly Tyr Thr Cys Glu Glu Asn Ser Leu Pro Tyr Gln Val
1               5                   10                  15

Ser Leu Asn Ser Gly Ser His Phe Cys Gly Gly Ser Leu Ile Ser Glu
            20                  25                  30

Gln Trp Val Val Ser Ala Ala His Cys Tyr Lys Thr Arg Ile Gln Val
        35                  40                  45

Arg Leu Gly Glu His Asn Ile Lys Val Leu Glu Gly Asn Glu Gln Phe
    50                  55                  60

Ile Asn Ala Ala Lys Ile Ile Arg His Pro Lys Tyr Asn Arg Asp Thr
65                  70                  75                  80

Leu Asp Asn Asp Ile Met Leu Ile Lys Leu Ser Ser Pro Ala Val Ile
                85                  90                  95

Asn Ala Arg Val Ser Thr Ile Ser Leu Pro Thr Ala Pro Pro Ala Ala
            100                 105                 110

Gly Thr Glu Cys Leu Ile Ser Gly Trp Gly Asn Thr Leu Ser Phe Gly
        115                 120                 125

Ala Asp Tyr Pro Asp Glu Leu Lys Cys Leu Asp Ala Pro Val Leu Thr
    130                 135                 140

Gln Ala Glu Cys Lys Ala Ser Tyr Pro Gly Lys Ile Thr Asn Ser Met
145                 150                 155                 160

Phe Cys Val Gly Phe Leu Glu Gly Gly Lys Asp Ser Cys Gln Arg Asp
                165                 170                 175
```

```
Ser Gly Gly Pro Val Val Cys Asn Gly Gln Leu Gln Gly Val Val Ser
            180                 185                 190

Trp Gly His Gly Cys Ala Trp Lys Asn Arg Pro Gly Val Tyr Thr Lys
        195                 200                 205

Val Tyr Asn Tyr Val Asp Trp Ile Lys Asp Thr Ile Ala Ala Asn Ser
    210                 215                 220
```